# EUROPEAN PATENT APPLICATION

(11) **EP 4 063 522 A1**
(43) Date of publication of application: **28.09.2022**
(21) Application number: 20889470.9
(22) Date of filing: 20.11.2020
(51) Int. Cl.: C12Q 1/6886, G01N 33/574

(54) **COMPOSITION FOR PREDICTING RESPONSE TO STANDARD PREOPERATIVE CHEMORADIATION THERAPY AND PROGNOSIS FOLLOWING TREATMENT, AND METHOD AND COMPOSITION FOR PREDICTING PATIENTS WITH VERY UNSATISFACTORY PROGNOSES FOLLOWING STANDARD THERAPY**

(30) Priority: 20.11.2019 KR 20190149594
(71) Applicant: Novomics Co., Ltd., Seoul 07217 (KR)
(72) Inventor: PAIK, Soonmyung, Seoul 03168 (KR); MIN, Byung Soh, Seoul 04035 (KR); KATHERINE, Pogue-Geile, Pittsburgh, Pennsylvania 15212 (US)
(74) Representative: Schiweck Weinzierl Koch Patentanwälte Partnerschaft mbB
(86) International application number: PCT/KR2020/016526
(87) International publication number: WO 2021/101339

(57) **Abstract**

The present invention relates to a biomarker composition for predicting the prognosis of a cancer patient, the biomarker composition including a first molecular subtype or a protein transcribed and translated from the first molecular subtype. The present invention also relates to a biomarker composition for predicting the prognosis of a cancer patient, the biomarker composition further including a second molecular subtype or a protein transcribed and translated from the second molecular subtype.

<110>   Industry-Academic Cooperation Foundation, Yonsei University

<120>   Composition for Prognosis and neoadjuvant chemoradiotherapy
        response prediction of rectal cancer and method and composition
        thereof for identification of extremely poor risk patients after
        standard neoadjuivant chemoradiotherapy in rectal cancer

<130>   POPB204169PCT

<150>   KR 10-2019-0149594
<151>   2019-11-20

<160>   11

<170>   KoPatentIn 3.0

<210>   1
<211>   660
<212>   PRT
<213>   Homo sapiens

<400>   1

<210>    2
<211>    359
<212>    PRT
<213>    Homo sapiens

<400>    2

<210>   3
<211>   321
<212>   PRT
<213>   Homo sapiens

<400>   3
Met Ala Ser Ser Gln Gly Lys Asn Glu Leu Lys Leu Ala Asp Trp Met
1               5                   10                  15

Ala Thr Leu Pro Glu Ser Met His Ser Ile Pro Leu Thr Asn Leu Ala
            20                  25                  30

Ile Pro Gly Ser His Asp Ser Phe Ser Phe Tyr Ile Asp Glu Ala Ser
        35                  40                  45

Pro Val Gly Pro Glu Gln Pro Glu Thr Val Gln Asn Phe Val Ser Val
    50                  55                  60

Phe Gly Thr Val Ala Lys Lys Leu Met Arg Lys Trp Leu Ala Thr Gln
65                  70                  75                  80

Thr Met Asn Phe Thr Gly Gln Leu Gly Ala Gly Ile Arg Tyr Phe Asp
                85                  90                  95

Leu Arg Ile Ser Thr Lys Pro Arg Asp Pro Asp Asn Glu Leu Tyr Phe
            100                 105                 110

Ala His Gly Leu Phe Ser Ala Lys Val Asn Glu Gly Leu Glu Glu Ile
        115                 120                 125

Asn Ala Phe Leu Thr Asp His His Lys Glu Val Val Phe Leu Asp Phe
    130                 135                 140

Asn His Phe Tyr Gly Met Gln Lys Tyr His His Glu Lys Leu Val Gln
145                 150                 155                 160

Met Leu Lys Asp Ile Tyr Gly Asn Lys Met Cys Pro Ala Ile Phe Ala
                165                 170                 175

Gln Glu Val Ser Leu Lys Tyr Leu Trp Glu Lys Asp Tyr Gln Val Leu
            180                 185                 190

Val Phe Tyr His Ser Pro Val Ala Leu Glu Val Pro Phe Leu Trp Pro
        195                 200                 205

Gly Gln Met Met Pro Ala Pro Trp Ala Asn Thr Thr Asp Pro Glu Lys
    210                 215                 220

Leu Ile Gln Phe Leu Gln Ala Ser Ile Thr Glu Arg Arg Lys Lys Gly
225                 230                 235                 240

Ser Phe Phe Ile Ser Gln Val Val Leu Thr Pro Lys Ala Ser Thr Val
                245                 250                 255

Val Lys Gly Val Ala Ser Gly Leu Arg Glu Thr Ile Thr Glu Arg Ala
            260                 265                 270

Leu Pro Ala Met Met Gln Trp Val Arg Thr Gln Lys Pro Gly Glu Ser
        275                 280                 285

Gly Ile Asn Ile Val Thr Ala Asp Phe Val Glu Leu Gly Asp Phe Ile
    290                 295                 300

Ser Thr Val Ile Lys Leu Asn Tyr Val Phe Asp Glu Gly Glu Ala Asn
305                 310                 315                 320

Thr



<210>    4
<211>    183
<212>    PRT
<213>    Homo sapiens

<400>    4
Met Glu Lys Pro Tyr Asn Lys Asn Glu Gly Asn Leu Glu Asn Glu Gly
1               5                   10                  15

Lys Pro Glu Asp Glu Val Glu Pro Asp Asp Glu Gly Lys Ser Asp Glu
            20                  25                  30

Glu Glu Lys Pro Asp Ala Glu Gly Lys Thr Glu Cys Glu Gly Lys Arg
        35                  40                  45

Lys Ala Glu Gly Glu Pro Gly Asp Glu Gly Gln Leu Glu Asp Lys Gly
    50                  55                  60

Ser Gln Glu Lys Gln Gly Lys Ser Glu Gly Glu Gly Lys Pro Gln Gly
65                  70                  75                  80

Glu Gly Lys Pro Ala Ser Gln Ala Lys Pro Glu Gly Gln Pro Arg Ala
                85                  90                  95

Ala Glu Lys Arg Pro Ala Gly Asp Tyr Val Pro Arg Lys Ala Lys Arg
            100                 105                 110

Lys Thr Asp Arg Gly Thr Asp Asp Ser Pro Lys Asp Ser Gln Glu Asp
        115                 120                 125

Leu Gln Glu Arg His Leu Ser Ser Glu Glu Met Met Arg Glu Cys Gly
    130                 135                 140

Asp Val Ser Arg Ala Gln Glu Glu Leu Arg Lys Lys Gln Lys Met Gly
145                 150                 155                 160

Gly Phe His Trp Met Gln Arg Asp Val Gln Asp Pro Phe Ala Gln Gly
                165                 170                 175

Asp Asn Gly Val Ser Gly Glu
            180

<210>    5
<211>    319
<212>    PRT
<213>    Homo sapiens

<400>    5
Met Met Val Leu Lys Val Glu Glu Leu Val Thr Gly Lys Lys Asn Gly
 1               5                  10                  15

Asn Gly Glu Ala Gly Glu Phe Leu Pro Glu Asp Phe Arg Asp Gly Glu
            20                  25                  30

Tyr Glu Ala Ala Val Thr Leu Glu Lys Gln Glu Asp Leu Lys Thr Leu
        35                  40                  45

Leu Ala His Pro Val Thr Leu Gly Glu Gln Gln Trp Lys Ser Glu Lys
    50                  55                  60

Gln Arg Glu Ala Glu Leu Lys Lys Lys Lys Leu Glu Gln Arg Ser Lys
65                  70                  75                  80

Leu Glu Asn Leu Glu Asp Leu Glu Ile Ile Ile Gln Leu Lys Lys Arg
                85                  90                  95

Lys Lys Tyr Arg Lys Thr Lys Val Pro Val Val Lys Glu Pro Glu Pro
            100                 105                 110

Glu Ile Ile Thr Glu Pro Val Asp Val Pro Thr Phe Leu Lys Ala Ala
        115                 120                 125

Leu Glu Asn Lys Leu Pro Val Val Glu Lys Phe Leu Ser Asp Lys Asn
    130                 135                 140

Asn Pro Asp Val Cys Asp Glu Tyr Lys Arg Thr Ala Leu His Arg Ala
145                 150                 155                 160

Cys Leu Glu Gly His Leu Ala Ile Val Glu Lys Leu Met Glu Ala Gly
                165                 170                 175

Ala Gln Ile Glu Phe Arg Asp Met Leu Glu Ser Thr Ala Ile His Trp

180                 185                 190

Ala Ser Arg Gly Gly Asn Leu Asp Val Leu Lys Leu Leu Leu Asn Lys
        195                 200                 205

Gly Ala Lys Ile Ser Ala Arg Asp Lys Leu Leu Ser Thr Ala Leu His
    210                 215                 220

Val Ala Val Arg Thr Gly His Tyr Glu Cys Ala Glu His Leu Ile Ala
225                 230                 235                 240

Cys Glu Ala Asp Leu Asn Ala Lys Asp Arg Glu Gly Asp Thr Pro Leu
                245                 250                 255

His Asp Ala Val Arg Leu Asn Arg Tyr Lys Met Ile Arg Leu Leu Ile
            260                 265                 270

Met Tyr Gly Ala Asp Leu Asn Ile Lys Asn Cys Ala Gly Lys Thr Pro
        275                 280                 285

Met Asp Leu Val Leu His Trp Gln Asn Gly Thr Lys Ala Ile Phe Asp
    290                 295                 300

Ser Leu Arg Glu Asn Ser Tyr Lys Thr Ser Arg Ile Ala Thr Phe
305                 310                 315

<210>   6
<211>   11670
<212>   DNA
<213>   Homo sapiens

<400>   6
gcacaggggc acccattgaa ccagggcagg gcctgttagc ttatgaccag caggtgacct      60

gatttccttg tccactggca cagagcacac cctgccacag tgcaagcctg agaagcagtg     120

aggaccccat gaagctgaag gacaggccga tcccccgata gacgattagg taatgactgc     180

tggtatggag tcagtattgg gcttcaagcc aatgaggaaa gctgggtaga gcgagaatcc     240

tgatgaatgg acctgaaaaa ggaactcacc tcaggcttga tagaggaatt tttcagtaca     300

gctaaagtgg acacctgtcc ttttacttgt ttggaatcca ttccccagac tttgggacag     360

catctcaagc ttgttttgga gaacctccct tctccacttg cattctatgg ggtgatggtg     420

acagggtgat caaccccaac cccttgcccc caccccaatc ccacagctcc tgggttcaag     480

ttccagctcc aacatagatc agatatgtaa ccaaccctgg gttagtcata aagtctcagt     540

tatctcatct gacggaggct gtcagcccgt gggttaggca gggaggatcc agtgagaaac     600

actgtatagg cactctgcac atcacaagac tcagttctag gtatagctgg gctattcttt     660

cctcatatat gaatcctcca gtaagaggaa ccaggaaagg taagcatgaa taactgtttg     720

gagctcggag aagttaaagg actgacctaa gagtgcaagg ccactaagga gaggacctgt     780

gacttaagtc ctgtgaactg agatccttgt agtatgtggg aacaccatac cacttcaccc     840

cattaaggta aaccctctat gttacttgag agattgaaaa caacactaaa aagagaactc     900

tctacctaaa aaccattgct tcttgccaat ttcaatccag taagattggt ggtatcttat 960

gtagattttc accttggata tggagcagcc caaagattta tttcaagagc tttccctctg 1020

atgcacacag gaatctaaca cacacattcc aataagagga gagagaaaag agaagaggct 1080

ggcactggtg ctgggtgcat gccaagcgtc gtggcaccct cctcatataa ccctgctgtt 1140

tcatgtgcag gcagtgcaag acacagcaag gagccaagca gagtcaataa tatttacttt 1200

gcagaaacat ctgttgctag aaaagcagaa tgctggccag agtggaggga agtcataact 1260

gccggcatct ggggaggcca agtatctgct ttccagagta ggtagggctg tgcttctctg 1320

agtgagggga ccagatggct aagaaatatg gaagcagcta gaagtttggg ccttgagaga 1380

ctgctcccag agccacctgc tcactcaggg actagaggaa gtgcccttct tttcttggag 1440

atgccagcag atattaatct gcaccggatt taagagaatt taacccaaat agatccaact 1500

ttcactaaca cagaaggttt accataattt tttaaaaatt agaatctacc ttaattttat 1560

acaagttgta ccagtaatac cacaagtcaa gccatttcct ttttctcctt gggtaaaact 1620

taacatacat gtgcacacac aaacatggac tcaggtaacc ttactactgt attttcagtg 1680

catctattgc ctttgtgacc tccaaagtta ccaaaattgt gtattaacta cccttgaaat 1740

gttttgctgc tcagtcacat ctttcccttt ggacttagaa agtgattaca gttccgggag 1800

gccaaggcgg gtggatcatg aggtcaggag ttcgagacca gtctggccaa catagtgaaa 1860

cctcatctct actaaaaata caaaaaaaaa taaaataaaa tagccgggtg tactggtgtg 1920

cacctgtaat cccagctact cagggaggct gaggcaggag aatcgcgtga acccgggagg 1980

ctgaggctgc agtgagccga ggttgcacca ttgcactcca gcctaggcga cagtgtgaga 2040

cgctgtctca aaaaaaaaaa aaaaagaaaa aagaaaaaga aaaagaaaag aaaagaaagt 2100

gattacagct cagaaaggcc atcattgagg ttagaacttg caacaggatt ggcctcatgg 2160

ctgcctaaag ttcaagaata ccaaagtccc cgggccttgt taatctcttc ctttcctgga 2220

cgccagttac tctctgtgcc tcatcacctt gtatttggaa taattctgga actagtcatg 2280

aatggcttca tgtcacaatg cacaaagagt gcctcggaca gggctggaca cagtgtccct 2340

ccaaacctgt tgtacgatgt gagcgggcaa ggaaacacaa gctggcttac aggtggaata 2400

cctgagagtc tccagagagg acccatgaca gacagctgag acatggatgg acacgagccc 2460

cagagagact tcaggtttag cccacacccc cattcctagg tcaaactcaa cacactcaaa 2520

acaaaactct ctctactcaa acaccctctg ccaccctctc cccagagagc cagccgaggg 2580

cctcttcctg catccccagt gtaacgaagg gcaccaccat tcaccctgcc gcacaagtca 2640

ccagtacatg tcatcctctc acacagcagg ctccctcatg tgccatgtcc attcccaagt 2700

ctcctaagca catctcaatt ctgcccactc cttcccatca ctatcagcaa cccctaaaca 2760

aaacacctcc atttgcccag ataactgcaa ctgctcctct ctggcctccc cacatccact 2820

tttgtccgtc aaatcagttc tccacccagt tgtgtcaata ccagcagtga gccctgtgat 2880

ggcttcccag agctctggag gtgaagacga aagtccctcc caaggcctga gaggccctgc 2940

agggtctggc ctccccacac ccctctctgg gctccagctg ctccagcctc tgccagttcc 3000

cggggcatgc ccaactccta cctgccacca catgccacct ctgcctgcag cccttggccc 3060

aaaagttctt tcctccagga agccttccct gacttctcta atcaggccaa acccaaccct 3120

atttctcact ctctgagtac tatactatag atcttttctt cagagcaatt ttatatttgt 3180

tcaggtaatt cttctcttca agtaatgcct ccccaactag tacattctat aaggatgagg 3240

atcatatccc ttcatgctca ccactctatc cccaaagccc aacactctac tgcttgactg 3300

agagcaggta ttgcagaaag aacaaagaga gaggaagttg ggcactccat aagtccatta 3360

gccttcatca gaggtgaacc tcccaactca atgtttgaca tgtgaaaaaa aaagcaggta 3420

agttaggaca aactctccaa cttatgtaag tctggctaga gcctgatata tatgtgggaa 3480

tgtgcttcct attagcatgt gaccacctcc cttcatgttg tccattgagg aagagtccca 3540

cagagctgga gggagagcta ccaccagcag gagcataaaa taaaacctca cctataccaa 3600

ggaagctgga gaaatgagct acatacaacc caactgctgg ccacaaccaa ctgtcctctc 3660

ttagccccag cacaagcccc aggttgcagt taaaatggtt taaggaagag tccccagaga 3720

tcgctctttt ctttctcccc gtatctggga aaactgagaa catatccaat cagaaactgt 3780

gcttggagag aggcatttcc aacgactccc catttcccca agtctgtatg tagcagttta 3840

aacctagttc gatgtgcttc attataagaa aagcaagtta cagtcccttg taaataccat 3900

tctgcagccc agactcctgg cattgtacta gcaaaaacaa acaaaacaca cacacacaca 3960

cacacacaca cacacacaca cacaccctgt gcaaatctta gaatgaaaca aaattctcca 4020

tagcattttt ctcgtttccg tgacacacgg tcttacctgt ggccactagg tggcaatcaa 4080

ctatatcaga gaacaccttg accaagcagc taatttaaac aacagctaag actcaaggag 4140

aaatgacagg gccagcctta aaggcagtgc tgagaacaga tcctgtccca ctgaggaaca 4200

aataaccacc tagaaatgtc cagtggacac tcagcaactg agagaggggc cctggcttcc 4260

acattggaag aagtgctcta acacattcgg agggcctgtg ctctcataca gcaagtcaag 4320

cctgagacat ggagtgggag gggtataggg atgaagtcac caccacagtg tctctcacat 4380

tctttcagat ctggctggag cagattcaga aaacctctta tctcccccag gcggatgatg 4440

tgcccaccga ccacatcagc cacggtacgt tacataacat gcattctgat catacatctg 4500

cgtgcttttc ctgaggccat ttccctaaaa cagcatttcc taaatgaggc catcaccagc 4560

actgattctg gaaaatttgt ttttccaacc agagcactaa ggctaatgaa attttcagag 4620

ttgattttta aaatgaaatc aagtagaata aagacgtgct cctctttcca aaatgaggtt 4680

tctaattcta tttagatgaa atacccctta aaaataattt gggctaccca aactcacctt 4740

aatctacatc caaataacta tgaatatatt atatacatta acttactgaa tactgtacct 4800

accaagtact ttaaatccct ccatcctcac aaccattctg ttgagtagat attattacct 4860

ccattttgca gatgggaaac tgaggctcag agaggttaaa taacgtgccc aagatcatgc 4920

agggaatgag tggtaatgta agaaacttgg tctctctgac tccaaaactc ttaactacta 4980

cactatattg cctcatttac tcaaatattt gagaatctgg tatcaatgta ttcaaagaaa 5040

tgaaaagatg gtatgtatac attccgtgcc ttaaggggaa tacaattttt agaagggcct 5100

aatctgtgtg tgcaaaacta caaaattaag gtagactgtg ctagggcaat cagagagtag 5160

acaaaaatgg ctacagaaat gcaagtggga tattattccg gctagggcca ttggaggggg 5220

cttccaaaaa aaaaaaaaga aaaaagaaaa agaacaggga gtagcgtttg agctgaacct 5280

taaagttcag tcctatttgg atatgtagaa aagggaagcg gaagcagtca tgggaagatg 5340

ctatagaagt ggaaacatgg agcaaggaga tgcgaaatgc agaacctacc cagaaaaagc 5400

agcaagttta actagatgga caggaggtca gagtgggaag accggttaga atcaaaccat 5460

ggaaggacct gtatgccaag ctaaggaatt tgaacttact cccttaaaga aatgggaact 5520

ggattgttat acagaggagc aacaagatca acttaaacaa caattaagcc ttttactaag 5580

gagctggccc agaactttgt tggagacacc tactgtaact cctccagcaa gcttcactat 5640

gagaatgtgc aaccacagcc aaggggcatt ctccctgtag gtaactaaat gacaggcaac 5700

acctctcaac ttggacattt gtgcacagac cattggtcag ccaggagata aggaaagagt 5760

tataagggat tcaaaaaaaa aaaaaaaaaa aaaaacttct ccgtgttgaa aactccaagt 5820

tctgggccca ctacgactta tacataactg tatgcaaata atgtatacaa atacatatgt 5880

atttccaaag tgctgtattg gcttttcctc ataagaacat tgagagttaa gtgaaagcac 5940

ctgacctctt ctagatgaag aaattaagga ctggcttaat gacagtgcaa gtcacatagt 6000

aggtgcttaa taaacattta ctggatcaat gaccgctgtg tcaagtgagt tagtcagcat 6060

tggatctccg atgaaaactc aacccctaaa gagcccagcc ccagctcagc cctggcacag 6120

agaactgagg tactgtattt agaacaactg cattcacatg ctgccccaag acatgataag 6180

acagcagaag gtcaccacag gaaaaactcc ctcaaggaag actgtggaca tttcttaatg 6240

aaaacaaaga aacccatttt gagagggact aatcaagtga gggaacaaaa aagagagggt 6300

aattccaggc agctcaagta caaatatcac acacacacgg ggcaagggga aggtaattca 6360

gaggcgtatg acgcgcattg gctgcgcaaa catcagctta aatcccggtc tctcacttcc 6420

cagctgtgtg gcctttggca aattacccaa cctttccaag catgtatgta actgtgagga 6480

cagtatgtac ctcacagggc tgttgtggga attcaaaata ggaaagacac atgttctgga 6540

agagagagga cagggatgcc cccggccctg ccttgctttc cttcccacag cagaggtgga 6600

gtggaagttt accagcaggt tggagaaagg agaatcaacc aaaggagaat cttaattacc 6660

ataacaatta agcaattttt gcctcattga gtcaccagat caaccccatt cttgtgaact 6720

actaaaaaca cacctgagtt cctaagggtt ttcttcagtg ctcagaaaac ccctaatgct 6780

tctacaaatt tacaatttcc tttcacctct ggcagatgaa aggcaaggca aaacccggat 6840

ggaccatttt ttaactaaac caatttttaa ctaaacgaac tcaaagcagt atgggtttct 6900

ttttgtgaag tttattaagc tcaagtcgtg ccttttaaat gcaccgggga aacagtgcta 6960

tcatcaaatc ctttctgtcc actgcctggg gaggcaggcc agccccagat gaaacagata 7020

aaagaatgag tgtgttaaga cgacatttaa gacacaagag actgacagcg ttcacaggcc 7080

tgaagtactg ggcttcctca gaaggtggaa cttgagcttg gcccctaagg tagacacgtt 7140

tctagaaagg ggagggggtg gtgaattaag aggagaccac acaagtcaga cccactcact 7200

gggtcagact agggctggaa aaaccggtag gggctggtgg gtggagatag agggagatag 7260

aggtctaaga atggtgggga ggggcaggtg gagcagggat tcctctctct caacacctca 7320

gcagttctgt tctgctcaac accaagcagg tgcgcagata ggaggcagtg gggctgggag 7380

ggttgatcct ggcttgagcc tcaagactta gattttgttt ctggaatgcc ttttcctctg 7440

cagcagagac agctactcaa gctttaaggt gcacccaatc acctgggatc ttgtcaaaat 7500

tcccatgtga tccagcaggc ctgggagccc gagagtctac atttctaatt cccaggtgaa 7560

gccaatggtg ctgatccagg caccacaccc tccaaggcaa gggtttggac tcccactcaa 7620

gaccctgaca gagaagatca cagcttcttt attctcctag cacgtgcctg gaacatacag 7680

gacctcaata tatattggag gaatgaatga ataaattcct tgagagaact taagctgttg 7740

cagacagaat agaaaagaac ccagaaaaag aaccagaaat ggcgtgtctc cttgtgcagc 7800

caccaactat acatgtgaac ctagggagag ttatgtgaca cactcggcct taaaagaact 7860

cacttgaggc cgggcgcagt ggctcacgcc tgtaatccca gcactctggg aggccgaggt 7920

gggcggatca cgaggtcagg agttcaagac cagcctggcc aacacagtga aaccctgtct 7980

ctggctaata caaaaattag ctgggcttgg tggtgggcac ctgtaatcac agctactcgg 8040

gaggctgagg caggagaatc gcttgaaccc aggaggcggg gcttgcagtg agccgagatc 8100

gtgccatagc actccagcct gtgtgacaga gcaagactcc ttctcaaaaa gaaaaaaaaa 8160

aaaaaaacct cacttgaaaa ccaaggagcc aacccctttc cgtagctcta agatccctcc 8220

caaagtatct tttctgtgcc ttttattttt aatggtgagg ttatatactg gatattccta 8280

cagcaaggca cacctctatg tcattctcaa agagcagttt ccaaaaatcc aattgagaga 8340

acacagactc tgttagtctc agcaaataac catagcaact aatactcccc aaatgaaatg 8400

atgtcttcac aatgatgtgc tggctcaatt agattaagag ggtgacgtgt accatctgaa 8460

aatttatcgt cgtaaactta cagtgaaagc cagaccttct tggaagaggt ttataagtaa 8520

aagaaaggcc ttgccaataa atggaattca ggacaaagag ccagagaagt caagcagatg 8580

aggaaattgg gaaggactgc tctgcctctc caggttcaca cagtcagggc ccacgcaccc 8640

agacagaacc cagaacttgg ttaaacagtt agaaatctga gtaccaccac ttctaccagg 8700

gagttatcaa aagactgcca ttttatcctt gttctatgcc tttcttcata catattacaa 8760

aaggaaaata ctgctacctc agaaaagtct gcagaaatat ttaccaaaat aattgtaaaa 8820

cttttaaatg aaaaagagga caacttcaaa ataacaaatt agggctgata atacatttcc 8880

agccaaatta atttctccac aggtcatcac aggatgcaaa tctttctgtt acaaggtttt 8940

ctgactggag ttagaactgt gaatggaaaa ctcaacgtct ataagttcta caacttactg 9000

tctttccaca tccttaggcc tgcctcttcc agggtccatg caatcacatt tgccctgaac 9060

aaatttgggc aagcaagaac ctcacttgaa aagcaggaaa agagaagcca aggcctgtca 9120

cctccctgta atccctgagc tttctagctt tctctagcaa agaacaaaaa gaaaaaaagg 9180

aacaacaaac aagccaaccc ataaccaagc ccaaagaatc agagtttcac tggcattcaa 9240

cagttgtgaa ttcggaaagt cacagacggc agaacctgga gccatacaca aggatcagaa 9300

acccctgcac tgttcctacc aacagctaac gtgaatttga gggcaatcaa gagaaaaaca 9360

aggtatggtg ggtaaatcga ggccttcatc cagccaaata agcttagatt tgtggttaac 9420

agactcccag cgttcaaaat cccatctcac ccatttacaa ggtactcaat ttccacctgt 9480

gtgaaaaggc actaaaggga aacctgcctc ctgaggctcc tgtgagaaga gagatgatgc 9540

actgaagtgc ttagcacagg gcctggccgg cagcaaacac tcaataaacc ataggtatta 9600

gaatcattta catatatatt taataaataa catgccaggc tgatttgttt ctgcgtaaaa 9660

atgaaaggac aatgaagcca cccggcccct accactttga ctccctctcg ccctcctcac 9720

cgtcagtcca cacagcataa ttaggatcaa aactgaatcc acctgatccc tggacacagc 9780

tcagacactc cttgccacta ataccacagg aagcaaggtg gcaactccta agcatcagaa 9840

taaattcagt taaaaaacaa aaatccgctc atctccccct ggatgaaatt catgtttctg 9900

gaagaaaaaa gaaaaaaaaa aaacggacct agccattctg aacaaagcca cacctaataa 9960

cattcccaga aattggtttc actttctttt atcccctctt taattagtca cttttctgct 10020

atacctcagg ggaggctgtg ctccagggac caagaacgga agcctgagtg ggcaaacact 10080

taccctcctc ataggtgccc acgaaatccc caggctcccc gcctccccac tctgcaccgt 10140

ctcctctcca gcctggattc cagtccaggc aggaactcat gctccagcaa ggagtacccc 10200

ttgccactgc agaccaccac agaatgagag aggccaaaga taagtgtttc tcaaaacgtg 10260

tctgaacacc gcttgcaata ggtgttgcaa gagcccttcc agggagccca ccctcaatca 10320

aaatctctag ggctgaggcc taggagcctg ggcttcccag gcagttctta tccatgctga 10380

agtttgagag ctgataccac ctaacccttc agaacatcta atactaagta ccagtaatgg 10440

aacccctcca gatgacaaag gtggagctcc acagagcaag ccggatcttc taaatcatga 10500

cagtgggttt acaaatgttc ccaaagcagg caattaatgc tcgtggtaga agaaagctag 10560

gtgacagcag gggtgggagg aggaaacatc ctaattcaat ttaaaaatca caaattctga 10620

agcctgagtc ttcttcaaaa catgttctca atcatgtctc cttattctga agcctacaca 10680

actgactggg aacacatctt tgttttgtag aaacccagct catggcacct ggagctgatg 10740

aatggccctg gtgctgactg cccagaaacc aatccggaaa ccaggcttcc cacagaagag 10800

gcagctggag agccaagcac tcttggacta cagtcctagg ctggagctgc caatacaggc 10860

tctgcccagc taatcttctc tgagcctgca aatattaaac ctgacataca gactcaagtg 10920

aatccagaaa acaggctcac agggcagctg aaaattccaa gtgcctaagg tataggcagg 10980

gcggcttcat gggcattcaa ccagtacaga tgcacagggt cccatgctca gaagggcctg 11040

cttggcttaa tgctctgctg tagctgtctt gaaattttta ataattttat ctttgaactt 11100

gtgttttgca agtgagatcc agtgcgacag tggagcatgt gtgcgcagag gagacaggaa 11160

caacaaccgg gatgtcatgc gggcgcactg gggctccttg tggccccagt gcacagaatt 11220

ctggtggacc caccatgtgt gggagctcag tgagactcaa agtaagtgca aggtaagtgt 11280

cttatatcta cgactgagta agggggaacg ctgacagccc tgaggggtta cacctaccat 11340

ttcaaccaga acttgccaga tgcaagaaga tgacaatgag acaatggcat ccaaagaaac 11400

atgaacggcc caggaaccct atcatagcct tcttcctcat gttatttccc tggataagcc 11460

aaccactgac actgatcatg gtaacctagg acagagacag actgggcaac ccatagttcc 11520

tttcctttca ggccctcctc acacatcgta cgctgaaggt ggagcacact gggaaaatgt 11580

gcatgcatca agaagaaaaa tgaaaactga ttttgtgcaa catttccact gttcttggaa 11640

gaatgaaata aatgcatatg agctatgaaa 11670

<210> 7
<211> 1280
<212> PRT
<213> Homo sapiens

<400> 7
Met Asp Leu Glu Gly Asp Arg Asn Gly Gly Ala Lys Lys Lys Asn Phe
1               5                   10                  15

Phe Lys Leu Asn Asn Lys Ser Glu Lys Asp Lys Lys Glu Lys Lys Pro
            20                  25                  30

Thr Val Ser Val Phe Ser Met Phe Arg Tyr Ser Asn Trp Leu Asp Lys
        35                  40                  45

Leu Tyr Met Val Val Gly Thr Leu Ala Ala Ile Ile His Gly Ala Gly
    50                  55                  60

Leu Pro Leu Met Met Leu Val Phe Gly Glu Met Thr Asp Ile Phe Ala
65                  70                  75                  80

Asn Ala Gly Asn Leu Glu Asp Leu Met Ser Asn Ile Thr Asn Arg Ser
                85                  90                  95

Asp Ile Asn Asp Thr Gly Phe Phe Met Asn Leu Glu Glu Asp Met Thr
            100                 105                 110

Arg Tyr Ala Tyr Tyr Tyr Ser Gly Ile Gly Ala Gly Val Leu Val Ala
        115                 120                 125

Ala Tyr Ile Gln Val Ser Phe Trp Cys Leu Ala Ala Gly Arg Gln Ile
    130                 135                 140

His Lys Ile Arg Lys Gln Phe Phe His Ala Ile Met Arg Gln Glu Ile
145                 150                 155                 160

Gly Trp Phe Asp Val His Asp Val Gly Glu Leu Asn Thr Arg Leu Thr
                165                 170                 175

Asp Asp Val Ser Lys Ile Asn Glu Gly Ile Gly Asp Lys Ile Gly Met
            180                 185                 190

Phe Phe Gln Ser Met Ala Thr Phe Phe Thr Gly Phe Ile Val Gly Phe
        195                 200                 205

Thr Arg Gly Trp Lys Leu Thr Leu Val Ile Leu Ala Ile Ser Pro Val
    210                 215                 220

Leu Gly Leu Ser Ala Ala Val Trp Ala Lys Ile Leu Ser Ser Phe Thr
225                 230                 235                 240

Asp Lys Glu Leu Leu Ala Tyr Ala Lys Ala Gly Ala Val Ala Glu Glu
                245                 250                 255

Val Leu Ala Ala Ile Arg Thr Val Ile Ala Phe Gly Gly Gln Lys Lys
            260                 265                 270

Glu Leu Glu Arg Tyr Asn Lys Asn Leu Glu Glu Ala Lys Arg Ile Gly
        275                 280                 285

Ile Lys Lys Ala Ile Thr Ala Asn Ile Ser Ile Gly Ala Ala Phe Leu
    290                 295                 300

Leu Ile Tyr Ala Ser Tyr Ala Leu Ala Phe Trp Tyr Gly Thr Thr Leu
305                 310                 315                 320

Val Leu Ser Gly Glu Tyr Ser Ile Gly Gln Val Leu Thr Val Phe Phe
                325                 330                 335

Ser Val Leu Ile Gly Ala Phe Ser Val Gly Gln Ala Ser Pro Ser Ile
            340                 345                 350

Glu Ala Phe Ala Asn Ala Arg Gly Ala Ala Tyr Glu Ile Phe Lys Ile
        355                 360                 365

Ile Asp Asn Lys Pro Ser Ile Asp Ser Tyr Ser Lys Ser Gly His Lys
    370                 375                 380

Pro Asp Asn Ile Lys Gly Asn Leu Glu Phe Arg Asn Val His Phe Ser
385                 390                 395                 400

Tyr Pro Ser Arg Lys Glu Val Lys Ile Leu Lys Gly Leu Asn Leu Lys
                405                 410                 415

Val Gln Ser Gly Gln Thr Val Ala Leu Val Gly Asn Ser Gly Cys Gly
            420                 425                 430

Lys Ser Thr Thr Val Gln Leu Met Gln Arg Leu Tyr Asp Pro Thr Glu
        435                 440                 445

Gly Met Val Ser Val Asp Gly Gln Asp Ile Arg Thr Ile Asn Val Arg
    450                 455                 460

Phe Leu Arg Glu Ile Ile Gly Val Val Ser Gln Glu Pro Val Leu Phe
465                 470                 475                 480

Ala Thr Thr Ile Ala Glu Asn Ile Arg Tyr Gly Arg Glu Asn Val Thr
                485                 490                 495

Met Asp Glu Ile Glu Lys Ala Val Lys Glu Ala Asn Ala Tyr Asp Phe
            500                 505                 510

Ile Met Lys Leu Pro His Lys Phe Asp Thr Leu Val Gly Glu Arg Gly
        515                 520                 525

Ala Gln Leu Ser Gly Gly Gln Lys Gln Arg Ile Ala Ile Ala Arg Ala
    530                 535                 540

Leu Val Arg Asn Pro Lys Ile Leu Leu Leu Asp Glu Ala Thr Ser Ala
545                 550                 555                 560

Leu Asp Thr Glu Ser Glu Ala Val Val Gln Val Ala Leu Asp Lys Ala
                565                 570                 575

Arg Lys Gly Arg Thr Thr Ile Val Ile Ala His Arg Leu Ser Thr Val
            580                 585                 590

Arg Asn Ala Asp Val Ile Ala Gly Phe Asp Asp Gly Val Ile Val Glu
        595                 600                 605

Lys Gly Asn His Asp Glu Leu Met Lys Glu Lys Gly Ile Tyr Phe Lys
    610                 615                 620

Leu Val Thr Met Gln Thr Ala Gly Asn Glu Val Glu Leu Glu Asn Ala
625                 630                 635                 640

Ala Asp Glu Ser Lys Ser Glu Ile Asp Ala Leu Glu Met Ser Ser Asn
                645                 650                 655

Asp Ser Arg Ser Ser Leu Ile Arg Lys Arg Ser Thr Arg Arg Ser Val
            660                 665                 670

Arg Gly Ser Gln Ala Gln Asp Arg Lys Leu Ser Thr Lys Glu Ala Leu
        675                 680                 685

Asp Glu Ser Ile Pro Pro Val Ser Phe Trp Arg Ile Met Lys Leu Asn
    690                 695                 700

Leu Thr Glu Trp Pro Tyr Phe Val Val Gly Val Phe Cys Ala Ile Ile
705                 710                 715                 720

Asn Gly Gly Leu Gln Pro Ala Phe Ala Ile Ile Phe Ser Lys Ile Ile
                725                 730                 735

Gly Val Phe Thr Arg Ile Asp Asp Pro Glu Thr Lys Arg Gln Asn Ser
        740                 745                 750

Asn Leu Phe Ser Leu Leu Phe Leu Ala Leu Gly Ile Ile Ser Phe Ile
        755                 760                 765

Thr Phe Phe Leu Gln Gly Phe Thr Phe Gly Lys Ala Gly Glu Ile Leu
    770                 775                 780

Thr Lys Arg Leu Arg Tyr Met Val Phe Arg Ser Met Leu Arg Gln Asp
785                 790                 795                 800

Val Ser Trp Phe Asp Asp Pro Lys Asn Thr Thr Gly Ala Leu Thr Thr
                805                 810                 815

Arg Leu Ala Asn Asp Ala Ala Gln Val Lys Gly Ala Ile Gly Ser Arg
            820                 825                 830

Leu Ala Val Ile Thr Gln Asn Ile Ala Asn Leu Gly Thr Gly Ile Ile
        835                 840                 845

Ile Ser Phe Ile Tyr Gly Trp Gln Leu Thr Leu Leu Leu Leu Ala Ile
    850                 855                 860

Val Pro Ile Ile Ala Ile Ala Gly Val Val Glu Met Lys Met Leu Ser
865                 870                 875                 880

Gly Gln Ala Leu Lys Asp Lys Lys Glu Leu Glu Gly Ser Gly Lys Ile
                885                 890                 895

Ala Thr Glu Ala Ile Glu Asn Phe Arg Thr Val Val Ser Leu Thr Gln
            900                 905                 910

Glu Gln Lys Phe Glu His Met Tyr Ala Gln Ser Leu Gln Val Pro Tyr
        915                 920                 925

Arg Asn Ser Leu Arg Lys Ala His Ile Phe Gly Ile Thr Phe Ser Phe
    930                 935                 940

Thr Gln Ala Met Met Tyr Phe Ser Tyr Ala Gly Cys Phe Arg Phe Gly
945                 950                 955                 960

Ala Tyr Leu Val Ala His Lys Leu Met Ser Phe Glu Asp Val Leu Leu
                965                 970                 975

Val Phe Ser Ala Val Val Phe Gly Ala Met Ala Val Gly Gln Val Ser
            980                 985                 990

Ser Phe Ala Pro Asp Tyr Ala Lys Ala Lys Ile Ser Ala Ala His Ile
        995                 1000                1005

Ile Met Ile Ile Glu Lys Thr Pro Leu Ile Asp Ser Tyr Ser Thr Glu
    1010                1015                1020

Gly Leu Met Pro Asn Thr Leu Glu Gly Asn Val Thr Phe Gly Glu Val
1025                1030                1035                1040

Val Phe Asn Tyr Pro Thr Arg Pro Asp Ile Pro Val Leu Gln Gly Leu
                1045                1050                1055

Ser Leu Glu Val Lys Lys Gly Gln Thr Leu Ala Leu Val Gly Ser Ser
            1060                1065                1070

Gly Cys Gly Lys Ser Thr Val Val Gln Leu Leu Glu Arg Phe Tyr Asp
        1075                1080                1085

Pro Leu Ala Gly Lys Val Leu Leu Asp Gly Lys Glu Ile Lys Arg Leu
    1090                1095                1100

Asn Val Gln Trp Leu Arg Ala His Leu Gly Ile Val Ser Gln Glu Pro
1105                1110                1115                1120

Ile Leu Phe Asp Cys Ser Ile Ala Glu Asn Ile Ala Tyr Gly Asp Asn
                1125                1130                1135

Ser Arg Val Val Ser Gln Glu Glu Ile Val Arg Ala Ala Lys Glu Ala
            1140                1145                1150

Asn Ile His Ala Phe Ile Glu Ser Leu Pro Asn Lys Tyr Ser Thr Lys
        1155                1160                1165

Val Gly Asp Lys Gly Thr Gln Leu Ser Gly Gly Gln Lys Gln Arg Ile
    1170                1175                1180

Ala Ile Ala Arg Ala Leu Val Arg Gln Pro His Ile Leu Leu Leu Asp
1185                1190                1195                1200

Glu Ala Thr Ser Ala Leu Asp Thr Glu Ser Glu Lys Val Val Gln Glu
                1205                1210                1215

Ala Leu Asp Lys Ala Arg Glu Gly Arg Thr Cys Ile Val Ile Ala His
            1220                1225                1230

Arg Leu Ser Thr Ile Gln Asn Ala Asp Leu Ile Val Val Phe Gln Asn
        1235                1240                1245

Gly Arg Val Lys Glu His Gly Thr His Gln Gln Leu Leu Ala Gln Lys
    1250                1255                1260

Gly Ile Tyr Phe Ser Met Val Ser Val Gln Ala Gly Thr Lys Arg Gln
1265                1270                1275                1280


<210> 8
<211> 764
<212> PRT
<213> Homo sapiens

<400> 8

<210>   9
<211>   103
<212>   PRT
<213>   Homo sapiens

<400>   9

<210>   10
<211>   463
<212>   PRT
<213>   Homo sapiens

<400>   10
Met Ala Thr Val Thr Ala Thr Thr Lys Val Pro Glu Ile Arg Asp Val
1               5                   10                  15

Thr Arg Ile Glu Arg Ile Gly Ala His Ser His Ile Arg Gly Leu Gly
            20                  25                  30

Leu Asp Asp Ala Leu Glu Pro Arg Gln Ala Ser Gln Gly Met Val Gly
        35                  40                  45

Gln Leu Ala Ala Arg Arg Ala Ala Gly Val Val Leu Glu Met Ile Arg
    50                  55                  60

Glu Gly Lys Ile Ala Gly Arg Ala Val Leu Ile Ala Gly Gln Pro Gly
65                  70                  75                  80

Thr Gly Lys Thr Ala Ile Ala Met Gly Met Ala Gln Ala Leu Gly Pro
                85                  90                  95

Asp Thr Pro Phe Thr Ala Ile Ala Gly Ser Glu Ile Phe Ser Leu Glu
            100                 105                 110

Met Ser Lys Thr Glu Ala Leu Thr Gln Ala Phe Arg Arg Ser Ile Gly
        115                 120                 125

Val Arg Ile Lys Glu Glu Thr Glu Ile Ile Glu Gly Glu Val Val Glu
    130                 135                 140

Ile Gln Ile Asp Arg Pro Ala Thr Gly Thr Gly Ser Lys Val Gly Lys
145                 150                 155                 160

Leu Thr Leu Lys Thr Thr Glu Met Glu Thr Ile Tyr Asp Leu Gly Thr
                165                 170                 175

Lys Met Ile Glu Ser Leu Thr Lys Asp Lys Val Gln Ala Gly Asp Val
            180                 185                 190

Ile Thr Ile Asp Lys Ala Thr Gly Lys Ile Ser Lys Leu Gly Arg Ser
        195                 200                 205

Phe Thr Arg Ala Arg Asp Tyr Asp Ala Met Gly Ser Gln Thr Lys Phe
    210                 215                 220

Val Gln Cys Pro Asp Gly Glu Leu Gln Lys Arg Lys Glu Val Val His
225                 230                 235                 240

Thr Val Ser Leu His Glu Ile Asp Val Ile Asn Ser Arg Thr Gln Gly
                245                 250                 255

Phe Leu Ala Leu Phe Ser Gly Asp Thr Gly Glu Ile Lys Ser Glu Val
            260                 265                 270

Arg Glu Gln Ile Asn Ala Lys Val Ala Glu Trp Arg Glu Glu Gly Lys
        275                 280                 285

Ala Glu Ile Ile Pro Gly Val Leu Phe Ile Asp Glu Val His Met Leu
    290                 295                 300

Asp Ile Glu Ser Phe Ser Phe Leu Asn Arg Ala Leu Glu Ser Asp Met
305                 310                 315                 320

Ala Pro Val Leu Ile Met Ala Thr Asn Arg Gly Ile Thr Arg Ile Arg
                325                 330                 335

Gly Thr Ser Tyr Gln Ser Pro His Gly Ile Pro Ile Asp Leu Leu Asp
            340                 345                 350

Arg Leu Leu Ile Val Ser Thr Thr Pro Tyr Ser Glu Lys Asp Thr Lys
        355                 360                 365

Gln Ile Leu Arg Ile Arg Cys Glu Glu Glu Asp Val Glu Met Ser Glu
    370                 375                 380

Asp Ala Tyr Thr Val Leu Thr Arg Ile Gly Leu Glu Thr Ser Leu Arg
385                 390                 395                 400

Tyr Ala Ile Gln Leu Ile Thr Ala Ala Ser Leu Val Cys Arg Lys Arg
                405                 410                 415

Lys Gly Thr Glu Val Gln Val Asp Asp Ile Lys Arg Val Tyr Ser Leu
            420                 425                 430

Phe Leu Asp Glu Ser Arg Ser Thr Gln Tyr Met Lys Glu Tyr Gln Asp
        435                 440                 445

Ala Phe Leu Phe Asn Glu Leu Lys Gly Glu Thr Met Asp Thr Ser
    450                 455                 460

<210>    11
<211>    129
<212>    PRT
<213>    Homo sapiens

<400>    11
Met Ser Gly Arg Gly Lys Gln Gly Gly Lys Ala Arg Ala Lys Ala Lys
1               5                   10                  15

Ser Arg Ser Ser Arg Ala Gly Leu Gln Phe Pro Val Gly Arg Val His
            20                  25                  30

Arg Leu Leu Arg Lys Gly Asn Tyr Ala Glu Arg Val Gly Ala Gly Ala
        35                  40                  45

Pro Val Tyr Met Ala Ala Val Leu Glu Tyr Leu Thr Ala Glu Ile Leu
    50                  55                  60

Glu Leu Ala Gly Asn Ala Ala Arg Asp Asn Lys Lys Thr Arg Ile Ile
65                  70                  75                  80

Pro Arg His Leu Gln Leu Ala Ile Arg Asn Asp Glu Glu Leu Asn Lys
                85                  90                  95

Leu Leu Gly Lys Val Thr Ile Ala Gln Gly Gly Val Leu Pro Asn Ile
            100                 105                 110

Gln Ala Val Leu Leu Pro Lys Lys Thr Glu Ser His Lys Ala Lys Ser
        115                 120                 125

Lys

## Description

### [Technical Field]

The present invention relates to a composition for predicting a response to standard neoadjuvant chemoradiotherapy and a prognosis after the standard therapy in rectal cancer, or a method and a composition for predicting extremely poor risk patients after the standard therapy in rectal cancer.

### [Technical Field]

Rectal cancer is the most common cancer occurring in the intestines, but it is easy to detect and treat early and has a high cure rate. Rectal cancer has a higher incidence after middle age. Rectal cancer may originate from rectal polyps. Symptoms are similar to those of rectal catarrh, and may include foul-smelling bloody stool. Mucus discharge and frequent urges to have a bowel movement after defecation are observed. In addition, symptoms of rectal stenosis include a feeling of heaviness around the rectum and anus, stubborn constipation, stool like rabbit droppings and thinning of poop thickness, and as the symptoms progress, fecal incontinence occurs due to sphincter insufficiency.

Upon palpation, a lumpy, hard tumor in the rectum is palpable. A definite diagnosis may be received by directly examining the tumor with a proctoscope, excising the tumor and performing a speculum examination. When surgery is performed at an early stage of development, which is confined to the rectal wall, the surgical outcome is extremely good. When radical surgery is impossible, an artificial anus is made for defecation, and radium and high-pressure radiation are irradiated locally. In some cases, even with this treatment, patients can survive up to 10 years.

Although rectal cancer and colon cancer are morphologically similar, their treatment methods are different. While colon cancer is treated with adjuvant chemotherapy after curative surgery, rectal cancer is treated with preoperative chemoradiotherapy, followed by curative surgery.

Since a colon cancer molecular subtype classifier is largely determined by the amount of interstitial tissue in the tumor mass, a colon cancer subtype classifier may not be applied to rectal cancer which is generally diagnosed with a small pre-treated biopsy specimen. That is, colon cancer is classified into four molecular subtypes based on gene expression patterns, and it was proven that CMS4 molecular subtype has a poor prognosis and is resistant to existing anticancer drugs. Since the CMS4 molecular subtype is determined by the ratio of fibroblasts in cancer tissue, in the case of rectal cancer, which is diagnosed with a small biopsy tissue before treatment and immediately treated without surgery, the proportion of fibroblasts is unknown, so it is impossible to apply the method of molecular subtyping for colon cancer.

Therefore, rectal cancer requires biomarkers that provide clinical usefulness with respect to the response, recurrence rate and survival rate to intrinsic preoperative chemoradiotherapy for rectal cancer, different from colon cancer.

### [Disclosure]

### [Technical Problem]

The present invention is directed to providing a biomarker composition that can accurately and simply predict a therapeutic response to anticancer therapy or a prognosis after the anticancer therapy.

The present invention is also directed to providing a composition for predicting a prognosis, which can accurately and simply predict a therapeutic response to anticancer therapy or a prognosis after the anticancer therapy.

The present invention is also directed to providing a kit that can accurately and simply predict a therapeutic response to anticancer therapy or a prognosis after the anticancer therapy.

The present invention is also directed to providing a method of providing information, which can accurately and simply predict a therapeutic response to anticancer therapy or a prognosis after the anticancer therapy.

The present invention is also directed to providing a device for predicting a therapeutic response to anticancer therapy or a prognosis after the anticancer therapy.

The present invention is also directed to providing a biomarker composition that identifies a target group for total neoadjuvant therapy through the analysis of cancer tissue obtained by biopsy for diagnosis prior to the initiation of rectal cancer treatment.

The present invention is also directed to providing a composition that identifies a target group for total neoadjuvant therapy through the analysis of cancer tissue obtained by biopsy for diagnosis prior to the initiation of rectal cancer treatment.

The present invention is also directed to providing a kit that identifies a target group for total neoadjuvant therapy through the analysis of cancer tissue obtained by biopsy for diagnosis prior to the initiation of rectal cancer treatment.

The present invention is also directed to providing a device that identifies a target group for total neoadjuvant therapy through the analysis of cancer tissue obtained by biopsy for diagnosis prior to the initiation of rectal cancer treatment.

However, technical problems to be solved in the present invention are not limited to the above-described problems, and other problems which are not described herein will be fully understood by those of ordinary skill in the art from the following descriptions.

### [Technical Solution]

Hereinafter, the present invention will be described with reference to various embodiments. In the following description, for full understanding of the present invention, various specific details, for example, specific forms, compositions and processes will be described. However, certain embodiments may be implemented without one or more of the specific details, or in combination with other known methods and forms. In another example, known processes and manufacturing techniques are not described in particular detail so as to not unnecessarily obscure the present invention. Throughout the specification, the reference to "one embodiment" or "embodiment" means that a specific feature, form, composition or property described in conjunction with the embodiment is included in one or more embodiments of the present invention. Therefore, the context of the "one embodiment" or "embodiment" expressed in various locations throughout the specification does not necessarily represent the same embodiment of the present invention. In addition, a specific feature, form, composition or property may be combined by any suitable manner in one or more embodiments.

Unless specifically defined otherwise, all scientific and technical terms used herein have the same meaning as conventionally understood by those of ordinary skill in the art to which the present invention belongs.

In one aspect of the present invention, the present invention provides a biomarker composition for predicting a therapeutic response to anticancer therapy or a prognosis after anticancer therapy, or identifying a target patient for neoadjuvant therapy prior to anticancer therapy in cancer patients, the composition including at least one gene of a first molecular subtype and a second molecular subtype or a protein encoded thereby.

In the present invention, the first molecular subtype may include one or more types of genes selected from PMP2, AGTR1, PLCXD3, TCEAL6, ANKRD1 and ARHGAP26-AS1.

The "PMP2" gene used herein is a gene encoding Myelin P2 protein (PMP2 protein), and the PMP2 protein is a component of peripheral nervous system (PNS) myelin, and central nervous system (CNS) myelin, which is a constituent of P2. As a structural protein, P2 is considered to stabilize the myelin membranes, and may be involved in lipid transport in Schwann cells. In the present invention, the PMP2 protein may consist of an amino acid sequence represented by SEQ ID NO: 1, but the present invention is not limited thereto.

The "AGTR1" gene used herein is a gene encoding Angiotensin II receptor type 1 (AGTR1) protein, which is the most characteristic angiotensin receptor, has a vasoconstrictive effect and regulates aldosterone secretion. In the present invention, the AGTR1 protein may consist of an amino acid sequence represented by SEQ ID NO: 2, but the present invention is not limited thereto.

The "PLCXD3" gene used herein is phosphatidylinositol-specific phospholipase C, X domain-containing 1, present in a pseudoautosomal region (PAR), and a gene encoding PI-PLC X domain-containing protein 3 (PLCXD3). In the present invention, the PLCXD3 may consist of an amino acid sequence represented by SEQ ID NO: 3, but the present invention is not limited thereto.

The "TCEAL6" gene used herein encodes Transcription elongation factor A (SII)-like 6 (TCEAL6) or ankyrin repeat domain-containing protein 1, and may be involved in transcription regulation. In the present invention, the TCEAL6 or ankyrin repeat domain-containing protein 1 may consist of an amino acid sequence represented by SEQ ID NO: 4, but the present invention is not limited thereto.

The "ANKRD1" gene used herein encodes Ankyrin repeat domain-containing protein 1 (ANKRD1), is highly expressed in the heart and the skeletal muscle, and increased in expression level under a stress condition. In the present invention, the ANKRD1 may consist of an amino acid sequence represented by SEQ ID NO: 5, but the present invention is not limited thereto.

The term "ARHGAP26-AS1" gene used herein is an RNA gene associated with the IncRNA class, and may be represented by SEQ ID NO: 6, but the present invention is not limited thereto.

In one embodiment of the present invention, the first molecular subtype may further include one or more types of genes selected from the group consisting of GTF2IP1, TBC1D3L, BLOC1S5-TXNDC5, MIR4477B, HIST2H3C, HNRNPA1P33, CTAGE8, GOLGA8K, KRT222, LOC440434, C10orf131, PGM5-AS1, ACADL, PGM5P3-AS1, LOC101929607, KIAA0408, PLGLB2, ZNF676, KIAA2022, SEMA3E, PLCXD3, NLGN1, SLITRK4, GAS1RR, TCEAL2, LOC642131, LONRF2, GRIN2A, ADAMTS9-AS1, LOC644838, LOC100507387, FAM35BP, EPHA6, MIR186, LINC01266, FAM47E-STBD1, LINC01489, TVP23C-CDRT4, FAM133A, NEXN, LGI1, OR7E12P, MIR3911, MYH8, ZNF728, BCHE, CCDC144B, LINC01537, LOC101928509, KCTD8, LOC100507073, ARHGEF18, BVES-AS1, LINGO2, SCN7A, GRIA2, LINC00504, LINC01352, MIR133A1HG, SCN9A, HLX-AS1, LOC100506289, FILIP1, MEIS1-AS2, FGF13-AS1, HCG23, PLN, RANBP3L, SPOCK3, PCDH10, LCN10, COL25A1, MEF2C-AS1, ATP2B2, CDH19, ADIPOQ, CRP, ALB, OTOP2, MYOC, FGL1, ATP1A2, CHRM2, PCSK2, SLITRK3, GPM6A, HAND2-AS1, RIMS4, NRXN1, PDZRN4, KRT24, ANGPTL1, TRARG1, MYH11, CASQ2, NGB, LOC101928731, KCNA1, SLC5A7, PMP2, CTNNA3, OGN, SYT4, FABP4, ADH1B, ADCYAP1R1, PI16, GCG, HP, CADM2, MYH2, CLVS2, MAMDC2, FRMPD4, CA1, FAM180B, CMA1, SERTM1, KCNB1, NEXMIF, GC, PLP1, APCS, SLC17A8, ANGPTL7, SYNM, PHOX2B, AGTR1, C7, ST8SIA3, LMO3, LDB3, RERGL, ASB5, SGCG, OTOP3, CCBE1, BMP3, HAND1, CADM3, SYNPO2, TMIGD1, NPTX1, ABCA8, NEFL, PLIN4, CD300LG, LEP, MORN5, ECRG4, SFRP1, SLC7A14, SCN2B, FMO2, SORCS1, CLCA4, OMD, VEGFD, STMN4, PTPRZ1, AQP4, SMYD1, SCRG1, ADGRB3, TMEFF2, CNR1, CIDEA, CNTN1, DPP6, HAND2, TCEAL5, FRMD6-AS2, SMIM28, SYT10, NOS1, PLD5, TNNT3, ABCA9, EPHA7, GALR1, RSPO2, NPY2R, CHRDL1, APOC3, FUT9, PRIMA1, LINC00924, TNXB, LOC102724050, NTNG1, CNGA3, AQP8, PGM5, ASTN1, RNF150, ADAMTSL3, LYVE1, ZDHHC22, LRRTM4, RBFOX3, ABCA6, NECAB1, FGG, NEFM, APOB, RIC3, VSTM2A, OLFM3, CILP, LINC00682, NIBAN1, LMOD1, MYOT, ABI3BP, PPP1R1A, WSCD2, FDCSP, HSPB8, KHDRBS2, NSG2, PKHD1L1, CHST9, ZMAT4, POU3F4, LIX1, MUSK, NRK, PGR, AADACL2, CLDN8, ADAMTS9-AS2, METTL24, NRSN1, LOC729558, SCGN, BEST4, SLITRK2, RELN, NPR3, CCN5, CDH10, CA7, LINC02268, SPIB, ABCB5, CNTNAP4, PTCHD1, UGT2B4, ANKS1B, LINC01829, DPT, MGAT4C, MYT1L, CPEB1, ERICH3, SORCS3, CYP1B1, LINC02023, SALL3, ANK2, PRELP, ART4, PIRT, MYLK, C1QTNF7, LINC01798, DCLK1, DES, KCNC2, CNN1, BRINP3, FAM135B, PYY, GAP43, NAP1L2, ACSM5, THBS4, HTR2B, PYGM, IGSF10, TAFA4, KRTAP13-2, VIT, LRAT, LRRC3B, TMOD1, EPHA5, IRX6, PCDH11X, SLC4A4, HRK, RBM20, LOC283856, LINC00507, ZBTB16, PRG4, APOA2, ASPA, ANXA8L1, CLCNKB, SERTM2, GABRG2, SLC6A2, ZFHX4, MMRN1, STUM, PCOLCE2, DIRAS2, XKR4, SFTPA1, GNAO1, LVRN, DAO, TMEM100, ANGPTL5, LINC01505, SST, HEPACAM, KCNK2, HRG, MFAP5, LINCO2544, RORB, FGF14, CP, MIR8071-1, NEUROD1, MYOCD, CNTN2, SCARA5, CAVIN2, LRRC4C, TCF23, MS4A12, C14orf180, PCDH9, PENK, CARTPT, HPCAL4, ZNF716, PCP4L1, CLEC3B, MYOM1, CCDC160, CA2, GFRA1, LOC107986321, LOC101928134, FHL1, NALCN, MAS1L, MS4A1, PEG3, SFTPC, POPDC2, GPRACR, SLIT2, TRDN, LINC02185, SCNN1G, SNAP25, MAGEB2, ACTG2, MEOX2, C8orf88, ATP2B3, TNS1, GPR119, ZNF385B, SFRP2, KCNQ5, KCNMA1, STON1-GTF2A1L, LIFR, ELAVL4, ADRA1A, ATCAY, LINC01474, FGF10, PIK3C2G, SLC13A5, NUDT10, CCDC169, STMN2, AVPR1B, MAB21L1, MASP1, LINC02408, VXN, PGM5P4-AS1, SNAP91, LRCH2, ISM1, NOVA1, NEGR1, SPHKAP, LINC01697, SHISAL1, CDKN2B-AS1, CR2, MYO3A, AFF3, MROH2B, P2RX2, KIF1A, LINC02015, IGSF11, SV2B, ARPP21, SYT6, GABRA5, EVX2, COL19A1, FGFBP2, FAM106A, VGLL3, KCNT2, PTGIS, EBF2, CTSG, CACNA2D1, B3GALT5-AS1, GUCA2B, UNC80, NETO1, GPR12, LOC105378318, PLIN1, RGS22, SLC30A10, TMEM35A, TACR1, AICDA, MSRB3, NRG3, PLAAT5, CT45A10, LINC01446, TLL1, CLEC4M, DDR2, MAB21L2, MPPED2, CALN1, MICU3, BVES, LOC107986400, DHRS7C, KERA, MAPK4, CDO1, PROKR1, PAPPA2, KCNMB1, P2RY12, MAGEE2, FLNC, GDF6, NBEA, BHMT2, CPXM2, NTRK3, TENM1, RNF180, MRGPRE, CCDC158, PRDM6, RGS13, PAK3, MGP, UGT2B10, and PTPRQ, but the present invention is not limited thereto.

In another embodiment of the present invention, the first molecular subtype may further include one or more types of genes selected from the group consisting of ACADL, ADAMTS9-AS1, ARHGEF18, BCHE, BLOC1S5-TXNDC5, BVES-AS1, C10orf131, CCDC144B, CDH19, CTAGE8, EPHA6, FAM133A, FAM35BP, FAM47E-STBD1, FILIP1, GAS1RR, GOLGA8K, GRIA2, GRIN2A, GTF2IP1, HCG23, HIST2H3C, HLX-AS1, HNRNPA1P33, KCTD8, KIAA0408, KIAA2022, KRT222, LGI1, LINC00504, LINC01266, LINC01352, LINC01489, LINC01537, LINGO2, LOC100507073, LOC100507387, LOC101928509, LOC101929607, LOC440434, LOC642131, LOC644838, LONRF2, MEIS1-AS2, MIR133A1HG, MIR186, MIR3911, MIR4477B, MYH8, NEXN, NLGN1, OR7E12P, PCDH10, PGM5-AS1, PGM5P3-AS1, PLCXD3, PLGLB2, PLN, RANBP3L, SCN7A, SCN9A, SEMA3E, SLITRK4, SYT4, TBC1D3L, TCEAL2, TVP23C-CDRT4, ZNF676, and ZNF728, but the present invention is not limited thereto.

In still another embodiment of the present invention, the first molecular subtype may further include one or more types of genes selected from the group consisting of AADACL2, ABCA6, ABCA8, ABCA9, ABCB5, ABI3BP, ACADL, ACSM5, ACTG2, ADAMTS9-AS1, ADAMTS9-AS2, ADAMTSL3, ADCYAP1R1, ADGRB3, ADH1B, ADIPOQ, ADRA1A, AFF3, AGTR1, AICDA, ALB, ANGPTL1, ANGPTL5, ANGPTL7, ANK2, ANKS1B, ANXA8L1, APOA2, APOB, APOC3, AQP4, AQP8, ARPP21, ART4, ASB5, ASPA, ASTN1, ATCAY, ATP1A2, ATP2B2, ATP2B3, AVPR1B, B3GALT5-AS1, BCHE, BEST4, BHMT2, BLOC1S5-TXNDC5, BMP3, BRINP3, BVES, BVES-AS1, C140rf180, C1QTNF7, C7, C8orf88, CA1, CA2, CA7, CACNA2D1, CADM2, CADM3, CALN1, CARTPT, CASQ2, CAVIN2, CCBE1, CCDC144B, CCDC158, CCDC160, CCDC169, CCN5, CD300LG, CDH10, CDH19, CDKN2B-AS1, CDO1, CHRDL1, CHRM2, CHST9, CIDEA, CILP, CLCA4, CLCNKB, CLDN8, CLEC3B, CLEC4M, CLVS2, CMA1, CNGA3, CNN1, CNR1, CNTN1, CNTN2, CNTNAP4, COL19A1, CP, CPEB1, CPXM2, CR2, CRP, CTNNA3, CTSG, CYP1B1, DAO, DCLK1, DDR2, DES, DHRS7C, DIRAS2, DPP6, DPT, EBF2, ECRG4, ELAVL4, EPHA5, EPHA6, EPHA7, ERICH3, EVX2, FABP4, FAM106A, FAM133A, FAM135B, FAM180B, FDCSP, FGF10, FGF13-AS1, FGF14, FGFBP2, FGG, FGL1, FHL1, FILIP1, FLNC, FMO2, FRMD6-AS2, FRMPD4, FUT9, GABRA5, GABRG2, GALR1, GAP43, GAS1RR, GC, GCG, GDF6, GFRA1, GNAO1, GPM6A, GPR119, GPR12, GPRACR, GRIA2, GRIN2A, GTF2IP1, GUCA2B, HAND1, HAND2, HAND2-AS1, HEPACAM, HP, HPCAL4, HRG, HRK, HSPB8, HTR2B, IGSF10, IGSF11, IRX6, ISM1, KCNA1, KCNB1, KCNC2, KCNK2, KCNMA1, KCNMB1, KCNQ5, KCNT2, KCTD8, KERA, KHDRBS2, KIAA0408, KIF1A, KRT222, KRT24, KRTAP13-2, LCN10, LDB3, LEP, LGI1, LIFR, LINC00504, LINC00507, LINC00682, LINC00924, LINC01266, LINC01352, LINC01474, LINC01505, LINC01697, LINC01798, LINC01829, LINC02015, LINC02023, LINC02185, LINC02268, LINC02408, LINC02544, LIX1, LMO3, LMOD1, LOC100506289, LOC101928731, LOC102724050, LOC107986321, LOC283856, LOC440434, LOC729558, LONRF2, LRAT, LRCH2, LRRC3B, LRRC4C, LRRTM4, LVRN, LYVE1, MAB21L1, MAB21L2, MAGEE2, MAMDC2, MAPK4, MASP1, MEF2C-AS1, MEOX2, METTL24, MFAP5, MGAT4C, MGP, MICU3, MIR133A1HG, MIR8071-1, MMRN1, MORN5, MPPED2, MRGPRE, MS4A1, MS4A12, MSRB3, MUSK, MYH11, MYH2, MYLK, MYO3A, MYOC, MYOCD, MYOM1, MYOT, MYT1L, NALCN, NAP1L2, NBEA, NECAB1, NEFL, NEFM, NEGR1, NETO1, NEUROD1, NEXMIF, NEXN, NGB, NIBAN1, NLGN1, NOS1, NOVA1, NPR3, NPTX1, NPY2R, NRG3, NRK, NRSN1, NRXN1, NSG2, NTNG1, NTRK3, NUDT10, OGN, OLFM3, OMD, OTOP2, OTOP3, P2RX2, P2RY12, PAK3, PAPPA2, PCDH10, PCDH11X, PCDH9, PCOLCE2, PCP4L1, PCSK2, PDZRN4, PEG3, PENK, PGM5, PGM5-AS1, PGM5P4-AS1, PGR, PHOX2B, PI16, PIK3C2G, PIRT, PKHD1L1, PLAAT5, PLCXD3, PLD5, PLIN1, PLIN4, PLN, PLP1, PMP2, POPDC2, POU3F4, PPP1R1A, PRDM6, PRELP, PRG4, PRIMA1, PROKR1, PTCHD1, PTGIS, PTPRQ, PTPRZ1, PYGM, PYY, RANBP3L, RBFOX3, RBM20, RELN, RERGL, RGS13, RGS22, RIC3, RIMS4, RNF150, RNF180, RORB, RSPO2, SCARA5, SCGN, SCN2B, SCN7A, SCN9A, SCNN1G, SCRG1, SEMA3E, SERTM1, SERTM2, SFRP1, SFRP2, SFTPA1, SGCG, SHISAL1, SLC13A5, SLC17A8, SLC30A10, SLC4A4, SLC5A7, SLC6A2, SLC7A14, SLIT2, SLITRK2, SLITRK3, SLITRK4, SMIM28, SMYD1, SNAP25, SNAP91, SORCS1, SORCS3, SPHKAP, SPIB, SPOCK3, SST, ST8SIA3, STMN2, STMN4, STON1-GTF2A1L, STUM, SV2B, SYNM, SYNPO2, SYT10, SYT4, SYT6, TACR1, TAFA4, TCEAL2, TCEAL5, TCF23, TENM1, THBS4, TLL1, TMEFF2, TMEM100, TMEM35A, TMIGD1, TMOD1, TNNT3, TNS1, TNXB, TRARG1, TRDN, UGT2B10, UGT2B4, UNC80, VEGFD, VGLL3, VIT, VSTM2A, VXN, WSCD2, XKR4, ZBTB16, ZDHHC22, ZFHX4, ZMAT4, ZNF385B, ZNF676, and ZNF728, but the present invention is not limited thereto.

In the present invention, the second molecular subtype may include one or more types of genes selected from PGP, SLC26A3, HIST1H4C, RUVBL2, RAB19, HIST2H2AC, and SNORD69.

The "PGP" gene used herein is a gene encoding P-glycoprotein 1 (PGP), which is known as multidrug-resistance protein 1 (MDR1), ATP-binding cassette sub-family B member 1 (ABCB1) or cluster of differentiation 243 (CD243), and is an important protein of the cell membrane that pumps many foreign substances outside cells or an ATP-dependent efflux pump with broad substrate specificity. In the present invention, the PGP may consist of an amino acid sequence represented by SEQ ID NO: 7, but the present invention is not limited thereto.

The "SLC26A3" gene used herein encodes a chloride anion exchanger (down-regulated in adenoma; DRA), which is a SAT-family anion exchanger and transports sulfates and other anions in the intestinal mucosa. In the present invention, the chloride anion exchanger may consist of an amino acid sequence represented by SEQ ID NO: 8, but the present invention is not limited thereto.

The "HIST1H4C" gene used herein encodes Histone H4 without an intron, and the Histone H4 forms one of the four key histones such as H1, H2, H3 and H4, which form a histone octamer. In the present invention, the histone H4 may consist of an amino acid sequence represented by SEQ ID NO: 9, but the present invention is not limited thereto.

The "RAB19" gene used herein encodes RuB-1-like 2 protein, and is a homolog of the bacterial RubB gene. In the present invention, the RuB-1-like 2 protein may consist of an amino acid sequence represented by SEQ ID NO: 10, but the present invention is not limited thereto.

The "HIST2H2AC" gene used herein encodes histone H2A type 2-C (HIST2H2AC) protein, which forms one of the four key histones such as H1, H2, H3, and H4, which form a histone octamer, and the linker histone H1 interacts with linker DNA between nucleosomes to compress the chromatin into a higher-order structure. In the present invention, the histone H2A type 2-C may consist of an amino acid sequence represented by SEQ ID NO: 11, but the present invention is not limited thereto.

The "SNORD69" used herein belongs to the C/D family of snoRNA, and is a human orthologue of mouse MBII-210.

In one embodiment of the present invention, the second molecular subtype may further include one or more types of genes selected from TMEM160, TRAPPC5, FEZF2, SNHG25, C4orf48, SNORD38A, PRR7, EIF3IP1, MIR3661, LOC440311, SNORD30, PDF, TPGS1, CTU1, FAM173A, gene, PRSS2, MIR6807, SPRR2E, ADAT3, HIST1H4L, CDH16, GALR3, DEFA5, FOXI3, SMCR5, LIN28B, MESP1, MIR203A, RAET1E-AS1, ANP32D, BOD1L2, SMARCA5-AS1, RNU4-1, RNU5E-1, CCDC85B, ONECUT3, FAM230C, DBET, UBE2NL, MIR4479, CSNK1A1L, BHLHA9, PITPNM2-AS1, SNORA36A, PRSS56, SPRR2G, MAGEA10, GPR25, SLC32A1, LOC101927972, LKAAEAR1, CT83, HES4, TMEM238, RPRML, SNORD41, PTGER1, ITLN2, WBP11P1, MIR324, RNU5A-1, HLA-L, PNMA5, MIR6891, MT4, MIR6858, HIST1H4A, SHISAL2B, LOC101928372, RNU6ATAC, SKOR2, MIR4737, NACA2, FRMD8P1, REG3A, LOC101927795, MIR4767, RNU5B-1, DDC-AS1, PCSK1N, SNORD3B-2, LOC344967, SNORD48, ZAR1, MIR4665, RPL29P2, RNY1, PTTG3P, GJD3, SBF1P1, CLMAT3, KCNE1B, LRRC26, LCN15, HBA1, IGFBP7-AS1, MIR4449, MIR8075, NOXO1, and RNA5S9, but the present invention is not limited thereto.

In another embodiment of the present invention, the second molecular subtype may further include one or more types of genes selected fromC4orf48, CTU1, EIF3IP1, FAM173A, FEZF2, LOC440311, MIR3661, NOXO1, PDF, PRR7, SNHG25, SNORD30, SNORD38A, TMEM160, TPGS1, and TRAPPC5, but the present invention is not limited thereto.

In still another embodiment of the present invention, the second molecular subtype may further include one or more types of genes selected from ADAT3, ANP32D, BHLHA9, BOD1L2, C4orf48, CCDC85B, CDH16, CLMAT3, CSNK1A1L, CTU1, DBET, DDC-AS1, DEFA5, EIF3IP1, FAM173A, FEZF2, FOXI3, FRMD8P1, GALR3, GJD3, GPR25, HBA1, HES4, HIST1H4A, HIST1H4L, HLA-L, IGFBP7-AS1, ITLN2, KCNE1B, LCN15, LKAAEAR1, LOC101927795, LOC101927972, LOC101928372, LOC344967, LRRC26, MAGEA10, MESP1, MIR203A, MIR324, MIR3661, MIR4449, MIR4479, MIR4665, MIR4737, MIR4767, MIR6807, MIR6858, MIR6891, MIR8075, NACA2, NOXO1, ONECUT3, PCSK1N, PDF, PITPNM2-AS1, PNMA5, PRR7, PRSS2, PRSS56, PTGER1, PTTG3P, REG3A, RNA5S9, RNU4-1, RNU5A-1, RNU5B-1, RNU5E-1, RNU6ATAC, RNY1, RPL29P2, RPRML, SBF1P1, SHISAL2B, SKOR2, SLC32A1, SMARCA5-AS1, SMCR5, SNHG25, SNORA36A, SNORD30, SNORD38A, SNORD3B-2, SNORD41, SNORD48, TMEM160, TMEM238, TPGS1, TRAPPC5, UBE2NL, WBP11P1, and ZAR1, but the present invention is not limited thereto.

In the present invention, the anticancer therapy may be chemotherapy, radiation therapy, surgical treatment or a combination thereof, in which the chemotherapy or radiation therapy is preferably preceding anticancer therapy, and more preferably the anticancer therapy may be standard neoadjuvant chemoradiotherapy or surgical treatment after standard neoadjuvant chemoradiotherapy, but the present invention is not limited thereto.

The "neoadjuvant anticancer therapy" is an anticancer therapy performed prior to local surgery with curative intent or radiation therapy. The neoadjuvant anticancer therapy is usually performed on a patient who cannot receive total resection since cancer has spread to surrounding areas, and may preserve major organs by reducing the size and range of a primary tumor to reduce the scope of surgery in colon cancer, rectal cancer, head and neck tumors, osteosarcoma, anal cancer, breast cancer or the like.

The "chemotherapy" used herein is also referred to as CTX, and as a type of standardized chemotherapy that uses one or more anticancer agents, may be used for the purpose of treatment or aimed at extending life or reducing symptoms.

The "radiation therapy" used herein is a treatment that kills cancer cells using high-energy radiation, and may include external radiation therapy or brachytherapy, but the present invention is not limited thereto. The radiation may be included without limitation as long as it is a phenomenon in which energy propagates through space or a material that mediates such propagation.

The "therapeutic response" used herein refers to the degree of effectiveness of treatment for a subject, and preferably, a subject as a cancer patient. For example, the term "increased response" or "good response" when used in connection with the treatment of a cancer patient may refer to an increase in the effectiveness of treatment when measured using any method known in the art. As another example, the response of a cancer patient to treatment may be characterized by a complete or partial response. In still another example, the increased response of a cancer patient to treatment may be characterized by an overall survival rate, disease-free survival, a target response rate, time to tumor progression, progression-free survival or time to treatment failure.

The "prognosis" used herein refers to an act of predicting the course of a disease and the outcome of death or survival, and the "prognostic prediction" refers to an act of predicting the course of a disease and the outcome of death or survival. The prognosis or prognostic prediction may be interpreted to mean any act of predicting the course of a disease before/after treatment by considering a patient's condition since the course of a disease may vary according to a physiological or environmental condition of the patient. In addition, the prognosis may refer to the progression of a disease, such as migration and infiltration of cancer into tissue, metastasis to different tissue and death from a disease, and whether the disease is completely cured. According to the purpose of the present invention, the prognosis refers to the progression or rectal cancer survival prognosis of rectal cancer patients. According to the purpose of the present invention, the prognosis may mean the identification of a pre-metastatic cancer or metastatic cancer status, the determination of a cancer stage, or the determination of the therapeutic response to anticancer treatment, but the present invention is not limited thereto.

In the present invention, the prognosis may be pathologic complete response (pCR), recurrence, metastasis or death after the anticancer therapy.

In one embodiment of the present invention, the prognosis may refer to a case showing distant metastasis or a mortality of 60% or more within 3 years after surgical treatment following the anticancer therapy, and preferably, surgical treatment after standard neoadjuvant chemoradiotherapy, but the present invention is not limited thereto.

In another embodiment of the present invention, the prognosis may refer to a case showing distant metastasis or a mortality of 60% or more within 3 years after pCR is achieved as a result of the anticancer therapy, and preferably, surgical treatment after standard neoadjuvant chemoradiotherapy, but the present invention is not limited thereto.

The "pathologic complete response (pCR)" refers to the case in which cancer cells are not found during pathological examination of rectal tissue removed by total resection after neoadjuvant chemoradiotherapy.

The "biomarker" used herein is a measurable indicator of some biological state or condition using cells, blood vessels, proteins, DNA, RNA or metabolites in the body, and the National Institutes of Health (NIH) has defined a biomarker as "a characteristic that is objectively measured and evaluated as an indicator of normal biological processes, pathogenic processes, or pharmacological responses to therapeutic intervention." That is, in the case of a specific disease or cancer, the biomarker is a marker that can distinguish between normal and pathological conditions, predict a therapeutic response, and objectively measure it. Accordingly, the biomarker must serve to objectively measure and evaluate a normal biological process, the progression of a disease, and drug responsiveness in a treatment method. According to utilization, there are a target marker that confirms the presence of a drug target according to availability, a diagnostic marker that diagnoses the presence or absence of a disease, a predictive marker that can distinguish a responder and a non-responder to a specific drug, a surrogate marker that can monitor a drug treatment effect, and a prognostic biomarker that indicates the prognosis of a disease.

The "tumor" or "cancer" used herein is a disease in which the cell cycle is not controlled and cell division continues, and is classified into a carcinoma and a sarcoma according to the site of occurrence. Carcinomas refer to malignant tumors generated in epithelial cells such as a mucous membrane and skin, and sarcomas refer to malignant tumors generated in non-epithelial cells such as muscle, connective tissue, bone, cartilage and blood vessels.

The cancer used herein may be one or more types of cancer selected from the group consisting of breast cancer, uterine cancer, esophageal cancer, stomach cancer, brain cancer, rectal cancer, colon cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma, and leukemia, and preferably rectal cancer.

In another aspect of the present invention, the present invention provides a composition for predicting a therapeutic response to anticancer therapy or a prognosis after anticancer therapy, or identifying a target patient for neoadjuvant therapy prior to anticancer treatment in cancer patients, the composition including: an agent that measures the expression level of at least one gene of a first molecular subtype and a second molecular subtype or a protein encoded thereby.

In the present invention, the descriptions on the first molecular subtype and the second molecular subtype overlap with those described in the biomarker composition, and will be omitted to avoid excessive complexity of the specification.

The "expression" or "expression level" used herein may include transcription and translation. The increase in expression level may be caused by various methods including, for example, an increase in the number of polypeptide-encoding genes, an increase in gene transcription (e.g., by arranging a gene under the control of a constitutive promoter), the increase in gene translation, the knockout of a competing gene, or a combination thereof and/or in combination with other methods. The decrease in expression may be caused by a decrease in the number of genes, a decrease in gene transcription, and the expression of a completing gene. In addition, the expression level of the first molecular subtype or the second molecular subtype may be normalized by comparison with the expression level of a comparative gene.

In the present invention, an agent for measuring the expression level of one or more selected from the first molecular subtype and the second molecular subtype may include one or more selected from the group consisting of an antibody, oligopeptide, ligand, peptide nucleic acid (PNA), and aptamer, which specifically bind to the protein.

The "antibody" used herein refers to a material that specifically binds to an antigen to have an antigen-antibody interaction. For the purpose of the present invention, the antibody refers to an antibody specifically binding to each of the proteins. The antibody of the present invention encompasses a polyclonal antibody, a monoclonal antibody and a recombinant antibody. The antibody may be easily prepared using technology widely known in the art. For example, the polyclonal antibody may be produced by a method widely known in the art, which includes a procedure of injecting an antigen of the protein into an animal and collecting blood from the animal to obtain a serum including antibodies. The polyclonal antibody may be prepared by any animal such as goat, a rabbit, sheep, a monkey, a horse, a pig, a cow or a dog. In addition, the monoclonal antibody may be prepared using a hybridoma method (Kohler and Milstein (1976) European Journal of Immunology 6:511-519) or phage antibody library technology (Clackson et al, Nature, 352:624-628, 1991; Marks et al, J. Mol. Biol., 222:58, 1-597, 1991), which is widely known in the art. The antibody prepared by the method may be separated and purified by gel electrophoresis, dialysis, salting out, ion exchange chromatography or affinity chromatography. In addition, the antibody of the present invention includes not only a complete form with two full-length light chains and two full-length heavy chains, but also a functional fragment of the antibody molecule. The functional fragment of the antibody molecule refers to a fragment having at least an antigen-binding function, and includes Fab, F(ab'), F(ab')₂ or Fv.

The "oligopeptide" used herein may consist of 2 to 20 amino acids, and include a dipeptide, a tripeptide, a tetrapeptide, and a pentapeptide, but the present invention is not limited thereto.

The "peptide nucleic acid (PNA)" used herein refers to an artificially synthesized polymer similar to DNA or RNA, which was first reported by Prof. Nielsen, Egholm, Berg and Buchardt of the University of Copenhagen, Denmark in 1991. DNA has a phosphate-ribose backbone, whereas PNA has a backbone in which repeating N-(2-aminoethyl)-glycine units are linked by peptide bonds, thereby greatly increasing the binding strength to DNA or RNA and stability, and thus is used in molecular biology, diagnostic Assays and antisense therapy. PNA is described in detail in the literature, Nielsen PE, Egholm M, Berg RH, Buchardt O (December 1991), "Sequence-selective recognition of DNA by strand displacement with a thymine-substituted polyamide," Science 254 (5037): 1497-1500.

The "aptamer" used herein is an oligonucleotide or peptide molecule, and the general description of the aptamer is disclosed in detail in the literature, Bock LC et al., Nature 355(6360):5646(1992); Hoppe-Seyler F, Butz K "Peptide aptamers: powerful new tools for molecular medicine," J Mol Med. 78(8):42630(2000); Cohen BA, Colas P, Brent R. "An artificial cell-cycle inhibitor isolated from a combinatorial library," Proc Natl Acad Sci USA. 95(24): 142727(1998).

In the present invention, the agent for measuring the expression level of the first molecular subtype and the second molecular subtype may include one or more selected from the group consisting of a primer, probe and antisense nucleotide, which specifically bind to RNA sequencing (RNAseq) or the gene.

The "primer" used herein is a fragment recognizing a target gene sequence, includes a pair of forward and reverse primers, and preferably, a primer pair providing an analysis result with specificity and sensitivity. Since the nucleic acid sequence of a primer is a sequence that does not match a non-target sequence present in a sample, when the primer is a primer that amplifies only a target gene sequence containing a complementary primer binding site and does not cause non-specific amplification, high specificity may be provided.

The "probe" used herein refers to a material that can specifically bind to a target material to be detected in a sample, and a material that can be used to specifically detect the presence of a target material in a sample by the above-described binding. The type of probe is a material conventionally used in the art, but there is not limitation thereto. The probe is preferably PNA, a locked nucleic acid (LNA), a peptide, a polypeptide, a protein, RNA or DNA, and most preferably PNA. More specifically, the probe is one derived from or similar to a living organism as a biomaterial, or one prepared in vitro, for example, an enzyme, a protein, an antibody, a microorganism, animal/plant cells and organs, neurons, DNA or RNA. DNA may be cDNA, genomic DNA, or an oligonucleotide, RNA may be genomic RNA, mRNA, or an, oligonucleotide, and a protein may be an antibody, an antigen, an enzyme or a peptide.

The "locked nucleic acid (LNA)" used herein refers to a nucleic acid analogue having a 2'-O, 4'-C methylene bridge [J Weiler, J Hunziker and J Hall Gene Therapy (2006) 13, 496.502]. A LNA nucleoside includes common DNA or RNA nucleobases, and may form a base pair according to the Watson-Crick base pairing rule. However, due to 'locking' of a molecule by the methylene bridge, LNA fails to form an ideal shape in Watson-Crick combinations. When LNA is included in a DNA or RNA oligonucleotide, LNA may more rapidly pair with a complementary nucleotide chain, increasing the stability of the double helix. The "antisense" used herein refers to an oligomer having a nucleotide base sequence and an intersubunit backbone, in which an antisense oligomer is hybridized with a target sequence in RNA by the formation of Watson-Crick base pairs to typically allow the formation of mRNA and RNA: oligomer heterodimers in the target sequence. The oligomer may have exact sequence complementarity or approximate complementarity to the target sequence.

Since the sequence information of genes corresponding to the first molecular subtype and the second molecular subtype according to the present invention is known, based on this, a primer, probe or antisense nucleotide specifically binding to the gene may be easily designed by those of ordinary skill in the art, and quantitative analysis is possible by a general RNA sequencing method without a specific design.

In the present invention, the anticancer therapy may be chemotherapy, radiation therapy, surgical treatment, or a combination thereof, and preferably, the chemotherapy or radiation therapy is preceding anticancer therapy, and more preferably, the anticancer therapy may be standard neoadjuvant chemoradiotherapy or surgical treatment after standard neoadjuvant chemoradiotherapy, but the present invention is not limited thereto.

In one embodiment of the present invention, the prognostic prediction may be to predict a survival rate after the anticancer therapy, and preferably, standard neoadjuvant chemoradiotherapy or surgical treatment after standard neoadjuvant chemoradiotherapy, but the present invention is not limited thereto.

In another embodiment of the present invention, the prognostic prediction may be predicting pCR after the anticancer therapy, preferably, standard neoadjuvant chemoradiotherapy or surgical treatment after standard neoadjuvant chemoradiotherapy, but the present invention is not limited thereto.

In still another embodiment of the present invention, the prognostic prediction may be to predict whether cancer recurs after the anticancer therapy, and preferably, standard neoadjuvant chemoradiotherapy or surgical treatment after standard neoadjuvant chemoradiotherapy, but the present invention is not limited thereto.

In yet another embodiment of the present invention, the prognostic prediction may be to predict cancer metastasis after the anticancer therapy, and preferably, surgical treatment after standard neoadjuvant chemoradiotherapy, but the present invention is not limited thereto.

In yet another embodiment of the present invention, the prognostic prediction may be to predict whether distant metastasis occurs or a mortality is 60% or more within 3 years after the anticancer therapy, and preferably, surgical treatment after standard neoadjuvant chemoradiotherapy, but the present invention is not limited thereto.

In yet another embodiment of the present invention, the prognostic prediction may be to predict whether distant metastasis occurs or a mortality is 60% or more within 3 years after pCR is achieved as a result of the anticancer therapy, and preferably, surgical treatment after standard neoadjuvant chemoradiotherapy, but the present invention is not limited thereto.

In the present invention, the cancer may be one or more types of cancer selected from the group consisting of breast cancer, uterine cancer, esophageal cancer, stomach cancer, brain cancer, rectal cancer, colon cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma, and leukemia, and preferably, rectal cancer.

In still another aspect of the present invention, the present invention provides a kit for predicting a therapeutic response to anticancer therapy or a prognosis after anticancer therapy, or identifying a target patient of neoadjuvant therapy before anticancer treatment in cancer patients.

The "kit" used herein refers to a tool for evaluating the expression level of a biomarker by labeling a probe or antibody specifically binding to a biomarker component with a detectable label. The kit may include not only a direct label that directly labels a substance capable of being detected with respect to a probe or antibody by a reaction with a substrate, but also an indirect label to which a marker that develops color by a reaction with another reagent, which is directly-labeled, is conjugated. The kit may include a chromogenic substrate solution that develops color by reaction with the label, a washing solution and other solutions, and may be manufactured to include a reagent component used herein. In the present invention, the kit may be a kit that includes essential elements required for RT-PCR, and may include a test tube, a reaction buffer solution, deoxynucleotides (dNTPs), Taq-polymerase, reverse transcriptase, DNase, a RNase inhibitor, and distilled water as well as primer pairs specific for a marker gene. In addition, the kit may be a kit for detecting a gene for prognostic prediction, which includes essential elements required for a DNA chip. The DNA chip kit may include a substrate on which cDNA corresponding to a gene or a fragment thereof is attached to a probe, and the substrate may include cDNA corresponding to a quantitative control gene or a fragment thereof. The kit of the present invention is not limited as long as it is known in the art.

In the present invention, the kit may be an RT-PCR kit, a DNA chip kit, an ELISA kit, a protein chip kit, a rapid kit or a multiple reaction monitoring (MRM) kit.

The kit of the present invention may further include a composition, solution or device consisting of one or more types of different components, suitable for an analysis method. For example, the kit of the present invention may further include essential elements required for a reverse transcription polymerase chain reaction (RT-PCR). A kit for RT-PCR includes a primer pair specific for a marker protein-coding gene. The primers are nucleotides having a sequence specific for the nucleic acid sequence of the gene, having a length of approximately 7 bp to 50 bp, and more preferably, approximately 10 bp to 30 bp. In addition, the primers may include a primer specific for the nucleic acid sequence of a control gene. Alternatively, the kit for RT-PCR may include a test tube or another suitable container, a reaction buffer solution (pH and magnesium concentrations vary), deoxynucleotides (dNTPs), enzymes such as Taq-polymerase and reverse transcriptase, DNase, RNase inhibitor DEPC-water, and distilled water.

In addition, the kit of the present invention may include essential elements required for a DNA chip. The DNA chip kit may include a substrate to which cDNA or an oligonucleotide, which corresponds to a gene or a fragment thereof, is attached, and a reagent, agent and enzymes for preparing a fluorescence-labeled probe. In addition, the substrate may include cDNA or an oligonucleotide, which corresponds to a control gene or a fragment thereof, is attached.

In addition, the kit of the present invention may include essential elements required for ELISA. The ELISA kit includes an antibody specific for the protein. The antibody is a monoclonal, polyclonal or recombinant antibody, which has high specificity and affinity to a marker protein and almost no cross-reactivity with another protein. In addition, the ELISA kit may include an antibody specific for a control protein. In addition, the ELISA kit may include reagents capable of detecting a bound antibody, such as a labeled secondary antibody, chromophores, enzymes (e.g., conjugated with the antibody) and substrates thereof, or other substances capable of binding to the antibody.

As a scaffold for antigen-antibody interactions in the kit, a nitrocellulose membrane, a PVDF membrane, well plate synthesized of a polyvinyl resin or a polystyrene resin, or a slide glass formed of glass may be used, but the present invention is not limited thereto.

In addition, in the kit of the present invention, a label for a secondary antibody is preferably a conventional chromophore that develops color, and preferably, labels such as fluorescein or a dye such as poly L-lysine-fluorescein isothiocyanate (FITC), or rhodamine-B-isothiocyanate (RITC), horseradish peroxidase (HRP), alkaline phosphatase, colloidal gold, may be used, but the present invention is not limited thereto.

In addition, a chromogenic substrate for inducing color development in the kit of the present invention is preferably used according to a label that develops color, and may be 3,3',5,5'-tetramethyl benzidine (TMB), 2,2'-azino-bis(3-ethylbenzothiazolin-6-sulfonic acid) (ABTS) or o-phenylenediamine (OPD). Here, the chromogenic substrate is more preferably provided in a state of being dissolved in a buffer solution (0.1 M NaAc, pH 5.5). A chromogenic substrate such as TMB may be decomposed by HRP used as a label of a secondary antibody conjugate to generate a chromogenic deposit, and by visually observing the degree of deposition of the chromogenic deposit, the presence of the marker proteins is detected.

In the kit of the present invention, the washing solution preferably includes a phosphate-buffered solution, NaCl and Tween 20, and more preferably, a buffer solution (PBST) consisting of 0.02M phosphate-buffered solution, 0.13M NaCl and 0.05% Tween 20. The washing solution is used to wash a scaffold to which an appropriate amount of a product of reaction of a secondary antibody with an antigen-antibody conjugate after an antigen-antibody interaction is added 3 to 6 times. As a reaction stop solution, a sulfuric acid solution (H₂SO₄) is preferably used.

In one embodiment of the present invention, the kit may be used to diagnose the degree of sensitivity or therapeutic response to anticancer therapy, and the prognosis, stage, possibility of cancer metastasis, possibility of recurrence or survival rate after the treatment.

The cancer of the present invention may be one or more types of cancer selected from the group consisting of breast cancer, uterine cancer, esophageal cancer, stomach cancer, brain cancer, rectal cancer, colon cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma, and leukemia, and preferably, rectal cancer.

In yet another aspect of the present invention, the present invention provides a method of providing information for predicting a therapeutic response to anticancer therapy or a prognosis after anticancer therapy, or identifying a target patient of neoadjuvant therapy prior to anticancer therapy, the method including: measuring the expression level of at least one gene of a first molecular subtype and a second molecular subtype or a protein encoded thereby in a biological sample isolated from a desired subject.

The "subject" used herein is a subject with cancer or a high probability of developing cancer, which may be a subject or cancer patient receiving the anticancer therapy, and include all mammals. Here, examples of the mammals may include a human, non-human primates such as chimpanzees, other apes and monkey species; livestock animals such as cattle, horses, sheet, goats and pigs; domestic animals such as rabbits, dogs and cats; laboratory animals such as rodents including rats, mice, and guinea pigs, but the present invention is not limited thereto.

The "biological sample" used herein refers to any material, biological fluid, tissue or cells, obtained from or derived from a subject, and may include, for example, whole blood, leukocytes, peripheral blood mononuclear cells, buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, a cell extract, and cerebrospinal fluid, and preferably, cancer tissue obtained from a subject before the initiation of treatment.

In the present invention, descriptions of the first molecular subtype and the second molecular subtype overlap with those described in the biomarker composition and thus will be omitted to avoid excessive complexity of the specification.

In the present invention, an agent for measuring the expression level of at least one gene of the first molecular subtype and the second molecular subtype may include one or more selected from the group consisting of a primer, a probe, and an antisense nucleotide, which specifically bind to the gene.

In the present invention, the measurement of the expression level of at least one of the first molecular subtype and the second molecular subtype may be performed by RT-PCR, competitive RT-PCR, real-time RT-PCR, RNase protection assay (RPA), Northern blotting or a DNA chip.

In the present invention, an agent for measuring the expression level of at least one gene of the first molecular subtype and the second molecular subtype or a protein encoded thereby may include one or more selected from the group consisting of an antibody, an oligopeptide, a ligand, PNA, and an aptamer, specifically binding to the protein.

In the method of providing information according to the present invention, descriptions of an antibody, an oligopeptide, a ligand, PNA, and an aptamer overlap with those described above, and thus will be omitted to avoid excessive complexity of the specification.

In the present invention, the measurement of the expression level of a protein encoded by at least one gene of the first molecular subtype and the second molecular subtype may be performed by a protein chip, an immunoassay, ligand binding assay, matrix-assisted laser desorption/ionization time of flight (MALDI-TOF) mass spectrometry, surface-enhanced laser desorption/ionization time of flight (SELDI-TOF) mass spectrometry, radioimmunoassay, radial immunodiffusion, Ouchterlony immunodiffusion assay, Rocket immunoelectrophoresis, tissue immunostaining, complement fixation assay, two-dimensional electrophoresis, liquid chromatography-mass spectrometry (LC-MS), liquid chromatography-mass spectrometry/mass spectrometry (LC-MS/MS), Western blotting or enzyme linked immunosorbent assay (ELISA).

In the present invention, the anticancer therapy may be chemotherapy, radiation therapy, surgical treatment or a combination thereof, preferably, the chemotherapy or radiation therapy is preceding anticancer therapy, and more preferably, the anticancer therapy may be standard neoadjuvant chemoradiotherapy or surgical treatment after standard neoadjuvant chemoradiotherapy, but the present invention is not limited thereto.

In one embodiment of the present invention, the prognostic prediction may be to predict a survival rate after the anticancer therapy, and preferably, surgical treatment after standard neoadjuvant chemoradiotherapy, but the present invention is not limited thereto.

In another embodiment of the present invention, the prognostic prediction may be to predict whether pCR is achieved after the anticancer therapy, and preferably, surgical treatment after standard neoadjuvant chemoradiotherapy, but the present invention is not limited thereto.

In still another embodiment of the present invention, the prognostic prediction may be to predict whether cancer recurs after the anticancer therapy, and preferably, surgical treatment after standard neoadjuvant chemoradiotherapy, but the present invention is not limited thereto.

In yet another embodiment of the present invention, the prognostic prediction may be to predict cancer metastasis after the anticancer therapy, and preferably, surgical treatment after standard neoadjuvant chemoradiotherapy, but the present invention is not limited thereto.

In yet another embodiment of the present invention, predicting that a prognosis is poor may mean a case showing distant metastasis and a mortality rate of 60% or more within 3 years after pCR is achieved as a result of the standard neoadjuvant chemoradiotherapy.

In the present invention, the method may further include confirming the subject's TNM stage, age, sex, pCR or combined information thereof, but the present invention is not limited thereto.

The "TNM stage" used herein is a system for classifying the anatomical degree of a tumor or cancer, and the TNM classification of malignant tumors is a globally accepted standard for classifying the degree of cancer spread. In the TNM stages, T represents the size of a primary tumor and whether it has invaded surrounding tissue, N represents the number of nearby lymph nodes that have cancer, and M represents distant metastasis.

In the present invention, when the first molecular subtype or a protein encoded thereby is expressed in a biological sample isolated from a target subject, or its expression level is higher than a control, it can be predicted that the therapeutic response to the anticancer therapy is low. For example, when the expression level of the first molecular subtype or a protein encoded thereby is higher than that of the control, it can be predicted that the therapeutic response to standard neoadjuvant chemoradiotherapy or the therapeutic response to surgical treatment after standard neoadjuvant chemoradiotherapy is low.

In the present invention, when the first molecular subtype is expressed in a biological sample isolated from a target subject, or its expression level is higher than the control, it can be predicted that the prognosis after anticancer therapy is poor. For example, when the expression level of the first molecular subtype or a protein encoded thereby is higher than that of the control, it can be predicted that a prognosis after standard neoadjuvant chemoradiotherapy or a prognosis of surgical treatment after standard neoadjuvant chemoradiotherapy is poor, and specifically, that a survival rate is low, a recurrence probability is high, or a metastasis probability is high, but the present invention is not limited thereto.

In one embodiment of the present invention, when the expression level of the first molecular subtype measured in a biological sample isolated from a target subject is higher than that of a control, and pCR is not achieved after anticancer therapy, and preferably, standard neoadjuvant chemoradiotherapy, it can be predicted that a prognosis is poor.

In another embodiment of the present invention, when the expression level of the first molecular subtype measured in a biological sample isolated from a target subject is higher than that of a control, and the TNM stage of the subject is T3 or T4, it can be predicted that a prognosis after the anticancer therapy, and preferably, standard neoadjuvant chemoradiotherapy or surgical treatment after standard neoadjuvant chemoradiotherapy is poor.

In still another embodiment of the present invention, when the expression level of the first molecular subtype measured in a biological sample isolated from a target subject is higher than that of a control, and the TNM stage of the subject is N1 or N2, it can be predicted that a prognosis after the anticancer therapy, and preferably, standard neoadjuvant chemoradiotherapy or surgical treatment is poor.

In yet another embodiment of the present invention, when the expression level of the first molecular subtype measured in a biological sample isolated from a target subject is higher than that of a control, and the TNM stage of the subject is T3 or T4, and N1 or N2, it can be predicted that a prognosis after the anticancer therapy, and preferably, standard neoadjuvant chemoradiotherapy or surgical treatment is poor.

Meanwhile, in one embodiment of the present invention, when the expression level of the second molecular subtype measured in a biological sample isolated from a target subject is higher than that of a control, and pCR is not achieved after anticancer therapy, and preferably, standard neoadjuvant chemoradiotherapy, it can be predicted that the prognosis is good.

In another embodiment of the present invention, when the expression level of the second molecular subtype measured in a biological sample isolated from a target subject is higher than that of a control, and pCR is not achieved after anticancer therapy, and preferably, standard neoadjuvant chemoradiotherapy, it can be predicted that a prognosis after the anticancer therapy is good.

In still another embodiment of the present invention, when the expression level of the second molecular subtype measured in a biological sample isolated from a target subject is higher than that of a control, and the TNM stage of the subject is T0, T1 or T2, it can be predicted that a prognosis of anticancer therapy, and preferably, standard neoadjuvant chemoradiotherapy or surgical treatment after standard neoadjuvant chemoradiotherapy is good.

In yet another embodiment of the present invention, when the expression level of the second molecular subtype measured in a biological sample isolated from a target subject is higher than that of a control, and the TNM stage of the subject is N0, it can be predicted that a prognosis after anticancer therapy, and preferably, standard neoadjuvant chemoradiotherapy or surgical treatment is good.

In yet another embodiment of the present invention, when the expression level of the second molecular subtype measured in a biological sample isolated from a target subject is higher than that of a control, and the TNM stage of the subject is T0, T1 or T2, and N0, it can be predicted that a prognosis after anticancer therapy, and preferably, standard neoadjuvant chemoradiotherapy or surgical treatment is bad.

The "control" used herein may be an average value or median of the expression level of the first molecular subtype or the second molecular subtype or a protein encoded thereby in a normal subject, or the expression level of a corresponding gene or protein in a subject with cancer or a high probability of developing cancer, particularly, a subject who has been diagnosed with cancer, but the present invention is not limited thereto.

In the present invention, when the expression level of the first molecular subtype or a protein encoded thereby is higher than the control, a step of performing anticancer therapy may be further included. The anticancer therapy may be chemotherapy, but the present invention is not limited thereto.

In the present invention, when the expression level of the second molecular subtype or a protein encoded thereby is higher than the control, and pCR is not achieved, the step of performing anticancer therapy may be further included. The anticancer therapy may be chemotherapy, but the present invention is not limited thereto.

In the present invention, the cancer may be one or more types of cancer selected from the group consisting of breast cancer, uterine cancer, esophageal cancer, stomach cancer, brain cancer, rectal cancer, colon cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma and leukemia, and preferably, rectal cancer.

In yet another aspect of the present invention, the present invention provides a device for predicting a therapeutic response to anticancer therapy or a prognosis after anticancer therapy, or identifying a target patient of neoadjuvant therapy prior to anticancer therapy, the device includes: a measurement unit for measuring the expression level of at least one gene of a first molecular subtype and a second molecular subtype or a protein encoded thereby in a biological sample isolated from a target subject; and a calculation unit that provides information for predicting a therapeutic response to anticancer therapy for a subject or a prognosis after anticancer therapy from the expression level of at least one gene of the first molecular subtype and the second molecular subtype or a protein encoded thereby.

In the present invention, descriptions of the subjects, biological samples and the measurement of expression levels overlap with those described in the composition for predicting a therapeutic response to the anticancer therapy or a prognosis after anticancer therapy, or identifying a target subject of neoadjuvant therapy prior to anticancer therapy, and thus will be omitted to avoid excessive complexity of the specification.

In addition, in the present invention, the descriptions on the first molecular subtype and the second molecular subtype overlap with those of the biomarker composition, and thus will be omitted to avoid excessive complexity of the specification.

In the present invention, the anticancer therapy may be chemotherapy, radiation therapy, surgical treatment or a combination thereof, and preferably, the chemotherapy or radiation therapy may be preceding anticancer therapy, and more preferably, the anticancer therapy may be standard neoadjuvant chemoradiotherapy or surgical treatment after standard neoadjuvant chemoradiotherapy, but the present invention is not limited thereto.

In one embodiment of the present invention, predicting that a prognosis is poor may mean a case showing distant metastasis and a mortality rate of 60% or more within 3 years after pCR is achieved as a result of the standard neoadjuvant chemoradiotherapy.

In the present invention, the device may further include an output unit that outputs the prognostic prediction information, but the present invention is not limited thereto. The output unit is not limited as long as it can output information in the device, and the device is not limited as long as it outputs information as a web page or application, and may include, for example, a computing device, a mobile device, a server, and the like.

In the present invention, the device may further include an input unit for receiving the TNM stage, age or sex of the subject, pCR or combined information thereof, but the present invention is not limited thereto.

In the present invention, when the calculation unit determines that the first molecular subtype or a protein encoded thereby is expressed in a biological sample isolated from a target subject, or the expression level is higher than the control, it can be predicted that the therapeutic response to the anticancer therapy is low, and for example, when the calculation unit determines that the expression level of the first molecular subtype or a protein encoded thereby is higher than that of the control, it can be predicted that standard neoadjuvant chemoradiotherapy or surgical treatment after standard neoadjuvant chemoradiotherapy is low.

In the present invention, when the calculation unit determines that the first molecular subtype or a protein encoded thereby is expressed in a biological sample isolated from a target subject, or the expression level is higher than the control, it can be predicted that a prognosis after the anticancer therapy is poor. For example, when the calculation unit determines that the expression level of the first molecular subtype or a protein encoded thereby is higher than that of the control, it can be predicted that a prognosis after standard neoadjuvant chemoradiotherapy or a prognosis after surgical treatment following standard neoadjuvant chemoradiotherapy is poor, and specifically, it can be predicted that a survival rate is low, a recurrent probability is high, or a metastasis probability is high, but the present invention is not limited thereto.

In one embodiment of the present invention, when the calculation unit determines that the expression level of the first molecular subtype measured in a biological sample isolated from a target subject is higher than the control, and pCR is not achieved after anticancer therapy, and preferably, standard neoadjuvant chemoradiotherapy, it can be predicted that a prognosis is poor.

In another embodiment of the present invention, when the calculation unit determines that the expression level of the first molecular subtype measured in a biological sample isolated from a target subject is higher than the control, and the TNM stage of the subject is T3 or T4, it can be predicted that a prognosis after anticancer therapy, and preferably, standard neoadjuvant chemoradiotherapy or surgical treatment after standard neoadjuvant chemoradiotherapy is poor.

In still another embodiment of the present invention, when the calculation unit determines that the expression level of the first molecular subtype in a biological sample isolated from a target subject is higher than the control, and the TNM stage of the subject is N1 or N2, it can be predicted that a prognosis after anticancer therapy, and preferably, standard neoadjuvant chemoradiotherapy or surgical treatment is poor.

In yet another embodiment of the present invention when the calculation unit determines that the expression level of the first molecular subtype in a biological sample isolated from a target subject is higher than the control, and the TNM stage of the subject is T3 or T4, and N1 or N2, it can be predicted that a prognosis after anticancer therapy, and preferably, standard neoadjuvant chemoradiotherapy or surgical treatment is poor.

Meanwhile, in one embodiment of the present invention, when the calculation unit determines that the expression level of the second molecular subtype measured in a biological sample isolated from a target subject is higher than that of a control, and pCR is not achieved after anticancer therapy, and preferably, standard neoadjuvant chemoradiotherapy, it can be predicted that the prognosis is good.

In another embodiment of the present invention, when the calculation unit determines that the expression level of the second molecular subtype measured in a biological sample isolated from a target subject is higher than that of a control, and pCR is achieved after anticancer therapy, and preferably, standard neoadjuvant chemoradiotherapy, it can be predicted that a prognosis after the anticancer therapy is good.

In another embodiment of the present invention, when the calculation unit determines that the expression level of the second molecular subtype measured in a biological sample isolated from a target subject is higher than that of a control, and the TNM stage of the subject is T0, T1 or T2, it can be predicted that a prognosis after anticancer therapy, and preferably, standard neoadjuvant chemoradiotherapy or surgical treatment after standard neoadjuvant chemoradiotherapy is good.

In still another embodiment of the present invention, when the calculation unit determines that the expression level of the second molecular subtype measured in a biological sample isolated from a target subject is higher than that of a control, and the TNM stage of the subject is N0, it can be predicted that a prognosis after anticancer therapy, and preferably, standard neoadjuvant chemoradiotherapy or surgical treatment is good.

In yet another embodiment of the present invention, when the calculation unit determines that the expression level of the second molecular subtype measured in a biological sample isolated from a target subject is higher than that of a control, and the TNM stage of the subject is T0, T1 or T2, and N0, it can be predicted that a prognosis after anticancer therapy, and preferably, standard neoadjuvant chemoradiotherapy or surgical treatment is poor.

In the present invention, the control may be an average value of the expression level of the first molecular subtype or the second molecular subtype or a protein encoded thereby in a normal subject, or the expression level of a corresponding gene in a subject with cancer or a high probability of developing cancer, particularly, a subject who has been diagnosed with cancer, but the present invention is not limited thereto.

In the present invention, the cancer may be one or more types of cancer selected from the group consisting of breast cancer, uterine cancer, esophageal cancer, stomach cancer, brain cancer, rectal cancer, colon cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma, and leukemia, and preferably, rectal cancer.

### [Advantageous Effects]

According to the present invention, there are advantages in that a therapeutic response to anticancer therapy, and preferably, neoadjuvant chemoradiotherapy or a surgical treatment for a cancer patient, particularly, a rectal cancer patient or a prognosis after the treatment can be predicted, and an appropriate treatment or monitoring plan can be established depending on the predicted prognosis.

### [Description of Drawings]

FIG. 1 is a flowchart showing the flow of a study design according to Preparation Example 2 of the present invention.
FIG. 2 shows the result of TCGA rectal cancer data analysis using non-negative matrix factorization (NMF) with 2 to 5 ranks according to Preparation Example 7 of the present invention.
FIG. 3 shows the result of TCGA rectal cancer data analyzed by consensus clustering by non-negative matrix factorization (NMF) with 2 to 5 ranks according to Preparation Example 7 of the present invention.
FIG. 4 shows the result of gene set enrichment analysis according to Preparation Example 8 of the present invention, in which the first molecular subtype has characteristics of epithelial mesenchymal transition (EMT) and cancer stem cells.
FIG. 5 shows the result of gene set enrichment analysis performed to see the difference between a rectal cancer-intrinsic molecular subtype and a colon cancer molecular subtype according to Preparation Example 9 of the present invention, showing that a first molecular subtype has characteristics of stem cells.
FIG. 6 shows the disease-free survival (DFS) rates of 230 rectal cancer patients from the Yonsei Cancer Center by subtypes classified by the first selected gene set according to Experimental Example 1 of the present invention.
FIG. 7 shows the disease-free survival (DFS) rates of patients by subtypes classified by the second selected gene set according to one embodiment of the present invention.
FIG. 8 shows a difference between disease-free survival (DFS) rates of rectal cancer patients by molecular subtypes according to Experimental Example 2 of the present invention.
FIG. 9 shows a difference in overall survival (OS) rates of rectal cancer patients depending on pCR according to Experimental Example 2 of the present invention.
FIG. 10 shows a difference in disease-free survival rates of rectal cancer patients by N stages according to Experimental Example 4 of the present invention.
FIG. 11 shows a difference in disease-free survival rates of rectal cancer patients by molecular subtypes and N stages according to Experimental Example 4 of the present invention.
FIG. 12 shows a difference in overall survival (OS) rates of rectal cancer patients by N stages according to Experimental Example 4 of the present invention.
FIG. 13 shows a difference in overall survival (OS) rates of rectal cancer patients by molecular subtypes and N stages according to Experimental Example 4 of the present invention.
FIG. 14 shows the ability of CMS molecular subtypes to predict the disease-free survival rate of a rectal cancer patient according to Experimental Example 5 of the present invention.
FIG. 15 shows the ability of CRIS molecular subtypes to predict the disease-free survival rate of a rectal cancer according to Experimental Example 5 of the present invention.
FIG. 16 shows the protocol of rectal cancer treatment according to a first molecular subtype and a second molecular subtype and the pathological characteristics of a patient according to Example 1 of the present invention.

### [Modes of the Invention]

One purpose of the present invention is directed to providing a biomarker composition which can accurately and simply predict a therapeutic response to anticancer therapy or a prognosis after anticancer therapy.

Hereinafter, the present invention will be described in further detail with reference to examples. These examples are only for illustrating the present invention in further detail, and it will be apparent to those of ordinary skill in the art that the scope of the present invention is not limited by these examples according to the gist of the present invention.

### [Examples]

### [Preparation Example 11 Study cohort

To develop a rectal cancer-specific molecular subtype classifier, a total of two clinical cohorts including publicly available RNAseq data from 177 rectal cancer patients downloaded from the Cancer Genome Atlas Project and RNAseq data of a validation cohort consisting of 230 rectal cancer cases from the Yonsei Cancer Center were used.

### [Preparation Example 21 Study design

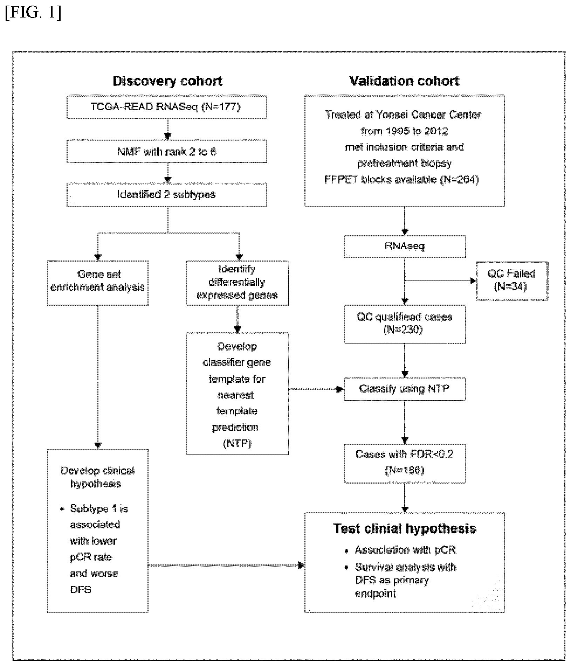

FIG. 1 is a flowchart showing the schematic flow of the study design.

To find intrinsic rectal cancer molecular subtypes, genes using non-negative matrix factorization (NMF) and differently expressed between identified subtypes were identified using the DEseq2 package. Based on the gene set enrichment analysis of DEseq2 data, the inventors established a clinical hypothesis on the role of molecular subtypes to predict responses to chemotherapy and a prognosis, and prospectively tested 230 cases of rectal cancer diagnosed and treated at the Yonsei Cancer Center. To classify a rectal cancer patient group from the Yonsei Cancer Center into molecular subtypes, a molecular subtyping gene list was constructed using a list of 522 genes that have a two-fold or more difference in expression level and a p-value of less than 10⁻⁵ in the DESeq2 analysis. In addition, to develop the optimal classification gene list for the molecular subtypes found, the prediction analysis of a microarray was used.

### [Preparation Example 31 Validation cohort

As a result of investigating the case history of rectal adenocarcinoma patients from the Yonsei Cancer Center (Seoul, Korea) from 1995 to 2012 in order to predict a prognosis of a rectal cancer patient, 264 cases were confirmed, and according to inclusion criteria, cases of 1) performing total mesenteric excision (TME) following pre-radiation therapy (preop-CRT) and 2) using a formalin-fixed paraffin-embedded (FFPE) pretreated biopsy sample were included. A case in which 3) a patient received incomplete CRT, metastatic disease or palliative treatment was excluded.

CRT consisted of a total of 45 Gy delivered to the pelvis in 25 fractions of 180 cGy 5 times a week using a 3D conformal technique, and boosted radiation therapy of 540 cGy was given in 3 fractions. The simultaneous chemotherapy used was 5-fluorouracil bonded with leucovorin or capcitabine. TME was performed within 6 to 8 weeks after the completion of CRT. Fluorouracil-based postoperative radiation (postop-CRT) was given unless a postoperative pathological examination reported pCR.

After surgery, patients were followed at 3-month intervals for the first 3 years, at 6-month intervals for the next 2 years, and annually thereafter. Routine surveillance included physical examination, endoscopy, serum cancer embryo antigens, chest and abdominal pelvic CT scans, and toxicity assessment. Histological confirmation, MRI or FDG-PET was performed for further evaluation when recurrence was suspected. Intrapelvic recurrence was defined as local recurrence, and other recurrences were defined as distant recurrence.

### [Preparation Example 41 RNAseq and quality control

RNA was extracted from paraffin-embedded biopsy tissues after formalin fixation for 264 cases using the Qiagen AllPrep DNA/RNA FFPE kit (Qiagen, Valencia, CA, USA). Tumor-rich areas were extracted from 1 to 17 5-micron thick tissue sections depending on the amount of available tissues. An RNA concentration was quantified by fluorescent analysis (Qubit RNA Assay kit, ThermoFisher Scientific, USA). RNAseq was performed using the Ion Proton platform according to the manufacturer's instructions. 34 cases with poor data quality were excluded.

### [Preparation Example 51 RNAseq data and gene set enrichment analysis

Gene expression values were quantified using HT-seq with the Ensembl GRCh37 gene model. Count data were normalized by the DESeq variance stabilizing transformation (VST). Cases were assigned to the NMF-derived native subtypes using Nearest Template Prediction with 522 classifier gene templates derived from DESeq2. In addition, the enrichment analysis of colon cancer and cancer-related gene sets was performed by utilizing the gene set enrichment analysis functions, "CMSgsa" and "fgsea" packages of the consensus molecular subtype (CMS) package for colon cancer and colorectal cancer (CRC).

### [Preparation Example 6] Statistical analysis of clinical results by intrinsic subtypes

All statistical analyses were performed using R statistical programming environment version 3.6.3 and R Studio version 1.2.5033. The primary evaluation criterion was a disease-free survival (DFS) rate defined as the period from the date of surgery to the last follow-up in the case of the first local or distant recurrence event, death or censorship. The secondary evaluation variables included a distant recurrence-free survival (DRFS) rate, a local recurrence-free survival (LRFS) rate, and an overall survival (OS) rate. For LRFS and DRFS analyses, data was censored at the time of a competition event. OS was defined as the period from the data of surgery to the date of death, or the last follow-up when censored. For survival analysis, an R survival package was used. Survminer and ggplot2 packages were used to generate a Kaplan-Meier plot. The Kaplan-Meier method was used to compare a survival difference between intrinsic subtypes and tested with a log-rank test. Factors related to DFS and OS were analyzed by Cox proportional hazard regression analysis. A two-sided P-value of less than 0.05 was considered statistically significant. The relative accuracy of each prognostic model was evaluated using the log-likelihood ratio and the correlation index (C-index).

### [Preparation Example 7] Discovery of rectal cancer molecular subtype using RNAseq data from TCGA-READ cohort

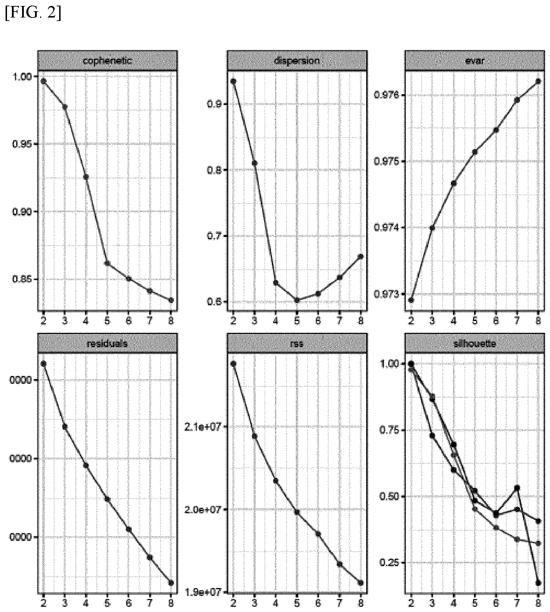

FIG. 2 shows TCGA rectal cancer data analysis result indexes using non-negative matrix factorization (NMF).

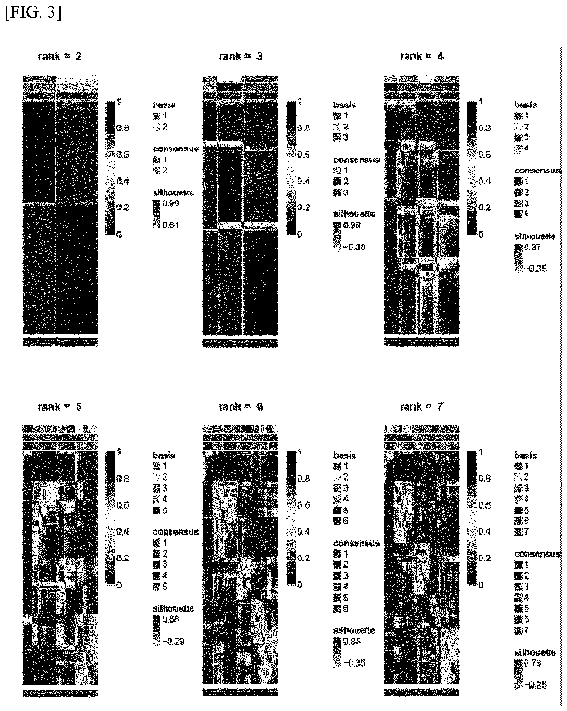

FIG. 3 shows the consensus clustering result of non-negative matrix factorization (NMF) with 2 to 5 ranks.

RNAseq data (TCGA-READ.htseq_fpkm-uq.tsv) of 177 rectal cancer samples were downloaded from the Cancer Genome Atlas Project. To identify the optical number of subtypes using R package "NMF," a non-negative matrix factorization analysis was performed with a rank from 2 to 5. The cophenetic index and silhouette discovered through consensus clustering suggest that dividing rectal cancer into two molecular subtypes would be the best option.

### [Preparation Example 8] Properties of two types of intrinsic molecular subtypes of rectal cancer

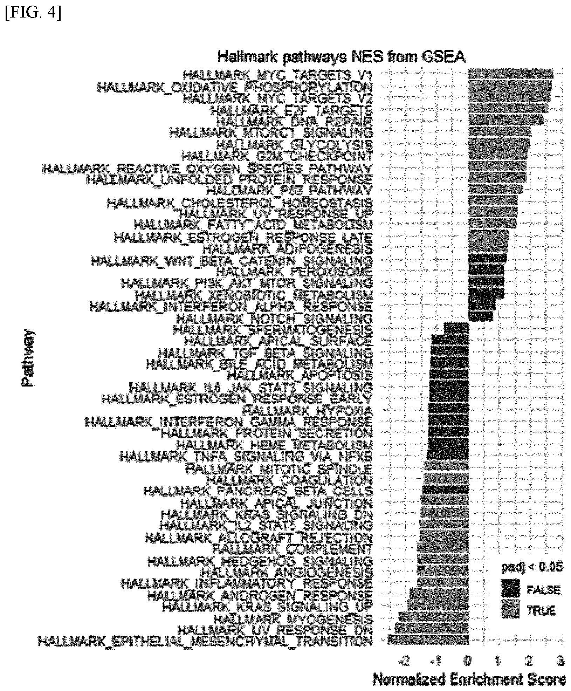

FIG. 4 shows the result of the gene set enrichment analysis for two molecular subtypes found.

To find the biological properties of the two molecular subtypes found, a gene set enrichment analysis was performed to identify significantly different biological pathways between two subtypes using the "fgsea" package of R and the "CMSgsa" package. As shown in FIG. 4, among specific cancer pathways, in the first molecular subtype, an epithelial mesenchymal transition (EMT) pathway and stem cell-specific gene expression are relatively abundant, but in the second molecular subtype, MYC targeting, cell division, oxidative phosphorylation and DNA recovery pathway gene expression are relatively abundant.

### [Preparation Example 9] Difference between rectal cancer-intrinsic molecular subtype and colon cancer molecular subtype

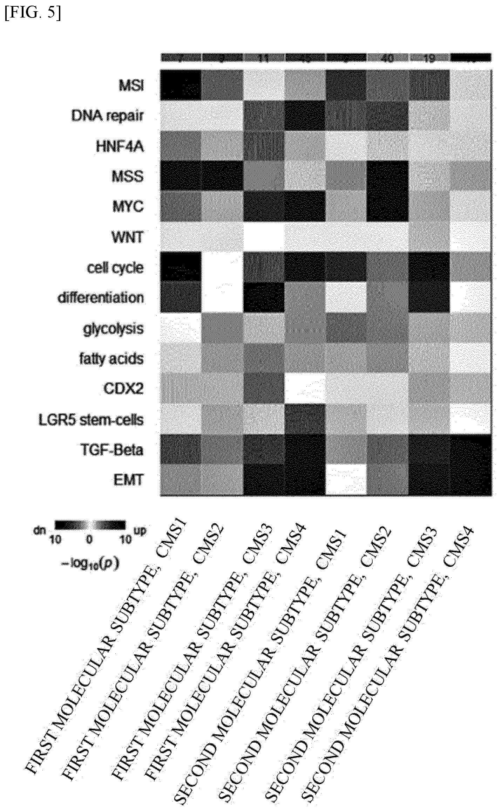

FIG. 5 is a heat map chart showing the result of gene set enrichment analysis to see the difference between a rectal cancer-intrinsic molecular subtype and a colon cancer molecular subtype.

In the meantime, rectal cancer has been included in the molecular subtype classification of colorectal cancer as it has been judged to be a part of colorectal cancer. The molecular subtype of colon cancer is classified into four molecular subtypes, that is, Consensus Molecular Subtype (CMS) 1 to 4, by global consensus. The CMS4 subtype has a poor prognosis and the epithelial-mesenchymal transition pathway gene is activated, so it is likely to be the same as the first molecular subtype found by the inventors. Accordingly, TCGA-READ RNAseq data was classified into CMS molecular subtypes using the CMScaller package, and the correlation between the two molecular subtypes found by the inventors was examined. Only 58.8% of the first molecular subtype was classified as CMS4, and 17.5% of the second molecular subtype was classified as CMS4. It showed that they have a statistically significant correlation, but do not match.

**[Table 1]**

| | CMS1 | CMS2 | CMS3 | CMS4 | Total |
|---|---|---|---|---|---|
| First molecular subtype | 8 | 13 | 12 | 47 (58·8%) | 80 |
| Second molecular subtype | 12 | 46 | 22 | 17 (17·5%) | 97 |
| Total | 20 | 59 | 34 | 64 | 177 |

As a result of a gene set enrichment analysis for 8 types of rectal cancer classified by two classification methods using the "CMSgsa" function of the "CMScaller" package in order to understand the difference between CMS4 and the first molecular subtype, as shown in FIG. 5, the EMT-related gene expression of the rectal cancer-intrinsic first molecular subtype for rectal cancer classified as CMS4 molecular subtype was similar to the second molecular subtype, but the expression of a gene set expressed in stem cells is increased. On the other hand, it was found that the expression of the gene sets expressed in stem cells was increased, whereas the expression of cell division-related gene sets, that is, MYC, DNA repair, and cell cycle, was decreased. There results prove that the rectal cancer-intrinsic molecular subtypes found by the inventors are sufficiently different from the colon cancer molecular subtypes.

In addition, based on the above data, it was hypothesized that the first molecular subtype was associated with a worse prognosis and a lower response to preoperative chemoradiotherapy, compared to the second molecular subtype.

### [Preparation Example 101 Development of classifier for newly found molecular subtype (1)

FIG. 5 shows 85 optimal genes that are able to classify two molecular subtypes using a Prediction Analysis of the Microarray R package (PAMr).

The Prediction Analysis of the Microarray R package (PAMr) was utilized to develop a classifier for the newly found molecular subtypes. For analysis, a threshold of 6 and a prop-selected-in-cv threshold of 0.6 were used. For reference, even when the threshold and the prop-selected-in-cv threshold are changed, it affects the number of selected genes and the final performance of the classifier, but the top classifier gene remains the same, and the clinical performance is similar to a partial change in p-value. As a result of this analysis, 94 genes were primarily selected as templates for subtype classification as shown in Table 2. In the molecular subtype items in Table 2, 1 denotes the first molecular subtype, and 2 denotes the second molecular subtype.

**[Table 2]**

| No. | Gene | Molecular subtype |
|---|---|---|
| **1** | ZNF728 | 1 |
| **2** | ZNF676 | 1 |
| **3** | TVP23C-CDRT4 | 1 |
| **4** | TCEAL2 | 1 |
| **5** | TBC1D3L | 1 |
| **6** | SYT4 | 1 |
| **7** | SLITRK4 | 1 |
| **8** | SEMA3E | 1 |
| **9** | SCN9A | 1 |
| **10** | SCN7A | 1 |
| **11** | RANBP3L | 1 |
| **12** | PLN | 1 |
| **13** | PLGLB2 | 1 |
| **14** | PLCXD3 | 1 |
| **15** | PGM5P3-AS1 | 1 |
| **16** | PGM5-AS1 | 1 |
| **17** | PCDH10 | 1 |
| **18** | OR7E12P | 1 |
| **19** | NLGN1 | 1 |
| **20** | NEXN | 1 |
| **21** | MYH8 | 1 |
| **22** | MIR4477B | 1 |
| **23** | MIR3911 | 1 |
| **24** | MIR186 | 1 |
| **25** | MIR133A1HG | 1 |
| **26** | MEIS1-AS2 | 1 |
| **27** | LONRF2 | 1 |
| **28** | LOC644838 | 1 |
| **29** | LOC642131 | 1 |
| **30** | LOC440434 | 1 |
| **31** | LOC101929607 | 1 |
| **32** | LOC101928509 | 1 |
| **33** | LOC100507387 | 1 |
| **34** | LOC100507073 | 1 |
| **35** | LINGO2 | 1 |
| **36** | LINC01537 | 1 |
| **37** | LINC01489 | 1 |
| **38** | LINC01352 | 1 |
| **39** | LINC01266 | 1 |
| **40** | LINC00504 | 1 |
| **41** | LGI1 | 1 |
| **42** | KRT222 | 1 |
| **43** | KIAA2022 | 1 |
| **44** | KIAA0408 | 1 |
| **45** | KCTD8 | 1 |
| **46** | HNRNPA1P33 | 1 |
| **47** | HLX-AS1 | 1 |
| **48** | HIST2H3C | 1 |
| **49** | HCG23 | 1 |
| **50** | GTF2IP1 | 1 |
| **51** | GRIN2A | 1 |
| **52** | GRIA2 | 1 |
| **53** | GOLGA8K | 1 |
| **54** | GAS1RR | 1 |
| **55** | FILIP 1 | 1 |
| **56** | FAM47E-STBD1 | 1 |
| **57** | FAM35BP | 1 |
| **58** | FAM133A | 1 |
| **59** | EPHA6 | 1 |
| **60** | CTAGE8 | 1 |
| **61** | CDH19 | 1 |
| **62** | CCDC144B | 1 |
| **63** | C10orf131 | 1 |
| **64** | BVES-AS1 | 1 |
| **65** | BLOC1S5-TXNDC5 | 1 |
| **66** | BCHE | 1 |
| **67** | ARHGEF18 | 1 |
| **68** | ADAMTS9-AS1 | 1 |
| **69** | ACADL | 1 |
| **70** | TRAPPC5 | 2 |
| **71** | TPGS1 | 2 |
| **72** | TMEM160 | 2 |
| **73** | SNORD38A | 2 |
| **74** | SNORD30 | 2 |
| **75** | SNHG25 | 2 |
| **76** | PRR7 | 2 |
| **77** | PDF | 2 |
| **78** | NOXO1 | 2 |
| **79** | MIR3661 | 2 |
| **80** | LOC440311 | 2 |
| **81** | FEZF2 | 2 |
| **82** | FAM173A | 2 |
| **83** | EIF3IP1 | 2 |
| **84** | CTU1 | 2 |
| **85** | C4orf48 | 2 |

As shown in Table 2, classification genes relatively over-expressed in the primarily selected first molecular subtype are ACADL, ADAMTS9-AS1, ARHGEF18, BCHE, BLOC1S5-TXNDC5, BVES-AS1, C10orf131, CCDC144B, CDH19, CTAGE8, EPHA6, FAM133A, FAM35BP, FAM47E-STBD1, FILIP1, GAS1RR, GOLGA8K, GRIA2, GRIN2A, GTF2IP1, HCG23, HIST2H3C, HLX-AS1, HNRNPA1P33, KCTD8, KIAA0408, KIAA2022, KRT222, LGI1, LINC00504, LINC01266, LINC01352, LINC01489, LINC01537, LINGO2, LOC100507073, LOC100507387, LOC101928509, LOC101929607, LOC440434, LOC642131, LOC644838, LONRF2, MEIS1-AS2, MIR133A1HG, MIR186, MIR3911, MIR4477B, MYH8, NEXN, NLGN1, OR7E12P, PCDH10, PGM5-AS1, PGM5P3-AS1, PLCXD3, PLGLB2, PLN, RANBP3L, SCN7A, SCN9A, SEMA3E, SLITRK4, SYT4, TBC1D3L, TCEAL2, TVP23C-CDRT4, ZNF676, and ZNF728.

Meanwhile, classification genes relatively over-expressed in the primarily-selected second molecular subtype are C4orf48, CTU1, EIF3IP1, FAM173A, FEZF2, LOC440311, MIR3661, NOXO1, PDF, PRR7, SNHG25, SNORD30, SNORD38A, TMEM160, TPGS1, and TRAPPC5.

When gene expression was analyzed by the RNAseq method, since the expression level of many genes can be measured at the same time, it is possible to apply a panel consisting of more genes than a 94-gene panel. Differentially expressed genes between two subtypes were identified using the "DEseq2" package in R. At the statistical significance level of p<10⁻⁷, there were 4877 differentially expressed genes between the two molecular subtypes. Among 4877 genes, it is possible to develop a molecular subtype classifier by selecting and combining some genes in various ways, and in one example, as shown in Table 3, classification is possible by selecting 522 genes in which the difference in expression level between two molecular subtypes is 2 times or more as templates. In the molecular subtype items of Table 3 below, 1 denotes the first molecular subtype, and 2 denotes the second molecular subtype.

**[Table 3]**

| | Gene | Molecular subtype |
|---|---|---|
| 1 | ADAT3 | 2 |
| 2 | ANP32D | 2 |
| 3 | BHLHA9 | 2 |
| 4 | BOD1L2 | 2 |
| 5 | C4orf48 | 2 |
| 6 | CCDC85B | 2 |
| 7 | CDH16 | 2 |
| 8 | CLMAT3 | 2 |
| 9 | CSNK1A1L | 2 |
| 10 | CTU1 | 2 |
| 11 | DBET | 2 |
| 12 | DDC-AS1 | 2 |
| 13 | DEFA5 | 2 |
| 14 | EIF3IP1 | 2 |
| 15 | FAM173A | 2 |
| 16 | FEZF2 | 2 |
| 17 | FOXI3 | 2 |
| 18 | FRMD8P1 | 2 |
| 19 | GALR3 | 2 |
| 20 | GJD3 | 2 |
| 21 | GPR25 | 2 |
| 22 | HBA1 | 2 |
| 23 | HES4 | 2 |
| 24 | HIST1H4A | 2 |
| 25 | HIST1H4L | 2 |
| 26 | HLA-L | 2 |
| 27 | IGFBP7-AS1 | 2 |
| 28 | ITLN2 | 2 |
| 29 | KCNE1B | 2 |
| 30 | LCN15 | 2 |
| 31 | LKAAEAR1 | 2 |
| 32 | LOC101927795 | 2 |
| 33 | LOC101927972 | 2 |
| 34 | LOC101928372 | 2 |
| 35 | LOC344967 | 2 |
| 36 | LRRC26 | 2 |
| 37 | MAGEA10 | 2 |
| 38 | MESP1 | 2 |
| 39 | MIR203A | 2 |
| 40 | MIR324 | 2 |
| 41 | MIR3661 | 2 |
| 42 | MIR4449 | 2 |
| 43 | MIR4479 | 2 |
| 44 | MIR4665 | 2 |
| 45 | MIR4737 | 2 |
| 46 | MIR4767 | 2 |
| 47 | MIR6807 | 2 |
| 48 | MIR6858 | 2 |
| 49 | MIR6891 | 2 |
| 50 | MIR8075 | 2 |
| 51 | NACA2 | 2 |
| 52 | NOXO1 | 2 |
| 53 | ONECUT3 | 2 |
| 54 | PCSK1N | 2 |
| 55 | PDF | 2 |
| 56 | PITPNM2-AS1 | 2 |
| 57 | PNMA5 | 2 |
| 58 | PRR7 | 2 |
| 59 | PRSS2 | 2 |
| 60 | PRSS56 | 2 |
| 61 | PTGER1 | 2 |
| 62 | PTTG3P | 2 |
| 63 | REG3A | 2 |
| 64 | RNA5S9 | 2 |
| 65 | RNU4-1 | 2 |
| 66 | RNU5A-1 | 2 |
| 67 | RNU5B-1 | 2 |
| 68 | RNU5E-1 | 2 |
| 69 | RNU6ATAC | 2 |
| 70 | RNY1 | 2 |
| 71 | RPL29P2 | 2 |
| 72 | RPRML | 2 |
| 73 | SBF1P1 | 2 |
| 74 | SHISAL2B | 2 |
| 75 | SKOR2 | 2 |
| 76 | SLC32A1 | 2 |
| 77 | SMARCA5-AS1 | 2 |
| 78 | SMCR5 | 2 |
| 79 | SNHG25 | 2 |
| 80 | SNORA36A | 2 |
| 81 | SNORD30 | 2 |
| 82 | SNORD38A | 2 |
| 83 | SNORD3B-2 | 2 |
| 84 | SNORD41 | 2 |
| 85 | SNORD48 | 2 |
| 86 | TMEM160 | 2 |
| 87 | TMEM238 | 2 |
| 88 | TPGS1 | 2 |
| 89 | TRAPPC5 | 2 |
| 90 | UBE2NL | 2 |
| 91 | WBP11P1 | 2 |
| 92 | ZAR1 | 2 |
| 93 | AADACL2 | 1 |
| 94 | ABCA6 | 1 |
| 95 | ABCA8 | 1 |
| 96 | ABCA9 | 1 |
| 97 | ABCB5 | 1 |
| 98 | ABI3BP | 1 |
| 99 | ACADL | 1 |
| 100 | ACSM5 | 1 |
| 101 | ACTG2 | 1 |
| 102 | ADAMTS9-AS1 | 1 |
| 103 | ADAMTS9-AS2 | 1 |
| 104 | ADAMTSL3 | 1 |
| 105 | ADCYAP1R1 | 1 |
| 106 | ADGRB3 | 1 |
| 107 | ADH1B | 1 |
| 108 | ADIPOQ | 1 |
| 109 | ADRA1A | 1 |
| 110 | AFF3 | 1 |
| 111 | AGTR1 | 1 |
| 112 | AICDA | 1 |
| 113 | ALB | 1 |
| 114 | ANGPTL1 | 1 |
| 115 | ANGPTL5 | 1 |
| 116 | ANGPTL7 | 1 |
| 117 | ANK2 | 1 |
| 118 | ANKS1B | 1 |
| 119 | ANXA8L1 | 1 |
| 120 | APOA2 | 1 |
| 121 | APOB | 1 |
| 122 | APOC3 | 1 |
| 123 | AQP4 | 1 |
| 124 | AQP8 | 1 |
| 125 | ARPP21 | 1 |
| 126 | ART4 | 1 |
| 127 | ASB5 | 1 |
| 128 | ASPA | 1 |
| 129 | ASTN1 | 1 |
| 130 | ATCAY | 1 |
| 131 | ATP1A2 | 1 |
| 132 | ATP2B2 | 1 |
| 133 | ATP2B3 | 1 |
| 134 | AVPR1B | 1 |
| 135 | B3GALT5-AS1 | 1 |
| 136 | BCHE | 1 |
| 137 | BEST4 | 1 |
| 138 | BHMT2 | 1 |
| 139 | BLOC1S5-TXNDC5 | 1 |
| 140 | BMP3 | 1 |
| 141 | BRINP3 | 1 |
| 142 | BVES | 1 |
| 143 | BVES-AS1 | 1 |
| 144 | C14orf180 | 1 |
| 145 | C1QTNF7 | 1 |
| 146 | C7 | 1 |
| 147 | C8orf88 | 1 |
| 148 | CA1 | 1 |
| 149 | CA2 | 1 |
| 150 | CA7 | 1 |
| 151 | CACNA2D1 | 1 |
| 152 | CADM2 | 1 |
| 153 | CADM3 | 1 |
| 154 | CALN1 | 1 |
| 155 | CARTPT | 1 |
| 156 | CASQ2 | 1 |
| 157 | CAVIN2 | 1 |
| 158 | CCBE1 | 1 |
| 159 | CCDC144B | 1 |
| 160 | CCDC158 | 1 |
| 161 | CCDC160 | 1 |
| 162 | CCDC169 | 1 |
| 163 | CCN5 | 1 |
| 164 | CD300LG | 1 |
| 165 | CDH10 | 1 |
| 166 | CDH19 | 1 |
| 167 | CDKN2B-AS1 | 1 |
| 168 | CDO1 | 1 |
| 169 | CHRDL1 | 1 |
| 170 | CHRM2 | 1 |
| 171 | CHST9 | 1 |
| 172 | CIDEA | 1 |
| 173 | CILP | 1 |
| 174 | CLCA4 | 1 |
| 175 | CLCNKB | 1 |
| 176 | CLDN8 | 1 |
| 177 | CLEC3B | 1 |
| 178 | CLEC4M | 1 |
| 179 | CLVS2 | 1 |
| 180 | CMA1 | 1 |
| 181 | CNGA3 | 1 |
| 182 | CNN1 | 1 |
| 183 | CNR1 | 1 |
| 184 | CNTN1 | 1 |
| 185 | CNTN2 | 1 |
| 186 | CNTNAP4 | 1 |
| 187 | COL19A1 | 1 |
| 188 | CP | 1 |
| 189 | CPEB1 | 1 |
| 190 | CPXM2 | 1 |
| 191 | CR2 | 1 |
| 192 | CRP | 1 |
| 193 | CTNNA3 | 1 |
| 194 | CTSG | 1 |
| 195 | CYP1B1 | 1 |
| 196 | DAO | 1 |
| 197 | DCLK1 | 1 |
| 198 | DDR2 | 1 |
| 199 | DES | 1 |
| 200 | DHRS7C | 1 |
| 201 | DIRAS2 | 1 |
| 202 | DPP6 | 1 |
| 203 | DPT | 1 |
| 204 | EBF2 | 1 |
| 205 | ECRG4 | 1 |
| 206 | ELAVL4 | 1 |
| 207 | EPHA5 | 1 |
| 208 | EPHA6 | 1 |
| 209 | EPHA7 | 1 |
| 210 | ERICH3 | 1 |
| 211 | EVX2 | 1 |
| 212 | FABP4 | 1 |
| 213 | FAM106A | 1 |
| 214 | FAM133A | 1 |
| 215 | FAM135B | 1 |
| 216 | FAM180B | 1 |
| 217 | FDCSP | 1 |
| 218 | FGF10 | 1 |
| 219 | FGF13-AS1 | 1 |
| 220 | FGF14 | 1 |
| 221 | FGFBP2 | 1 |
| 222 | FGG | 1 |
| 223 | FGL1 | 1 |
| 224 | FHL1 | 1 |
| 225 | FILIP 1 | 1 |
| 226 | FLNC | 1 |
| 227 | FMO2 | 1 |
| 228 | FRMD6-AS2 | 1 |
| 229 | FRMPD4 | 1 |
| 230 | FUT9 | 1 |
| 231 | GABRA5 | 1 |
| 232 | GABRG2 | 1 |
| 233 | GALR1 | 1 |
| 234 | GAP43 | 1 |
| 235 | GAS1RR | 1 |
| 236 | GC | 1 |
| 237 | GCG | 1 |
| 238 | GDF6 | 1 |
| 239 | GFRA1 | 1 |
| 240 | GNAO1 | 1 |
| 241 | GPM6A | 1 |
| 242 | GPR119 | 1 |
| 243 | GPR12 | 1 |
| 244 | GPRACR | 1 |
| 245 | GRIA2 | 1 |
| 246 | GRIN2A | 1 |
| 247 | GTF2IP1 | 1 |
| 248 | GUCA2B | 1 |
| 249 | HAND1 | 1 |
| 250 | HAND2 | 1 |
| 251 | HAND2-AS1 | 1 |
| 252 | HEPACAM | 1 |
| 253 | HP | 1 |
| 254 | HPCAL4 | 1 |
| 255 | HRG | 1 |
| 256 | HRK | 1 |
| 257 | HSPB8 | 1 |
| 258 | HTR2B | 1 |
| 259 | IGSF10 | 1 |
| 260 | IGSF11 | 1 |
| 261 | IRX6 | 1 |
| 262 | ISM1 | 1 |
| 263 | KCNA1 | 1 |
| 264 | KCNB1 | 1 |
| 265 | KCNC2 | 1 |
| 266 | KCNK2 | 1 |
| 267 | KCNMA1 | 1 |
| 268 | KCNMB1 | 1 |
| 269 | KCNQ5 | 1 |
| 270 | KCNT2 | 1 |
| 271 | KCTD8 | 1 |
| 272 | KERA | 1 |
| 273 | KHDRBS2 | 1 |
| 274 | KIAA0408 | 1 |
| 275 | KIF1A | 1 |
| 276 | KRT222 | 1 |
| 277 | KRT24 | 1 |
| 278 | KRTAP13-2 | 1 |
| 279 | LCN10 | 1 |
| 280 | LDB3 | 1 |
| 281 | LEP | 1 |
| 282 | LGI1 | 1 |
| 283 | LIFR | 1 |
| 284 | LINC00504 | 1 |
| 285 | LINC00507 | 1 |
| 286 | LINC00682 | 1 |
| 287 | LINC00924 | 1 |
| 288 | LINC01266 | 1 |
| 289 | LINC01352 | 1 |
| 290 | LINC01474 | 1 |
| 291 | LINC01505 | 1 |
| 292 | LINC01697 | 1 |
| 293 | LINC01798 | 1 |
| 294 | LINC01829 | 1 |
| 295 | LINC02015 | 1 |
| 296 | LINC02023 | 1 |
| 297 | LINC02185 | 1 |
| 298 | LINC02268 | 1 |
| 299 | LINC02408 | 1 |
| 300 | LINC02544 | 1 |
| 301 | LIX1 | 1 |
| 302 | LMO3 | 1 |
| 303 | LMOD1 | 1 |
| 304 | LOC100506289 | 1 |
| 305 | LOC101928731 | 1 |
| 306 | LOC102724050 | 1 |
| 307 | LOC107986321 | 1 |
| 308 | LOC283856 | 1 |
| 309 | LOC440434 | 1 |
| 310 | LOC729558 | 1 |
| 311 | LONRF2 | 1 |
| 312 | LRAT | 1 |
| 313 | LRCH2 | 1 |
| 314 | LRRC3B | 1 |
| 315 | LRRC4C | 1 |
| 316 | LRRTM4 | 1 |
| 317 | LVRN | 1 |
| 318 | LYVE1 | 1 |
| 319 | MAB21L1 | 1 |
| 320 | MAB21L2 | 1 |
| 321 | MAGEE2 | 1 |
| 322 | MAMDC2 | 1 |
| 323 | MAPK4 | 1 |
| 324 | MASP1 | 1 |
| 325 | MEF2C-AS1 | 1 |
| 326 | MEOX2 | 1 |
| 327 | METTL24 | 1 |
| 328 | MFAP5 | 1 |
| 329 | MGAT4C | 1 |
| 330 | MGP | 1 |
| 331 | MICU3 | 1 |
| 332 | MIR133A1HG | 1 |
| 333 | MIR8071-1 | 1 |
| 334 | MMRN1 | 1 |
| 335 | MORN5 | 1 |
| 336 | MPPED2 | 1 |
| 337 | MRGPRE | 1 |
| 338 | MS4A1 | 1 |
| 339 | MS4A12 | 1 |
| 340 | MSRB3 | 1 |
| 341 | MUSK | 1 |
| 342 | MYH11 | 1 |
| 343 | MYH2 | 1 |
| 344 | MYLK | 1 |
| 345 | MYO3A | 1 |
| 346 | MYOC | 1 |
| 347 | MYOCD | 1 |
| 348 | MYOM1 | 1 |
| 349 | MYOT | 1 |
| 350 | MYT1L | 1 |
| 351 | NALCN | 1 |
| 352 | NAP1L2 | 1 |
| 353 | NBEA | 1 |
| 354 | NECAB1 | 1 |
| 355 | NEFL | 1 |
| 356 | NEFM | 1 |
| 357 | NEGRI | 1 |
| 358 | NETO1 | 1 |
| 359 | NEUROD1 | 1 |
| 360 | NEXMIF | 1 |
| 361 | NEXN | 1 |
| 362 | NGB | 1 |
| 363 | NIBAN1 | 1 |
| 364 | NLGN1 | 1 |
| 365 | NOS1 | 1 |
| 366 | NOVA1 | 1 |
| 367 | NPR3 | 1 |
| 368 | NPTX1 | 1 |
| 369 | NPY2R | 1 |
| 370 | NRG3 | 1 |
| 371 | NRK | 1 |
| 372 | NRSN1 | 1 |
| 373 | NRXN1 | 1 |
| 374 | NSG2 | 1 |
| 375 | NTNG1 | 1 |
| 376 | NTRK3 | 1 |
| 377 | NUDT10 | 1 |
| 378 | OGN | 1 |
| 379 | OLFM3 | 1 |
| 380 | OMD | 1 |
| 381 | OTOP2 | 1 |
| 382 | OTOP3 | 1 |
| 383 | P2RX2 | 1 |
| 384 | P2RY12 | 1 |
| 385 | PAK3 | 1 |
| 386 | PAPPA2 | 1 |
| 387 | PCDH10 | 1 |
| 388 | PCDH11X | 1 |
| 389 | PCDH9 | 1 |
| 390 | PCOLCE2 | 1 |
| 391 | PCP4L1 | 1 |
| 392 | PCSK2 | 1 |
| 393 | PDZRN4 | 1 |
| 394 | PEG3 | 1 |
| 395 | PENK | 1 |
| 396 | PGM5 | 1 |
| 397 | PGM5-AS1 | 1 |
| 398 | PGM5P4-AS1 | 1 |
| 399 | PGR | 1 |
| 400 | PHOX2B | 1 |
| 401 | PI16 | 1 |
| 402 | PIK3C2G | 1 |
| 403 | PIRT | 1 |
| 404 | PKHD1L1 | 1 |
| 405 | PLAAT5 | 1 |
| 406 | PLCXD3 | 1 |
| 407 | PLD5 | 1 |
| 408 | PLIN1 | 1 |
| 409 | PLIN4 | 1 |
| 410 | PLN | 1 |
| 411 | PLP1 | 1 |
| 412 | PMP2 | 1 |
| 413 | POPDC2 | 1 |
| 414 | POU3F4 | 1 |
| 415 | PPP1R1A | 1 |
| 416 | PRDM6 | 1 |
| 417 | PRELP | 1 |
| 418 | PRG4 | 1 |
| 419 | PRIMA1 | 1 |
| 420 | PROKR1 | 1 |
| 421 | PTCHD1 | 1 |
| 422 | PTGIS | 1 |
| 423 | PTPRQ | 1 |
| 424 | PTPRZ1 | 1 |
| 425 | PYGM | 1 |
| 426 | PYY | 1 |
| 427 | RANBP3L | 1 |
| 428 | RBFOX3 | 1 |
| 429 | RBM20 | 1 |
| 430 | RELN | 1 |
| 431 | RERGL | 1 |
| 432 | RGS13 | 1 |
| 433 | RGS22 | 1 |
| 434 | RIC3 | 1 |
| 435 | RIMS4 | 1 |
| 436 | RNF150 | 1 |
| 437 | RNF180 | 1 |
| 438 | RORB | 1 |
| 439 | RSPO2 | 1 |
| 440 | SCARA5 | 1 |
| 441 | SCGN | 1 |
| 442 | SCN2B | 1 |
| 443 | SCN7A | 1 |
| 444 | SCN9A | 1 |
| 445 | SCNN1G | 1 |
| 446 | SCRG1 | 1 |
| 447 | SEMA3E | 1 |
| 448 | SERTM1 | 1 |
| 449 | SERTM2 | 1 |
| 450 | SFRP1 | 1 |
| 451 | SFRP2 | 1 |
| 452 | SFTPA1 | 1 |
| 453 | SGCG | 1 |
| 454 | SHISAL1 | 1 |
| 455 | SLC13A5 | 1 |
| 456 | SLC17A8 | 1 |
| 457 | SLC30A10 | 1 |
| 458 | SLC4A4 | 1 |
| 459 | SLC5A7 | 1 |
| 460 | SLC6A2 | 1 |
| 461 | SLC7A14 | 1 |
| 462 | SLIT2 | 1 |
| 463 | SLITRK2 | 1 |
| 464 | SLITRK3 | 1 |
| 465 | SLITRK4 | 1 |
| 466 | SMIM28 | 1 |
| 467 | SMYD1 | 1 |
| 468 | SNAP25 | 1 |
| 469 | SNAP91 | 1 |
| 470 | SORCS1 | 1 |
| 471 | SORCS3 | 1 |
| 472 | SPHKAP | 1 |
| 473 | SPIB | 1 |
| 474 | SPOCK3 | 1 |
| 475 | SST | 1 |
| 476 | ST8SIA3 | 1 |
| 477 | STMN2 | 1 |
| 478 | STMN4 | 1 |
| 479 | STON1-GTF2A1L | 1 |
| 480 | STUM | 1 |
| 481 | SV2B | 1 |
| 482 | SYNM | 1 |
| 483 | SYNPO2 | 1 |
| 484 | SYT10 | 1 |
| 485 | SYT4 | 1 |
| 486 | SYT6 | 1 |
| 487 | TACR1 | 1 |
| 488 | TAFA4 | 1 |
| 489 | TCEAL2 | 1 |
| 490 | TCEAL5 | 1 |
| 491 | TCEAL6 | 1 |
| 492 | TCF23 | 1 |
| 493 | TENM1 | 1 |
| 494 | THBS4 | 1 |
| 495 | TLL1 | 1 |
| 496 | TMEFF2 | 1 |
| 497 | TMEM100 | 1 |
| 498 | TMEM35A | 1 |
| 499 | TMIGD1 | 1 |
| 500 | TMOD1 | 1 |
| 501 | TNNT3 | 1 |
| 502 | TNS1 | 1 |
| 503 | TNXB | 1 |
| 504 | TRARG1 | 1 |
| 505 | TRDN | 1 |
| 506 | UGT2B10 | 1 |
| 507 | UGT2B4 | 1 |
| 508 | UNC80 | 1 |
| 509 | VEGFD | 1 |
| 510 | VGLL3 | 1 |
| 511 | VIT | 1 |
| 512 | VSTM2A | 1 |
| 513 | VXN | 1 |
| 514 | WSCD2 | 1 |
| 515 | XKR4 | 1 |
| 516 | ZBTB16 | 1 |
| 517 | ZDHHC22 | 1 |
| 518 | ZFHX4 | 1 |
| 519 | ZMAT4 | 1 |
| 520 | ZNF385B | 1 |
| 521 | ZNF676 | 1 |
| 522 | ZNF728 | 1 |

As shown in Table 3, secondly selected first molecular subtypes are AADACL2, ABCA6, ABCA8, ABCA9, ABCB5, ABI3BP, ACADL, ACSM5, ACTG2, ADAMTS9-AS1, ADAMTS9-AS2, ADAMTSL3, ADCYAP1R1, ADGRB3, ADH1B, ADIPOQ, ADRA1A, AFF3, AGTR1, AICDA, ALB, ANGPTL1, ANGPTL5, ANGPTL7, ANK2, ANKS1B, ANXA8L1, APOA2, APOB, APOC3, AQP4, AQP8, ARPP21, ART4, ASB5, ASPA, ASTN1, ATCAY, ATP1A2, ATP2B2, ATP2B3, AVPR1B, B3GALT5-AS1, BCHE, BEST4, BHMT2, BLOC1S5-TXNDC5, BMP3, BRINP3, BVES, BVES-AS1, C14orf180, C1QTNF7, C7, C8orf88, CA1, CA2, CA7, CACNA2D1, CADM2, CADM3, CALN1, CARTPT, CASQ2, CAVIN2, CCBE1, CCDC144B, CCDC158, CCDC160, CCDC169, CCN5, CD300LG, CDH10, CDH19, CDKN2B-AS1, CDO1, CHRDL1, CHRM2, CHST9, CIDEA, CILP, CLCA4, CLCNKB, CLDN8, CLEC3B, CLEC4M, CLVS2, CMA1, CNGA3, CNN1, CNR1, CNTN1, CNTN2, CNTNAP4, COL19A1, CP, CPEB1, CPXM2, CR2, CRP, CTNNA3, CTSG, CYP1B1, DAO, DCLK1, DDR2, DES, DHRS7C, DIRAS2, DPP6, DPT, EBF2, ECRG4, ELAVL4, EPHA5, EPHA6, EPHA7, ERICH3, EVX2, FABP4, FAM106A, FAM133A, FAM135B, FAM180B, FDCSP, FGF10, FGF13-AS1, FGF14, FGFBP2, FGG, FGL1, FHL1, FILIP1, FLNC, FMO2, FRMD6-AS2, FRMPD4, FUT9, GABRA5, GABRG2, GALR1, GAP43, GAS1RR, GC, GCG, GDF6, GFRA1, GNAO1, GPM6A, GPR119, GPR12, GPRACR, GRIA2, GRIN2A, GTF2IP1, GUCA2B, HAND1, HAND2, HAND2-AS1, HEPACAM, HP, HPCAL4, HRG, HRK, HSPB8, HTR2B, IGSF10, IGSF11, IRX6, ISM1, KCNA1, KCNB1, KCNC2, KCNK2, KCNMA1, KCNMB1, KCNQ5, KCNT2, KCTD8, KERA, KHDRBS2, KIAA0408, KIF1A, KRT222, KRT24, KRTAP13-2, LCN10, LDB3, LEP, LGI1, LIFR, LINC00504, LINC00507, LINC00682, LINC00924, LINC01266, LINC01352, LINC01474, LINC01505, LINC01697, LINC01798, LINC01829, LINC02015, LINC02023, LINC02185, LINC02268, LINC02408, LINC02544, LIX1, LMO3, LMOD1, LOC100506289, LOC101928731, LOC102724050, LOC107986321, LOC283856, LOC440434, LOC729558, LONRF2, LRAT, LRCH2, LRRC3B, LRRC4C, LRRTM4, LVRN, LYVE1, MAB21L1, MAB21L2, MAGEE2, MAMDC2, MAPK4, MASP1, MEF2C-AS1, MEOX2, METTL24, MFAP5, MGAT4C, MGP, MICU3, MIR133A1HG, MIR8071-1, MMRN1, MORN5, MPPED2, MRGPRE, MS4A1, MS4A12, MSRB3, MUSK, MYH11, MYH2, MYLK, MYO3A, MYOC, MYOCD, MYOM1, MYOT, MYT1L, NALCN, NAP1L2, NBEA, NECAB1, NEFL, NEFM, NEGR1, NETO1, NEUROD1, NEXMIF, NEXN, NGB, NIBAN1, NLGN1, NOS1, NOVA1, NPR3, NPTX1, NPY2R, NRG3, NRK, NRSN1, NRXN1, NSG2, NTNG1, NTRK3, NUDT10, OGN, OLFM3, OMD, OTOP2, OTOP3, P2RX2, P2RY12, PAK3, PAPPA2, PCDH10, PCDH11X, PCDH9, PCOLCE2, PCP4L1, PCSK2, PDZRN4, PEG3, PENK, PGM5, PGM5-AS1, PGM5P4-AS1, PGR, PHOX2B, PI16, PIK3C2G, PIRT, PKHD1L1, PLAAT5, PLCXD3, PLD5, PLIN1, PLIN4, PLN, PLP1, PMP2, POPDC2, POU3F4, PPP1R1A, PRDM6, PRELP, PRG4, PRIMA1, PROKR1, PTCHD1, PTGIS, PTPRQ, PTPRZ1, PYGM, PYY, RANBP3L, RBFOX3, RBM20, RELN, RERGL, RGS13, RGS22, RIC3, RIMS4, RNF150, RNF180, RORB, RSPO2, SCARA5, SCGN, SCN2B, SCN7A, SCN9A, SCNN1G, SCRG1, SEMA3E, SERTM1, SERTM2, SFRP1, SFRP2, SFTPA1, SGCG, SHISAL1, SLC13A5, SLC17A8, SLC30A10, SLC4A4, SLC5A7, SLC6A2, SLC7A14, SLIT2, SLITRK2, SLITRK3, SLITRK4, SMIM28, SMYD1, SNAP25, SNAP91, SORCS1, SORCS3, SPHKAP, SPIB, SPOCK3, SST, ST8SIA3, STMN2, STMN4, STON1-GTF2A1L, STUM, SV2B, SYNM, SYNPO2, SYT10, SYT4, SYT6, TACR1, TAFA4, TCEAL2, TCEAL5, TCF23, TENM1, THBS4, TLL1, TMEFF2, TMEM100, TMEM35A, TMIGD1, TMOD1, TNNT3, TNS1, TNXB, TRARG1, TRDN, UGT2B10, UGT2B4, UNC80, VEGFD, VGLL3, VIT, VSTM2A, VXN, WSCD2, XKR4, ZBTB16, ZDHHC22, ZFHX4, ZMAT4, ZNF385B, ZNF676, and ZNF728.

Meanwhile, secondly selected second molecular subtypes are ADAT3, ANP32D, BHLHA9, BOD1L2, C4orf48, CCDC85B, CDH16, CLMAT3, CSNK1A1L, CTU1, DBET, DDC-AS1, DEFA5, EIF3IP1, FAM173A, FEZF2, FOXI3, FRMD8P1, GALR3, GJD3, GPR25, HBA1, HES4, HIST1H4A, HIST1H4L, HLA-L, IGFBP7-AS1, ITLN2, KCNE1B, LCN15, LKAAEAR1, LOC101927795, LOC101927972, LOC101928372, LOC344967, LRRC26, MAGEA10, MESP1, MIR203A, MIR324, MIR3661, MIR4449, MIR4479, MIR4665, MIR4737, MIR4767, MIR6807, MIR6858, MIR6891, MIR8075, NACA2, NOXO1, ONECUT3, PCSK1N, PDF, PITPNM2-AS1, PNMA5, PRR7, PRSS2, PRSS56, PTGER1, PTTG3P, REG3A, RNA5S9, RNU4-1, RNU5A-1, RNU5B-1, RNU5E-1, RNU6ATAC, RNY1, RPL29P2, RPRML, SBF1P1, SHISAL2B, SKOR2, SLC32A1, SMARCA5-AS1, SMCR5, SNHG25, SNORA36A, SNORD30, SNORD38A, SNORD3B-2, SNORD41, SNORD48, TMEM160, TMEM238, TPGS1, TRAPPC5, UBE2NL, WBP11P1, and ZAR1.

Noticeably, the classifier gene templates include pseudogenes, miRNA and non-coding genes, which are generally excluded from this type of analysis, which may explain why robust subtype classifiers have not been reported so far.

### [Preparation Example 111 Development of classifier for newly found molecular subtype (2)

To confirm other possible versions of classifier gene templates, depending on the thresholds used in PAM analysis, a slightly different list of template genes having similar major contributing genes was found. Such template genes can be used with similar clinical utility. Tables 4 to 7 are templates that can replace the first or secondly-selected gene templates. In the molecular subtype items of Tables 4 to 7 below, 1 denotes the first molecular subtype, and 2 denotes the second molecular subtype.

**[Table 4]**

| | Gene | Molecular subtype |
|---|---|---|
| 1 | GTF2IP1 | 2 |
| 2 | TBC1D3L | 2 |
| 3 | MIR4477B | 2 |
| 5 | BLOC1S5-TXNDC5 | 2 |
| 6 | HIST2H3C | 2 |
| 7 | CTAGE8 | 2 |
| 8 | HNRNPA1P33 | 2 |
| 9 | LOC440434 | 2 |
| 10 | GOLGA8K | 2 |
| 11 | TMEM160 | 1 |
| 12 | FEZF2 | 1 |
| 13 | C10orf131 | 2 |
| 14 | TRAPPC5 | 1 |
| 15 | KRT222 | 2 |
| 16 | ACADL | 2 |
| 17 | LOC101929607 | 2 |
| 18 | SNHG25 | 1 |
| 19 | SNORD38A | 1 |
| 20 | LOC644838 | 2 |
| 21 | KIAA0408 | 2 |
| 22 | TCEAL2 | 2 |
| 23 | C4orf48 | 1 |
| 24 | LOC642131 | 2 |
| 25 | PLGLB2 | 2 |
| 26 | FAM47E-STBD1 | 2 |
| 27 | MIR186 | 2 |
| 28 | ADAMTS9-AS1 | 2 |
| 29 | TVP23C-CDRT4 | 2 |
| 30 | PGM5-AS1 | 2 |
| 31 | SLITRK4 | 2 |
| 32 | MIR3661 | 1 |
| 33 | SEMA3E | 2 |
| 34 | ZNF676 | 2 |
| 35 | PRR7 | 1 |
| 36 | PGM5P3-AS1 | 2 |
| 37 | KIAA2022 | 2 |
| 38 | LONRF2 | 2 |
| 39 | PLCXD3 | 2 |
| 40 | NLGN1 | 2 |
| 41 | LOC440311 | 1 |
| 42 | EPHA6 | 2 |
| 43 | LOC100507387 | 2 |
| 44 | PDF | 1 |
| 45 | GRIN2A | 2 |
| 46 | LOC105369187 | 2 |
| 47 | LINC01537 | 2 |
| 48 | EIF3IP1 | 1 |
| 49 | FAM35BP | 2 |
| 50 | BCHE | 2 |
| 51 | OPA1-AS1 | 2 |
| 52 | TPGS1 | 1 |
| 53 | GAS1RR | 2 |
| 54 | NOL12 | 2 |
| 55 | LINC01266 | 2 |
| 56 | LINC00504 | 2 |
| 57 | COL25A1 | 2 |
| 58 | LOC101928509 | 2 |
| 59 | SNORD30 | 1 |
| 60 | ATP2B2 | 2 |
| 61 | NOXO1 | 1 |
| 62 | MIR4449 | 1 |
| 63 | LINC01489 | 2 |
| 64 | FRMPD4 | 2 |
| 65 | LINC00670 | 2 |
| 66 | CCDC158 | 2 |
| 67 | HCG23 | 2 |
| 68 | CTU1 | 1 |
| 69 | AGTR1 | 2 |
| 70 | LOC102467147 | 2 |
| 71 | FAM173A | 1 |
| 72 | GOLGA8N | 2 |
| 73 | PCDH10 | 2 |
| 74 | MIR3911 | 2 |
| 75 | TICAM2 | 2 |
| 76 | LGI1 | 2 |
| 77 | MYOC | 2 |
| 78 | SCN7A | 2 |
| 79 | MEF2C-AS1 | 2 |
| 80 | SNORD3A | 2 |
| 82 | KCNQ5 | 2 |
| 83 | CCL16 | 2 |
| 84 | NEXN | 2 |
| 85 | MYH8 | 2 |
| 86 | LOC100507073 | 2 |
| 87 | SIAH3 | 2 |
| 90 | GRAPL | 2 |
| 92 | FILIP 1 | 2 |

**[Table 5]**

| | Gene | Molecular subtype |
|---|---|---|
| 1 | GTF2IP1 | 2 |
| 2 | TBC1D3L | 2 |
| 3 | MIR4477B | 2 |
| 5 | BLOC1S5-TXNDC5 | 2 |
| 6 | HIST2H3C | 2 |
| 7 | CTAGE8 | 2 |
| 8 | HNRNPA1P33 | 2 |
| 9 | LOC440434 | 2 |
| 10 | GOLGA8K | 2 |
| 11 | TMEM160 | 1 |
| 12 | FEZF2 | 1 |
| 13 | C10orf131 | 2 |
| 14 | TRAPPC5 | 1 |
| 15 | KRT222 | 2 |
| 16 | ACADL | 2 |
| 17 | LOC101929607 | 2 |
| 18 | SNHG25 | 1 |
| 19 | SNORD38A | 1 |
| 20 | LOC644838 | 2 |
| 21 | KIAA0408 | 2 |
| 22 | TCEAL2 | 2 |
| 23 | C4orf48 | 1 |
| 24 | LOC642131 | 2 |
| 25 | PLGLB2 | 2 |
| 26 | FAM47E-STBD1 | 2 |
| 27 | MIR186 | 2 |
| 28 | ADAMTS9-AS1 | 2 |
| 29 | TVP23C-CDRT4 | 2 |
| 30 | PGM5-AS1 | 2 |
| 31 | SLITRK4 | 2 |
| 32 | MIR3661 | 1 |
| 33 | SEMA3E | 2 |
| 34 | ZNF676 | 2 |
| 35 | PRR7 | 1 |
| 36 | PGM5P3-AS1 | 2 |
| 37 | KIAA2022 | 2 |
| 38 | LONRF2 | 2 |
| 39 | PLCXD3 | 2 |
| 40 | NLGN1 | 2 |
| 41 | LOC440311 | 1 |
| 42 | EPHA6 | 2 |
| 43 | LOC100507387 | 2 |
| 44 | PDF | 1 |
| 45 | GRIN2A | 2 |
| 46 | LOC105369187 | 2 |
| 47 | LINC01537 | 2 |
| 48 | EIF3IP1 | 1 |
| 49 | FAM35BP | 2 |
| 50 | BCHE | 2 |
| 51 | OPA1-AS1 | 2 |
| 52 | TPGS1 | 1 |
| 53 | GAS1RR | 2 |
| 54 | NOL12 | 2 |
| 55 | LINC01266 | 2 |
| 56 | LINC00504 | 2 |
| 57 | COL25A1 | 2 |
| 58 | LOC101928509 | 2 |
| 59 | SNORD30 | 1 |
| 60 | ATP2B2 | 2 |
| 61 | NOXO1 | 1 |
| 62 | MIR4449 | 1 |
| 63 | LINC01489 | 2 |
| 64 | FRMPD4 | 2 |
| 65 | LINC00670 | 2 |
| 66 | CCDC158 | 2 |
| 67 | HCG23 | 2 |
| 68 | CTU1 | 1 |
| 69 | AGTR1 | 2 |
| 70 | LOC102467147 | 2 |
| 71 | FAM173A | 1 |
| 72 | GOLGA8N | 2 |
| 73 | PCDH10 | 2 |
| 74 | MIR3911 | 2 |
| 75 | TICAM2 | 2 |
| 76 | LGI1 | 2 |
| 77 | MYOC | 2 |
| 78 | SCN7A | 2 |
| 79 | MEF2C-AS1 | 2 |
| 80 | SNORD3A | 2 |
| 81 | LCN10 | 2 |
| 82 | KCNQ5 | 2 |
| 83 | CCL16 | 2 |
| 84 | NEXN | 2 |
| 85 | MYH8 | 2 |
| 86 | LOC100507073 | 2 |
| 87 | SIAH3 | 2 |
| 88 | CCDC85B | 1 |
| 89 | MIR133A1HG | 2 |
| 90 | GRAPL | 2 |
| 91 | SFTPA1 | 2 |
| 92 | FILIP 1 | 2 |
| 93 | ADGRB3 | 2 |
| 94 | CCDC144B | 2 |
| 95 | SYT4 | 2 |
| 96 | BVES-AS1 | 2 |
| 97 | CFHR1 | 2 |
| 98 | RAB6C | 2 |
| 99 | ADAT3 | 1 |
| 100 | SPOCK3 | 2 |
| 101 | CTAGE9 | 2 |
| 102 | SLC35F4 | 2 |
| 103 | SEMA3D | 2 |
| 104 | GLUD1P7 | 2 |
| 105 | GRIA2 | 2 |
| 106 | KCTD8 | 2 |
| 107 | LINC01352 | 2 |
| 108 | MEIS1-AS2 | 2 |
| 109 | MROH7-TTC4 | 2 |
| 110 | MIR4668 | 2 |
| 111 | LOC729558 | 2 |
| 112 | OR7E12P | 2 |
| 113 | RANBP3L | 2 |
| 114 | SCN9A | 2 |
| 115 | EIF1AX-AS1 | 2 |
| 116 | FGF13-AS1 | 2 |
| 117 | ZNF727 | 2 |
| 118 | LOC102724663 | 2 |
| 119 | LOC283856 | 2 |
| 120 | BRDT | 2 |
| 121 | SGCG | 2 |
| 122 | SLC26A5 | 2 |
| 123 | TCEAL6 | 2 |
| 124 | LINGO2 | 2 |
| 125 | LRRC3B | 2 |
| 126 | PLN | 2 |
| 127 | CCDC54 | 2 |
| 128 | FOXI3 | 1 |
| 129 | CFHR3 | 2 |
| 130 | ANKRD20A1 | 2 |
| 131 | ARHGEF18 | 2 |
| 132 | EPHA5 | 2 |
| 133 | MIR6858 | 1 |
| 134 | ZCCHC5 | 2 |
| 135 | ZNF728 | 2 |
| 136 | KCNB1 | 2 |
| 137 | ZNF157 | 2 |
| 138 | LOC283683 | 2 |
| 139 | LOC100129216 | 2 |
| 140 | SLITRK2 | 2 |
| 141 | TCEAL5 | 2 |
| 142 | CLVS2 | 2 |
| 143 | C11orf88 | 2 |
| 144 | FAM133A | 2 |
| 145 | CDH19 | 2 |
| 146 | MORN5 | 2 |
| 147 | RBAK-RBAKDN | 2 |
| 148 | ZEB2-AS1 | 2 |
| 149 | ST3GAL6-AS1 | 2 |
| 150 | NRG3 | 2 |
| 151 | LEP | 2 |
| 152 | ANO3 | 2 |
| 153 | PGM5P3-AS1.1 | 2 |
| 154 | HLX-AS1 | 2 |
| 155 | LINC01505 | 2 |
| 156 | MACC1-AS1 | 2 |
| 157 | RALGAPA1P1 | 2 |
| 158 | MIR103A2 | 2 |
| 159 | DDC-AS1 | 1 |
| 160 | LOC101927588 | 2 |
| 161 | TMEM238 | 1 |
| 162 | HSPE1-MOB4 | 2 |
| 163 | GDF5 | 2 |
| 164 | BOLL | 2 |
| 165 | LINC01449 | 2 |
| 166 | GAP43 | 2 |
| 167 | LOC102724050 | 2 |
| 168 | FGF10-AS1 | 2 |
| 169 | TGFB2-AS1 | 2 |
| 170 | LINC01474 | 2 |
| 171 | GJD4 | 2 |
| 172 | LOC100506289 | 2 |
| 173 | C6orf58 | 2 |
| 174 | CIDEB | 2 |
| 175 | FRMD6-AS2 | 2 |
| 176 | USP32P2 | 2 |
| 177 | VGLL3 | 2 |
| 178 | LINC00862 | 2 |
| 179 | MUM1L1 | 2 |
| 180 | NKAPL | 2 |
| 181 | DPYS | 2 |
| 182 | SNURF | 2 |
| 183 | HFM1 | 2 |
| 184 | PDZRN4 | 2 |
| 185 | MIR8075 | 1 |
| 186 | SCRG1 | 2 |
| 187 | LOC101929595 | 2 |
| 189 | SLITRK3 | 2 |
| 190 | NUDT10 | 2 |
| 191 | LOC105373878 | 2 |
| 192 | PGP | 1 |
| 193 | SORCS3 | 2 |
| 194 | DBIL5P2 | 2 |
| 195 | SPECC1L-ADORA2A | 2 |
| 196 | MIR8071-1 | 2 |
| 197 | NDUFB8 | 2 |
| 199 | CNTN6 | 2 |
| 200 | CCBE1 | 2 |
| 201 | ACSM5 | 2 |
| 203 | HES4 | 1 |
| 204 | ASTN1 | 2 |
| 205 | PMP2 | 2 |
| 206 | EEF1G | 2 |
| 207 | ANGPTL1 | 2 |
| 209 | GALR1 | 2 |
| 210 | CNTN1 | 2 |
| 211 | SYT16 | 2 |
| 212 | MYH2 | 2 |
| 213 | MUSTN1 | 2 |
| 214 | MIR519A2 | 2 |
| 215 | ENDOG | 1 |
| 216 | LOC440895 | 2 |
| 217 | LOC102724488 | 2 |
| 218 | MIR3149 | 2 |
| 219 | RBM27 | 2 |
| 220 | LOC441666 | 2 |
| 221 | COMTD1 | 1 |
| 222 | ABCB5 | 2 |
| 223 | SOGA3.1 | 2 |
| 224 | ZNF747 | 2 |
| 225 | RAET1E-AS1.1 | 1 |
| 227 | IL12A-AS1 | 2 |
| 228 | MIR325HG | 2 |
| 229 | ADRA1A | 2 |
| 232 | NRXN1 | 2 |
| 233 | LRRC26 | 1 |
| 236 | CELF4 | 2 |
| 237 | CCDC144A | 2 |
| 238 | SYNPO2 | 2 |
| 239 | ZNF771 | 1 |
| 240 | KLF17 | 2 |
| 242 | SFTA1P | 2 |
| 243 | ZSCAN23 | 2 |
| 244 | CYP8B1 | 2 |
| 245 | CASQ2 | 2 |
| 247 | MYH11 | 2 |
| 248 | PRH1-PRR4 | 2 |
| 249 | GPR21 | 2 |
| 253 | MIR573 | 2 |
| 255 | SPAG6 | 2 |
| 257 | MIR4665 | 1 |
| 261 | LOC101926940 | 2 |
| 262 | ST8SIA3 | 2 |
| 265 | PALM2.1 | 2 |
| 269 | LOC101929095 | 2 |
| 270 | GOLGA8R | 2 |
| 272 | MIR659 | 2 |
| 276 | MIR4645 | 2 |
| 282 | RIC3 | 2 |
| 285 | TMEFF2 | 2 |
| 289 | AKAP12 | 2 |
| 303 | ABCA9 | 2 |

**[Table 6]**

| | Gene | Molecular subtype |
|---|---|---|
| 1 | GTF2IP1 | 2 |
| 2 | TBC1D3L | 2 |
| 3 | MIR4477B | 2 |
| 5 | BLOC1S5-TXNDC5 | 2 |
| 6 | HIST2H3C | 2 |
| 7 | CTAGE8 | 2 |
| 8 | HNRNPA1P33 | 2 |
| 9 | LOC440434 | 2 |
| 10 | GOLGA8K | 2 |
| 11 | TMEM160 | 1 |
| 12 | FEZF2 | 1 |
| 13 | C10orf131 | 2 |
| 14 | TRAPPC5 | 1 |
| 15 | KRT222 | 2 |
| 16 | ACADL | 2 |
| 17 | LOC101929607 | 2 |
| 18 | SNHG25 | 1 |
| 19 | SNORD38A | 1 |
| 20 | LOC644838 | 2 |
| 21 | KIAA0408 | 2 |
| 22 | TCEAL2 | 2 |
| 23 | C4orf48 | 1 |
| 24 | LOC642131 | 2 |
| 25 | PLGLB2 | 2 |
| 26 | FAM47E-STBD1 | 2 |
| 27 | MIR186 | 2 |
| 28 | ADAMTS9-AS1 | 2 |
| 29 | TVP23C-CDRT4 | 2 |
| 30 | PGM5-AS1 | 2 |
| 31 | SLITRK4 | 2 |
| 32 | MIR3661 | 1 |
| 33 | SEMA3E | 2 |
| 34 | ZNF676 | 2 |
| 35 | PRR7 | 1 |
| 36 | PGM5P3-AS1 | 2 |
| 37 | KIAA2022 | 2 |
| 38 | LONRF2 | 2 |
| 39 | PLCXD3 | 2 |
| 40 | NLGN1 | 2 |
| 41 | LOC440311 | 1 |
| 42 | EPHA6 | 2 |
| 43 | LOC100507387 | 2 |
| 44 | PDF | 1 |
| 45 | GRIN2A | 2 |
| 46 | LOC105369187 | 2 |
| 47 | LINC01537 | 2 |
| 50 | BCHE | 2 |
| 51 | OPA1-AS1 | 2 |
| 52 | TPGS1 | 1 |
| 57 | COL25A1 | 2 |
| 66 | CCDC158 | 2 |
| 68 | CTU1 | 1 |
| 71 | FAM173A | 1 |

**[Table 7]**

| | Gene | Molecular subtype |
|---|---|---|
| 1 | GTF2IP1 | 2 |
| 2 | TBC1D3L | 2 |
| 4 | MIR4477B | 2 |
| 5 | BLOC1S5-TXNDC5 | 2 |
| 6 | HIST2H3C | 2 |
| 7 | CTAGE8 | 2 |
| 8 | HNRNPA1P33 | 2 |
| 9 | GOLGA8K | 2 |
| 10 | LOC440434 | 2 |
| 11 | TMEM160 | 1 |
| 12 | KRT222 | 2 |
| 13 | TRAPPC5 | 1 |
| 14 | C10orf131 | 2 |
| 15 | FEZF2 | 1 |
| 16 | LOC101929607 | 2 |
| 17 | SNHG25 | 1 |
| 18 | SNORD38A | 1 |
| 19 | ACADL | 2 |
| 20 | LOC642131 | 2 |
| 21 | C4orf48 | 1 |
| 22 | PLGLB2 | 2 |
| 23 | SEMA3E | 2 |
| 24 | PGM5-AS1 | 2 |
| 25 | PLCXD3 | 2 |
| 26 | ZNF676 | 2 |
| 27 | LOC644838 | 2 |
| 28 | KIAA0408 | 2 |
| 29 | TCEAL2 | 2 |
| 30 | PGM5P3-AS1 | 2 |
| 31 | FAM47E-STBD1 | 2 |
| 32 | SLITRK4 | 2 |
| 33 | ADAMTS9-AS1 | 2 |
| 34 | MIR186 | 2 |
| 35 | TVP23C-CDRT4 | 2 |
| 36 | LOC100507387 | 2 |
| 37 | KIAA2022 | 2 |
| 38 | LONRF2 | 2 |
| 39 | MIR3661 | 1 |
| 40 | PRR7 | 1 |
| 41 | NLGN1 | 2 |
| 42 | GAS1RR | 2 |
| 43 | FAM35BP | 2 |
| 44 | LOC440311 | 1 |
| 45 | PDF | 1 |
| 46 | LINC01266 | 2 |
| 47 | EIF3IP1 | 1 |
| 48 | LINC01537 | 2 |
| 49 | GRIN2A | 2 |
| 50 | SNORD30 | 1 |
| 51 | LOC105369187 | 2 |
| 52 | EPHA6 | 2 |
| 53 | LINC01489 | 2 |
| 54 | TPGS1 | 1 |
| 55 | BCHE | 2 |
| 56 | LGI1 | 2 |
| 57 | OPA1-AS1 | 2 |
| 58 | MYOC | 2 |
| 59 | CCDC144B | 2 |
| 60 | NEXN | 2 |
| 61 | FAM173A | 1 |
| 62 | CTU1 | 1 |
| 63 | SCN7A | 2 |
| 64 | LINC00504 | 2 |
| 65 | SYT4 | 2 |
| 66 | LOC100507073 | 2 |
| 67 | ATP2B2 | 2 |
| 68 | NOL12 | 2 |
| 69 | MIR133A1HG | 2 |
| 70 | COL25A1 | 2 |
| 71 | BVES-AS1 | 2 |
| 72 | MYH8 | 2 |
| 73 | FRMPD4 | 2 |
| 74 | SPOCK3 | 2 |
| 76 | FILIP1 | 2 |
| 77 | MIR4449 | 1 |
| 78 | LOC102467147 | 2 |
| 79 | KCNQ5 | 2 |
| 80 | MEF2C-AS1 | 2 |
| 81 | LINC01352 | 2 |
| 82 | HCG23 | 2 |
| 83 | CCDC158 | 2 |
| 84 | LINC00670 | 2 |
| 85 | CCDC85B | 1 |
| 86 | PCDH10 | 2 |
| 87 | CFHR1 | 2 |
| 88 | TICAM2 | 2 |
| 89 | KCTD8 | 2 |
| 90 | NOXO1 | 1 |
| 91 | GRIA2 | 2 |
| 92 | ADGRB3 | 2 |
| 93 | OR7E12P | 2 |
| 94 | ZNF727 | 2 |
| 96 | GOLGA8N | 2 |
| 97 | MIR4668 | 2 |
| 99 | AGTR1 | 2 |
| 101 | SCN9A | 2 |

### [Preparation Example 12] Development of classifier for newly found molecular subtype (3)

In addition, Table 8 below is a template that can replace a gene subtype corresponding to the first molecular subtype, and Table 9 is a template that can replace a gene subtype corresponding to the second molecular subtype.

**[Table 8]**

| No. | Gene | Ensembl | Protein | Synonym |
|---|---|---|---|---|
| 1 | PMP2 | ENSG00000087245 | Myelin P2 protein | FABP8, M-FABP, MP2, P2, peripheral myelin protein 2, Myelin P2 protein, CMT1G |
| 2 | AGTR1 | ENSG00000144891 | Angiotensin II receptor type 1 | AG2S, AGTR1B, AT1, AT1AR, AT1B, AT1BR, AT1R, AT2R1, HAT1R |
| 3 | PLCXD3 | ENSG00000182836 | PI-PLC X domain-containing protein 3 | Phosphatidylinositol Specific Phospholipase C X Domain Containing 3, Phosphatidylinositol-Specific Phospholipase C, X Domain Containing, PLCXD3 |
| 4 | ARHGAP26-AS1 | ENSG00000226272 | - | ARHGAP26 Antisense RNA 1, NONHSAG041808.2 91, HSALNG0045520, ENSG00000226272 |
| 5 | TCEAL6 | ENSG00000204071 | Transcription elongation factor A (SII)-like 6 | Transcription Elongation Factor S-II Protein-Like 6, Transcription Elongation Factor A Protein-Like 6, Transcription Elongation Factor A (SII)-Like 6, TCEA-Like Protein 6, WEX2, Transcription Elongation Factor A (SII)-Like 3, Tceal3 |
| 6 | ANKRD1 | ENSG00000148677 | Ankyrin repeat domain-containing protein 1 | Ankyrin Repeat Domain 1, CARP, Ankyrin Repeat Domain 1 (Cardiac Muscle), Cytokine-Inducible Gene C-193 Protein, Cytokine-Inducible Nuclear Protein, Cardiac Ankyrin Repeat Protein, C-193, CVARP, MCARP, ALRP, Epididymis Secretory Sperm Binding Protein, Liver Ankyrin Repeat Domain 1, BA320F15.2, ANKRD1, HA1A2, C193 |

**[Table 9]**

| No. | Gene | Ensembl | Protein | Synonym |
|---|---|---|---|---|
| 1 | PGP | ENSG00000184207 | P-glycoprotein 1 | ABCB1, ABC20, CD243, CLCS, GP170, MDR1, PGY1, ATP binding cassette subfamily B member 1, P-glycoprotein, P-gp |
| 2 | SLC26A3 | ENSG00000091138 | Chloride anion exchange) | CLD, DRA, solute carrier family 26 member 3 |
| 3 | HIST1H4C | ENSG00000197061 | Histone H4 | H4C3, H4/g, H4FG, dJ221C16.1, histone cluster 1, H4c, histone cluster 1 H4 family member c, H4 clustered histone 3, H4C5, H4C4, H4C9, H4C12, H4-16, H4C13, H4C11, H4C1, H4C14, H4C15, H4C8, H4C6, H4C2 |
| 4 | SNORD69 | ENSG00000212452 | - | snoRNA HBII-210, RF00574 |
| 5 | RUVBL2 | ENSG00000183207 | RuvB-like 2 | ECP51, INO80J, REPTIN, RVB2, TIH2, TIP48, TIP49B, CGI-46, ECP-51, TAP54-beta, RuvB like AAA ATPase 2 |
| 6 | RAB19 | ENSG00000146955 | Ras-related protein Rab-19 | Member RAS Oncogene Family, RAB19B, GTP-Binding Protein RAB19B |
| 7 | HIST2H2AC | ENSG00000184260 | Histone H2A type 2-C | H2AC20, H2A, H2A-GL101, H2A/q, H2AFQ, histone cluster 2, H2ac, histone cluster 2 H2A family member c, H2A clustered histone 20 |

### [Experimental Example 11 Verification of clinical usefulness of newly-developed molecular subtype classifier (1)

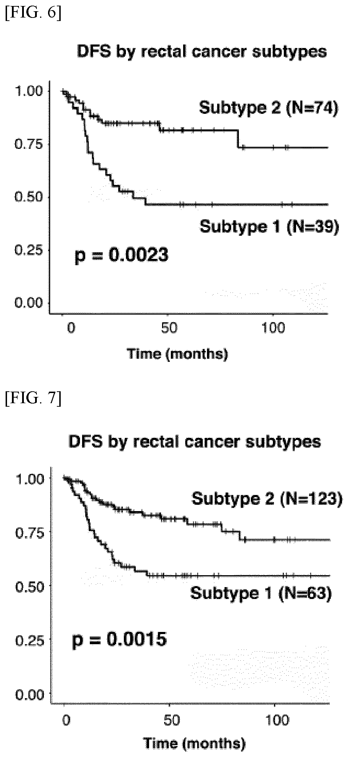

FIGS. 6 and 7 show RNAseq classification data of 230 rectal cancer patients from the Yonsei Cancer Center according to one embodiment of the present invention.

To classify pretreated biopsy samples from 230 rectal cancer patients treated at the Yonsei Cancer Center, a Nearest Template Prediction (NTP) method was used. Table 9 below shows the correlation between molecular subtypes classified by a primarily-selected 94-gene set and a response to preoperative chemoradiotherapy.

**[Table 10]**

| | First molecular subtype | Second molecular subtype | Total |
|---|---|---|---|
| Pathologic incomplete response | 33 | 48 | 81 |
| Pathologic complete response | 6 (15.4%) | 26 (35.1%) | 32 (28.3%) |
| Total | 39 | 74 | 113 |

230 rectal cancer patients were classified by applying the primarily-selected 94-gene set using an NTP method. 113 patients were reliably classified (false discovery rate <0.2), but it was impossible to accurately classify 97 patients. Among 115 that are able to be classified, the pCR rate of the first molecular subtype was 15.4% (6 of 39 patients), whereas the pCR rate of the second molecular subtype was two-fold higher than that of the first molecular subtype, which is 35.1% (26 of 74 patients) (chi-squared = 3.98, p = 0.046).

FIG. 6 shows the disease-free survival (DFS) rates of the patients according to subtypes classified by the primarily-selected gene set. As expected from the correlation with low pCR, the case of the first molecular subtype (prediction = 1) was associated with a worse DFS than the second molecular subtype (prediction = 2) (p = 0.0023).

Table 11 below shows the correlation between subtypes classified by a secondly-selected 522-gene set and a response to preoperative chemoradiotherapy.

**[Table 11]**

| | First molecular subtype | Second molecular subtype | Total |
|---|---|---|---|
| Pathologic incomplete response | 57 | 78 | 135 |
| Pathologic complete response | 6 (9.5%) | 45 (36.6%) | 51 |
| Total | 63 (33.9%) | 123 (66.1%) | 186 |

230 rectal cancer patients were classified by applying the secondly-selected 522-gene set using an NTP method. 186 patients were reliably classified (false discovery rate <0.2), but it was impossible to accurately classify 44 patients. Among 186 that are able to be classified, the pCR rate of the first molecular subtype was 9.5% (6 of 63 patients), whereas the pCR rate of the second molecular subtype was two-fold higher than that of the first molecular subtype, which is 36.6% (45 of 123 patients) (chi-squared = 14.0, p = 0.0002).

FIG. 7 shows the disease-free survival (DFS) rates of the patients according to subtypes classified by the primarily-selected gene set. As expected from the correlation with low pCR, the case of the first molecular subtype (prediction = 1) was associated with a worse DFS than the second molecular subtype (prediction = 2) (p = 0.0015).

### [Experimental Example 2] Verification of clinical usefulness of newly developed molecular subtype classifier (2)

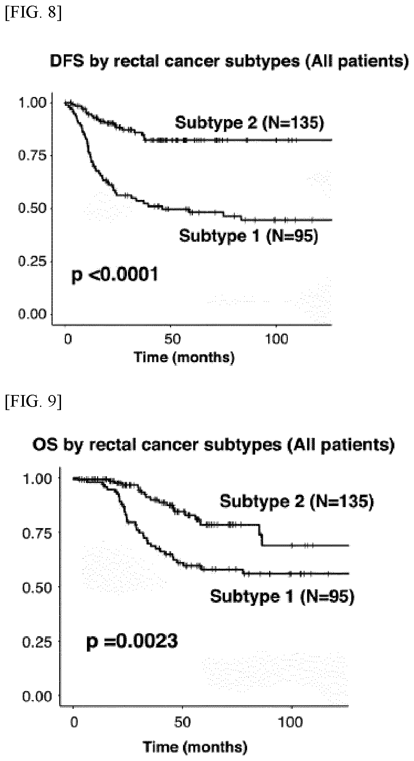

The ability to predict a rectal cancer prognosis according to the first molecular subtype in Table 8 and the second molecular subtype in Table 9 in surgery after preoperative chemoradiotherapy was confirmed, and indicated as DFS (FIG. 8) and OS (FIG. 9). However, the verification was performed for the entire patient cohort (N=230).

As shown in FIGS. 8 and 9, it was confirmed that in the first molecular subtype, compared to the second molecular subtype, DFS and OS are low in surgery after preoperative chemoradiotherapy, and when the molecular subtypes in FIGS. 8 and 9 were checked, it can be seen that the ability to predict a prognosis after rectal cancer treatment was excellent.

### [Experimental Example 3] Confirmation of ability to predict prognosis in rectal cancer patients before treatment according to molecular subtype and pathological characteristics

Since the diagnosis of rectal cancer is made by a pathological examination using small tissue biopsy and radiodiagnosis such as CT-MRI, it is not easy to predict a prognosis of a patient before the initiation of treatment. Table 12 shows the results of univariate and multivariate analyses performed on candidate prognostic factors that can be implemented or measured before the initiation of treatment. cN_stage indicates the clinically determined degree of lymph node metastasis, and cT_stage indicates a clinically determined tumor size. In Table 12, OR indicates an odds ratio, and CI indicates a confidence interval.

The analysis results clearly show that only molecular subtypes can predict the prognosis of patients before the initiation of treatment (p<0.001). This imparts a very clinically significant meaning to molecular subtypes. Recently, the treatment of rectal cancer is shifting to total neoadjuvant therapy (TNT), which performs all possible treatments before surgery. In this case, other therapeutic agents may be considered depending on the predicted patient's prognosis. That is, in the case of the first molecular subtype, a more powerful treatment can be considered, so the molecular subtype can play an important role in discriminating the target group for a clinical trial of a novel drug under development.

**[Table 12]**

| **Prognosis** | **Variate** | **Category** | **Univariate statistical analysis** | | | **Multivariate statistical analysis** | | |
|---|---|---|---|---|---|---|---|---|
| | | | **OR** | **95% CI** | ***P*** | **OR** | 95% **CI** | ***P*** |
| DFS | cT_stage | continuous | 0.86 | 0.49 tp 1.58 | 0.629 | 0.77 | 0.39 to 1.50 | 0.442 |
| | cN stage | continuous | 1.28 | 0.81 to 2.0 | 0.288 | 1.41 | 0.87 to 2.26 | 0.159 |
| | Age | >60 vs <=60 (ref) | 1.01 | 0.57 to 1.77 | 0.980 | 1.04 | 0.58 to 1.85 | 0.894 |
| | Sex | male vs female (ref) | 0.84 | 0.47 to 1.50 | 0.547 | 0.77 | 0.43 to 1.41 | 0.402 |
| | Molecular subtype | 1 vs 2 (ref) | 2.4 | 1.38 to 4.19 | 0.002 | 2.51 | 1.43 to 4.41 | 0.001 |
| OS | cT_stage | continuous | 1.06 | 0.54 to 2.08 | 0.860 | 1.06 | 0.51 to 2.21 | 0.863 |
| | cN stage | continuous | 1.25 | 0.77 to 2.04 | 0.373 | 1.27 | 0.76 to 2.13 | 0.355 |
| | Age | >60 vs <=60 (ref) | 1.21 | 0.66 to 2.21 | 0.534 | 1.22 | 0.66 to 2.27 | 0.521 |
| | Sex | male vs female (ref) | 0.77 | 0.41 tp 1.44 | 0.415 | 0.74 | 0.39 to 1.42 | 0.368 |
| | Molecular subtype | 1 vs 2 (ref) | 1.88 | 1.03 to 3.43 | 0.039 | 1.95 | 1.07 to 3.57 | 0.030 |

### [Experimental Example 4] Confirmation of ability to predict prognosis of rectal cancer patient after neoadjuvant chemoradiotherapy according to molecular subtype and pathological characteristics

Table 13 shows the result of investigating the correlation between candidate factors and molecular subtypes that can be used to determine the prognosis of rectal cancer patients after neoadjuvant chemoradiotherapy and surgery. It shows that molecular subtypes are statistically significantly correlated with the size of cancer after treatment (ypT stage) and pCR (in the case of the first molecular subtype, the size is large and pCR is low), but are not associated with the degree of lymph node metastasis (ypN stage), or a patient's age and sex.

**[Table 13]**

| Classification | Category | First molecular subtype (EMT subtype) | Second molecular subtype (MYC subtype) | Chi square | P value |
|---|---|---|---|---|---|
| ypT | T0 | 6 | 45 | 20.288 | 0.0000438 |
| | T1 | 0 | 3 | | |
| | T2 | 15 | 15 | | |
| | T3 | 41 | 55 | | |
| | T4 | 1 | 5 | | |
| ypN | N0 | 41 | 90 | 3.7365 | 0.1544 |
| | N1 | 17 | 19 | | |
| | N2 | 5 | 14 | | |
| pCR | No-pCR | 57 | 78 | 14.001 | 0.0001827 |
| | pCR | 6 | 45 | | |
| Age | <=60 | 38 | 66 | 0.50362 | 0.4479 |
| | >60 | 25 | 57 | | |
| Sex | Female | 18 | 45 | 0.86355 | 0.3527 |
| | Male | 45 | 78 | | |

Table 14 shows the results of univariate and multivariate statistical analyses on candidate factors that can be used to determine the prognosis of rectal cancer patients after neoadjuvant chemoradiotherapy and surgery.

**[Table 14]**

| **Prognosis** | **Variate** | **Category** | **Univariate statistical analysis** | | | **Multivariate statistical analysis** | | |
|---|---|---|---|---|---|---|---|---|
| | | | **OR** | **95% CI** | ***P*** | **OR** | **95% CI** | ***P*** |
| DFS | ypT3 | ypT3/4 v ypT0/1/2 (ref) | 2.15 | 1.15 to 3.98 | 0.010 | 1.16 | 0.53 to 2.55 | 0.704 |
| | ypN | ypNO v ypN1/2 (ref) | 3.92 | 2.23 to 6.89 | < 0.001 | 3.82 | 2.00 to 7.28 | < 0.001 |
| | pCR | pCR v no pCR (ref) | 2.59 | 1.10 to 6.10 | 0.010 | 0.88 | 0.29 to 2.71 | 0.834 |
| | Molecular subtype | EMT v MYC (ref) | 2.40 | 1.37 to 4.19 | 0.002 | 2.37 | 1.33 to 4.21 | 0.003 |
| OS | ypT3 | ypT3/4 v ypT0/1/2 (ref) | 2.31 | 1.16 to 4.60 | 0.010 | 1.20 | 0.53 to 2.67 | 0.655 |
| | ypN | ypNO v ypN1/2 (ref) | 3.16 | 1.72 to 5.78 | < 0.001 | 2.67 | 1.38 to 5.18 | 0.003 |
| | pCR | pCR v no pCR (ref) | 4.01 | 1.24 to 12.98 | 0.005 | 1.78 | 0.45 to 7.07 | 0.408 |
| | Molecular subtype | EMT v MYC (ref) | 1.89 | 1.03 to 3.42 | 0.040 | 1.77 | 0.95 to 3.29 | 0.068 |

As shown in Table 14, in the univariate analysis, it can be predicted from DFS and OS that all of the cancer size after treatment (ypT stage), the degree of lymph node metastasis (ypN stage), pCR, and a molecular subtype are statistically significant. However, from the result of multivariate analysis, it is seen that only ypN stage and a molecular subtype are significant. That is, this shows that, since the ypN stage and the molecular subtype each independently affect DFS, when two factors are used together, a prognosis can be more accurately predicted. To prove this, DFS and OS according to ypN stage and a molecular subtype were investigated using Kaplan-Meier plots.

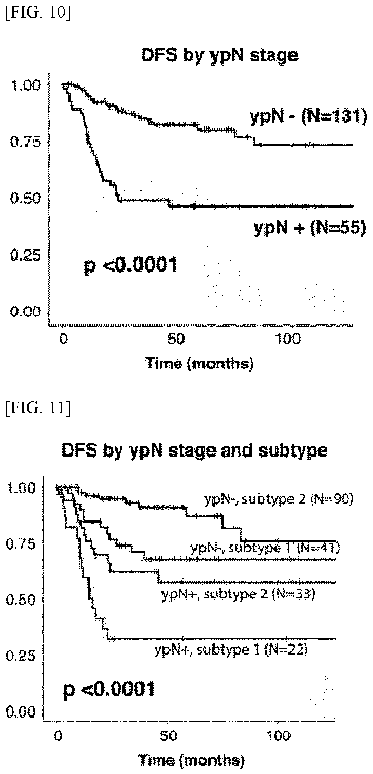

FIG. 10 is a Kaplan-Meier plot that analyzes DFS according to the presence or absence of lymph node metastasis found in surgery after treatment (ypN stage).

FIG. 11 is a Kaplan-Meier plot that analyzes DFS according to the presence or absence of lymph node metastasis found in surgery after treatment (ypN stage) and a molecular subtype.

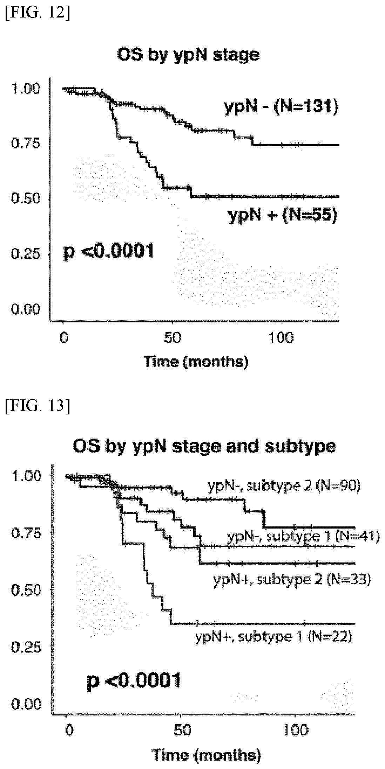

FIG. 12 is a Kaplan-Meier plot that analyzes OS according to the presence or absence of lymph node metastasis found in surgery after treatment (ypN stage).

FIG. 13 is a Kaplan-Meier plot that analyzes OS according to the presence or absence of lymph node metastasis found in surgery after treatment (ypN stage) and a molecular subtype.

When ypN stage and a molecular subtype are used together as shown in FIGS. 11 and 13, compared to when only ypN stage is used in FIGS. 10 and 12, despite standard neoadjuvant chemoradiotherapy, a patient group with an extremely high recurrence rate (60% or more recurrence within three years) and a low survival rate (OS) can be predicted in advance. This patient group is important as a target group for a clinical trial of a novel drug because it is necessary for these patients to try different therapeutic agents, other than standard therapy after surgery or before surgery following neoadjuvant chemoradiotherapy.

### [Experimental Example 5] Investigation of rectal cancer predicting ability of conventional developed CMS molecular subtype classifier

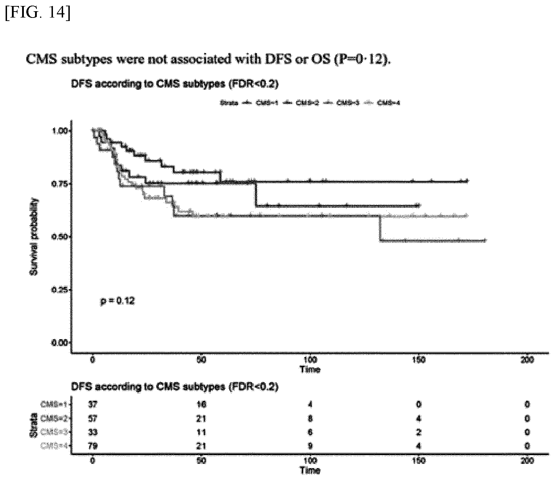

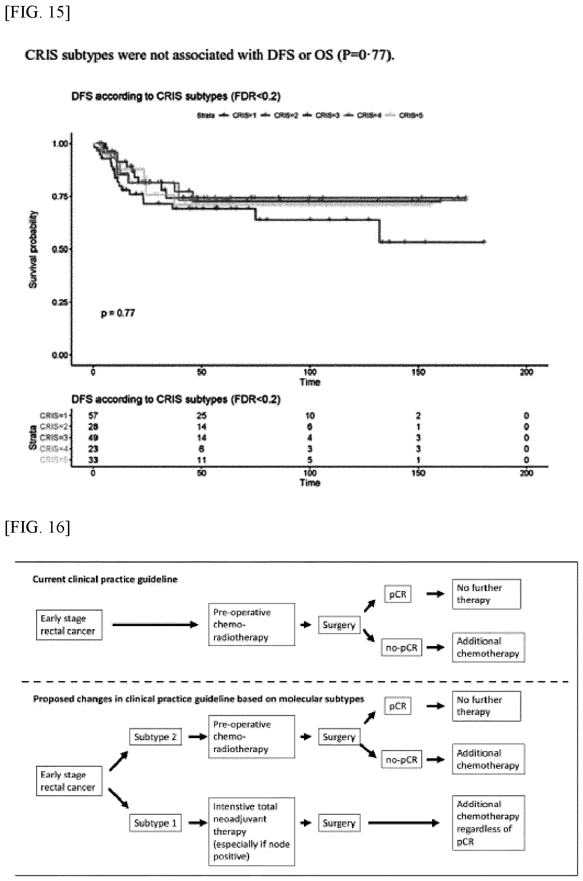

On the other hand, to investigate the rectal cancer predicting ability of a conventional classifier for CMS and CRIS subtypes, which are conventional molecular subtypes for predicting the prognosis of colorectal cancer (CRC), using NTP together with a classifier gene template provided by the CMScaller package, DFS and OS in a rectal cancer cohort when the CMS molecular subtypes were used are shown in FIG. 14, and DFS and OS obtained when the CRIS molecular subtypes were used are shown in FIG. 15.

As shown in FIGS. 14 and 15, neither CMS nor CRIS showed a statistically significant correlation with clinical evaluation variables. Specifically, since there was no significant difference in the survival rates (DFS and OS) of rectal cancer patients according to CMS molecular subtype, the ability to predict a prognosis for rectal cancer patients was not statistically significant (P=0.12). In addition, the survival rates (DFS and OS) of rectal cancer patients according to CRIS molecular subtype did not differ significantly, and the ability to predict a prognosis for rectal cancer patients was also not statistically significant (P=0.77).

### [Example 11 Rectal cancer treatment protocol according to molecular subtype and pathological characteristics

Based on Experimental Examples 1 to 4, a method of predicting the prognosis of rectal cancer according to a first molecular subtype and a second molecular subtype is shown in FIG. 16.

As shown in FIG. 16, conventionally, when pCR was achieved after surgery after standard neoadjuvant chemoradiotherapy, there was no further treatment, whereas a protocol in which, after classification according to a first molecular subtype (subtype 1) and a second molecular subtype (subtype 2) according to the present invention, (1) when the result corresponds to the second molecular subtype, and pCR is achieved after treatment, there is no further treatment, (2) when the result corresponds to the second molecular subtype and pCR is not achieved, additional chemotherapy is performed, and (3) when the result corresponds to the first molecular subtype, continuous chemotherapy is performed regardless of achieving pCR was established.

In the above, as specific parts of the specification have been described in detail, although it is clear to those skilled in the art that this specific technique is merely a preferred embodiment, the scope of the specification is not limited thereto. Thus, the substantial scope of the specification will be defined by the accompanying claims and their equivalents.

### [Industrial Availability]

The present invention relates to a composition for predicting a response to neoadjuvant chemoradiotherapy for rectal cancer or a prognosis after treatment and a prediction method using the same.

## Claims

1. **A composition** for predicting a therapeutic response to anticancer therapy or a prognosis after anticancer therapy, or identifying a target patient for neoadjuvant therapy prior to anticancer therapy in cancer patients, comprising:
an agent that measures the expression level of at least one gene of a first molecular subtype and a second molecular subtype or a protein encoded thereby,
wherein the first molecular subtype comprises one or more types of genes selected from PMP2, AGTR1, PLCXD3, TCEAL6, ANKRD1, and ARHGAP26-AS1, and
the second molecular subtype comprises one or more types of genes selected from PGP, SLC26A3, HIST1H4C, RUVBL2, RAB19, HIST2H2AC, and SNORD69.

2. The composition of claim 1, wherein the first molecular subtype further comprises one or more types of genes selected from the group consisting of AADACL2, ABCA6, ABCA8, ABCA9, ABCB5, ABI3BP, ACADL, ACSM5, ACTG2, ADAMTS9-AS1, ADAMTS9-AS2, ADAMTSL3, ADCYAP1R1, ADGRB3, ADH1B, ADIPOQ, ADRA1A, AFF3, AGTR1, AICDA, ALB, ANGPTL1, ANGPTL5, ANGPTL7, ANK2, ANKS1B, ANXA8L1, APOA2, APOB, APOC3, AQP4, AQP8, ARPP21, ART4, ASB5, ASPA, ASTN1, ATCAY, ATP1A2, ATP2B2, ATP2B3, AVPR1B, B3GALT5-AS1, BCHE, BEST4, BHMT2, BLOC1S5-TXNDC5, BMP3, BRINP3, BVES, BVES-AS1, C14orf180, C1QTNF7, C7, C8orf88, CA1, CA2, CA7, CACNA2D1, CADM2, CADM3, CALN1, CARTPT, CASQ2, CAVIN2, CCBE1, CCDC144B, CCDC158, CCDC160, CCDC169, CCN5, CD300LG, CDH10, CDH19, CDKN2B-AS1, CDO1, CHRDL1, CHRM2, CHST9, CIDEA, CILP, CLCA4, CLCNKB, CLDN8, CLEC3B, CLEC4M, CLVS2, CMA1, CNGA3, CNN1, CNR1, CNTN1, CNTN2, CNTNAP4, COL19A1, CP, CPEB1, CPXM2, CR2, CRP, CTNNA3, CTSG, CYP1B1, DAO, DCLK1, DDR2, DES, DHRS7C, DIRAS2, DPP6, DPT, EBF2, ECRG4, ELAVL4, EPHA5, EPHA6, EPHA7, ERICH3, EVX2, FABP4, FAM106A, FAM133A, FAM135B, FAM180B, FDCSP, FGF10, FGF13-AS1, FGF14, FGFBP2, FGG, FGL1, FHL1, FILIP1, FLNC, FMO2, FRMD6-AS2, FRMPD4, FUT9, GABRA5, GABRG2, GALR1, GAP43, GAS1RR, GC, GCG, GDF6, GFRA1, GNAO1, GPM6A, GPR119, GPR12, GPRACR, GRIA2, GRIN2A, GTF2IP1, GUCA2B, HAND1, HAND2, HAND2-AS1, HEPACAM, HP, HPCAL4, HRG, HRK, HSPB8, HTR2B, IGSF10, IGSF11, IRX6, ISM1, KCNA1, KCNB1, KCNC2, KCNK2, KCNMA1, KCNMB1, KCNQ5, KCNT2, KCTD8, KERA, KHDRBS2, KIAA0408, KIF1A, KRT222, KRT24, KRTAP13-2, LCN10, LDB3, LEP, LGI1, LIFR, LINC00504, LINC00507, LINC00682, LINC00924, LINC01266, LINC01352, LINC01474, LINC01505, LINC01697, LINC01798, LINC01829, LINC02015, LINC02023, LINC02185, LINC02268, LINC02408, LINC02544, LIX1, LMO3, LMOD1, LOC100506289, LOC101928731, LOC102724050, LOC107986321, LOC283856, LOC440434, LOC729558, LONRF2, LRAT, LRCH2, LRRC3B, LRRC4C, LRRTM4, LVRN, LYVE1, MAB21L1, MAB21L2, MAGEE2, MAMDC2, MAPK4, MASP1, MEF2C-AS1, MEOX2, METTL24, MFAP5, MGAT4C, MGP, MICU3, MIR133A1HG, MIR8071-1, MMRN1, MORN5, MPPED2, MRGPRE, MS4A1, MS4A12, MSRB3, MUSK, MYH11, MYH2, MYLK, MYO3A, MYOC, MYOCD, MYOM1, MYOT, MYT1L, NALCN, NAP1L2, NBEA, NECAB1, NEFL, NEFM, NEGR1, NETO1, NEUROD1, NEXMIF, NEXN, NGB, NIBAN1, NLGN1, NOS1, NOVA1, NPR3, NPTX1, NPY2R, NRG3, NRK, NRSN1, NRXN1, NSG2, NTNG1, NTRK3, NUDT10, OGN, OLFM3, OMD, OTOP2, OTOP3, P2RX2, P2RY12, PAK3, PAPPA2, PCDH10, PCDH11X, PCDH9, PCOLCE2, PCP4L1, PCSK2, PDZRN4, PEG3, PENK, PGM5, PGM5-AS1, PGM5P4-AS1, PGR, PHOX2B, PI16, PIK3C2G, PIRT, PKHD1L1, PLAAT5, PLCXD3, PLD5, PLIN1, PLIN4, PLN, PLP1, PMP2, POPDC2, POU3F4, PPP1R1A, PRDM6, PRELP, PRG4, PRIMA1, PROKR1, PTCHD1, PTGIS, PTPRQ, PTPRZ1, PYGM, PYY, RANBP3L, RBFOX3, RBM20, RELN, RERGL, RGS13, RGS22, RIC3, RIMS4, RNF150, RNF180, RORB, RSPO2, SCARA5, SCGN, SCN2B, SCN7A, SCN9A, SCNN1G, SCRG1, SEMA3E, SERTM1, SERTM2, SFRP1, SFRP2, SFTPA1, SGCG, SHISAL1, SLC13A5, SLC17A8, SLC30A10, SLC4A4, SLC5A7, SLC6A2, SLC7A14, SLIT2, SLITRK2, SLITRK3, SLITRK4, SMIM28, SMYD1, SNAP25, SNAP91, SORCS1, SORCS3, SPHKAP, SPIB, SPOCK3, SST, ST8SIA3, STMN2, STMN4, STON1-GTF2A1L, STUM, SV2B, SYNM, SYNPO2, SYT10, SYT4, SYT6, TACR1, TAFA4, TCEAL2, TCEAL5, TCF23, TENM1, THBS4, TLL1, TMEFF2, TMEM100, TMEM35A, TMIGD1, TMOD1, TNNT3, TNS1, TNXB, TRARG1, TRDN, UGT2B10, UGT2B4, UNC80, VEGFD, VGLL3, VIT, VSTM2A, VXN, WSCD2, XKR4, ZBTB16, ZDHHC22, ZFHX4, ZMAT4, ZNF385B, ZNF676, and ZNF728.

3. The composition of claim 1, wherein the second molecular subtype further comprises one or more types of genes selected from the group consisting of ADAT3, ANP32D, BHLHA9, BOD1L2, C4orf48, CCDC85B, CDH16, CLMAT3, CSNK1A1L, CTU1, DBET, DDC-AS1, DEFA5, EIF3IP1, FAM173A, FEZF2, FOXI3, FRMD8P1, GALR3, GJD3, GPR25, HBA1, HES4, HIST1H4A, HIST1H4L, HLA-L, IGFBP7-AS1, ITLN2, KCNE1B, LCN15, LKAAEAR1, LOC101927795, LOC101927972, LOC101928372, LOC344967, LRRC26, MAGEA10, MESP1, MIR203A, MIR324, MIR3661, MIR4449, MIR4479, MIR4665, MIR4737, MIR4767, MIR6807, MIR6858, MIR6891, MIR8075, NACA2, NOXO1, ONECUT3, PCSK1N, PDF, PITPNM2-AS1, PNMA5, PRR7, PRSS2, PRSS56, PTGER1, PTTG3P, REG3A, RNA5S9, RNU4-1, RNU5A-1, RNU5B-1, RNU5E-1, RNU6ATAC, RNY1, RPL29P2, RPRML, SBF1P1, SHISAL2B, SKOR2, SLC32A1, SMARCA5-AS1, SMCR5, SNHG25, SNORA36A, SNORD30, SNORD38A, SNORD3B-2, SNORD41, SNORD48, TMEM160, TMEM238, TPGS1, TRAPPC5, UBE2NL, WBP11P1, and ZAR1.

4. The composition of claim 1, wherein the anticancer therapy is chemotherapy, radiation therapy, surgical treatment or a combination thereof.

5. The composition of claim 1, wherein the anticancer therapy is standard neoadjuvant chemoradiotherapy or surgical treatment after standard neoadjuvant chemoradiotherapy.

6. The composition of claim 1, wherein the cancer is rectal cancer.

7. A kit for predicting a therapeutic response to anticancer therapy or a prognosis after anticancer therapy, or identifying a target patient for neoadjuvant therapy prior to anticancer treatment in cancer patients, comprising:
the composition of any one of claims 1 to 6.

8. The kit of claim 7, wherein the kit is an RT-PCR kit, a DNA chip kit, an ELISA kit, a protein chip kit, a rapid kit or a multiple reaction monitoring (MRM) kit.

9. A biomarker composition for predicting a therapeutic response to anticancer therapy or a prognosis after anticancer therapy, or identifying a target patient for neoadjuvant therapy prior to anticancer therapy in cancer patients, comprising:
at least one gene of a first molecular subtype and a second molecular subtype or a protein encoded thereby,
wherein the first molecular subtype comprises one or more types of genes selected from PMP2, AGTR1, PLCXD3, TCEAL6, ANKRD1, and ARHGAP26-AS1, and
the second molecular subtype comprises one or more types of genes selected from PGP, SLC26A3, HIST1H4C, RUVBL2, RAB19, HIST2H2AC, and SNORD69.

10. A method of providing information for predicting a therapeutic response to anticancer therapy or a prognosis after anticancer therapy, or identifying a target patient for neoadjuvant therapy prior to anticancer therapy, comprising:
measuring the expression level of at least one gene of a first molecular subtype and a second molecular subtype or a protein encoded thereby in a biological sample isolated from a target subject,
wherein the first molecular subtype comprises one or more types of genes selected from PMP2, AGTR1, PLCXD3, TCEAL6, ANKRD1, and ARHGAP26-AS1, and
the second molecular subtype comprises one or more types of genes selected from PGP, SLC26A3, HIST1H4C, RUVBL2, RAB19, HIST2H2AC, and SNORD69.

11. The method of claim 10, wherein the first molecular subtype further comprises one or more types of genes selected from the group consisting of AADACL2, ABCA6, ABCA8, ABCA9, ABCB5, ABI3BP, ACADL, ACSM5, ACTG2, ADAMTS9-AS1, ADAMTS9-AS2, ADAMTSL3, ADCYAP1R1, ADGRB3, ADH1B, ADIPOQ, ADRA1A, AFF3, AGTR1, AICDA, ALB, ANGPTL1, ANGPTL5, ANGPTL7, ANK2, ANKS1B, ANXA8L1, APOA2, APOB, APOC3, AQP4, AQP8, ARPP21, ART4, ASB5, ASPA, ASTN1, ATCAY, ATP1A2, ATP2B2, ATP2B3, AVPR1B, B3GALT5-AS1, BCHE, BEST4, BHMT2, BLOC1S5-TXNDC5, BMP3, BRINP3, BVES, BVES-AS1, C14orf180, C1QTNF7, C7, C8orf88, CA1, CA2, CA7, CACNA2D1, CADM2, CADM3, CALN1, CARTPT, CASQ2, CAVIN2, CCBE1, CCDC144B, CCDC158, CCDC160, CCDC169, CCN5, CD300LG, CDH10, CDH19, CDKN2B-AS1, CDO1, CHRDL1, CHRM2, CHST9, CIDEA, CILP, CLCA4, CLCNKB, CLDN8, CLEC3B, CLEC4M, CLVS2, CMA1, CNGA3, CNN1, CNR1, CNTN1, CNTN2, CNTNAP4, COL19A1, CP, CPEB1, CPXM2, CR2, CRP, CTNNA3, CTSG, CYP1B1, DAO, DCLK1, DDR2, DES, DHRS7C, DIRAS2, DPP6, DPT, EBF2, ECRG4, ELAVL4, EPHA5, EPHA6, EPHA7, ERICH3, EVX2, FABP4, FAM106A, FAM133A, FAM135B, FAM180B, FDCSP, FGF10, FGF13-AS1, FGF14, FGFBP2, FGG, FGL1, FHL1, FILIP1, FLNC, FMO2, FRMD6-AS2, FRMPD4, FUT9, GABRA5, GABRG2, GALR1, GAP43, GAS1RR, GC, GCG, GDF6, GFRA1, GNAO1, GPM6A, GPR119, GPR12, GPRACR, GRIA2, GRIN2A, GTF2IP1, GUCA2B, HAND1, HAND2, HAND2-AS1, HEPACAM, HP, HPCAL4, HRG, HRK, HSPB8, HTR2B, IGSF10, IGSF11, IRX6, ISM1, KCNA1, KCNB1, KCNC2, KCNK2, KCNMA1, KCNMB1, KCNQ5, KCNT2, KCTD8, KERA, KHDRBS2, KIAA0408, KIF1A, KRT222, KRT24, KRTAP13-2, LCN10, LDB3, LEP, LGI1, LIFR, LINC00504, LINC00507, LINC00682, LINC00924, LINC01266, LINC01352, LINC01474, LINC01505, LINC01697, LINC01798, LINC01829, LINC02015, LINC02023, LINC02185, LINC02268, LINC02408, LINC02544, LIX1, LMO3, LMOD1, LOC100506289, LOC101928731, LOC102724050, LOC107986321, LOC283856, LOC440434, LOC729558, LONRF2, LRAT, LRCH2, LRRC3B, LRRC4C, LRRTM4, LVRN, LYVE1, MAB21L1, MAB21L2, MAGEE2, MAMDC2, MAPK4, MASP1, MEF2C-AS1, MEOX2, METTL24, MFAP5, MGAT4C, MGP, MICU3, MIR133A1HG, MIR8071-1, MMRN1, MORN5, MPPED2, MRGPRE, MS4A1, MS4A12, MSRB3, MUSK, MYH11, MYH2, MYLK, MYO3A, MYOC, MYOCD, MYOM1, MYOT, MYT1L, NALCN, NAP1L2, NBEA, NECAB1, NEFL, NEFM, NEGR1, NETO1, NEUROD1, NEXMIF, NEXN, NGB, NIBAN1, NLGN1, NOS1, NOVA1, NPR3, NPTX1, NPY2R, NRG3, NRK, NRSN1, NRXN1, NSG2, NTNG1, NTRK3, NUDT10, OGN, OLFM3, OMD, OTOP2, OTOP3, P2RX2, P2RY12, PAK3, PAPPA2, PCDH10, PCDH11X, PCDH9, PCOLCE2, PCP4L1, PCSK2, PDZRN4, PEG3, PENK, PGM5, PGM5-AS1, PGM5P4-AS1, PGR, PHOX2B, PI16, PIK3C2G, PIRT, PKHD1L1, PLAAT5, PLCXD3, PLD5, PLIN1, PLIN4, PLN, PLP1, PMP2, POPDC2, POU3F4, PPP1R1A, PRDM6, PRELP, PRG4, PRIMA1, PROKR1, PTCHD1, PTGIS, PTPRQ, PTPRZ1, PYGM, PYY, RANBP3L, RBFOX3, RBM20, RELN, RERGL, RGS13, RGS22, RIC3, RIMS4, RNF150, RNF180, RORB, RSPO2, SCARA5, SCGN, SCN2B, SCN7A, SCN9A, SCNN1G, SCRG1, SEMA3E, SERTM1, SERTM2, SFRP1, SFRP2, SFTPA1, SGCG, SHISAL1, SLC13A5, SLC17A8, SLC30A10, SLC4A4, SLC5A7, SLC6A2, SLC7A14, SLIT2, SLITRK2, SLITRK3, SLITRK4, SMIM28, SMYD1, SNAP25, SNAP91, SORCS1, SORCS3, SPHKAP, SPIB, SPOCK3, SST, ST8SIA3, STMN2, STMN4, STON1-GTF2A1L, STUM, SV2B, SYNM, SYNPO2, SYT10, SYT4, SYT6, TACR1, TAFA4, TCEAL2, TCEAL5, TCF23, TENM1, THBS4, TLL1, TMEFF2, TMEM100, TMEM35A, TMIGD1, TMOD1, TNNT3, TNS1, TNXB, TRARG1, TRDN, UGT2B10, UGT2B4, UNC80, VEGFD, VGLL3, VIT, VSTM2A, VXN, WSCD2, XKR4, ZBTB16, ZDHHC22, ZFHX4, ZMAT4, ZNF385B, ZNF676, and ZNF728.

12. The method of claim 10, wherein the second molecular subtype further comprises one or more types of genes selected from the group consisting of ADAT3, ANP32D, BHLHA9, BOD1L2, C4orf48, CCDC85B, CDH16, CLMAT3, CSNK1A1L, CTU1, DBET, DDC-AS1, DEFA5, EIF3IP1, FAM173A, FEZF2, FOXI3, FRMD8P1, GALR3, GJD3, GPR25, HBA1, HES4, HIST1H4A, HIST1H4L, HLA-L, IGFBP7-AS1, ITLN2, KCNE1B, LCN15, LKAAEAR1, LOC101927795, LOC101927972, LOC101928372, LOC344967, LRRC26, MAGEA10, MESP1, MIR203A, MIR324, MIR3661, MIR4449, MIR4479, MIR4665, MIR4737, MIR4767, MIR6807, MIR6858, MIR6891, MIR8075, NACA2, NOXO1, ONECUT3, PCSK1N, PDF, PITPNM2-AS1, PNMA5, PRR7, PRSS2, PRSS56, PTGER1, PTTG3P, REG3A, RNA5S9, RNU4-1, RNU5A-1, RNU5B-1, RNU5E-1, RNU6ATAC, RNY1, RPL29P2, RPRML, SBF1P1, SHISAL2B, SKOR2, SLC32A1, SMARCA5-AS1, SMCR5, SNHG25, SNORA36A, SNORD30, SNORD38A, SNORD3B-2, SNORD41, SNORD48, TMEM160, TMEM238, TPGS1, TRAPPC5, UBE2NL, WBP11P1, and ZAR1.

13. The method of claim 10, wherein the anticancer therapy is chemotherapy, radiation therapy, surgical treatment or a combination thereof.

14. The method of claim 10, wherein the anticancer therapy is standard neoadjuvant chemoradiotherapy or surgical treatment after standard neoadjuvant chemoradiotherapy.

15. The method of claim 10, wherein when the first molecular subtype is expressed in the biological sample isolated from a target subject, or the expression level thereof is higher than a control, it is predicted that a therapeutic response to the anticancer therapy or a prognosis after the anticancer therapy is poor.

16. The method of claim 10, wherein when the second molecular subtype is expressed in the biological sample isolated from a target subject, or the expression level thereof is higher than a control, it is predicted that a therapeutic response to the anticancer therapy or a prognosis after the anticancer therapy is good.

17. The method of claim 10, further comprising:
confirming the subject's TNM stage, age, sex, a pathologic complete response (pCR) or combined information thereof.

18. The method of claim 17, wherein when the expression level of the first molecular subtype of the subject is higher than a control, and the TNM stage of the subject is T3 or T4, it is predicted that the prognosis after anticancer therapy is poor.

19. The method of claim 17, wherein when the expression level of the first molecular subtype of the subject is higher than a control, and the TNM stage of the subject is N1 or N2, it is predicted that the prognosis after anticancer therapy is poor.

20. The method of claim 17, wherein when the expression level of the second molecular subtype of the subject is higher than a control, and pCR is achieved after the anticancer therapy, it is predicted that a prognosis after anticancer therapy is good.

21. The method of claim 17, wherein when the expression level of the second molecular subtype of the subject is higher than a control, and the TNM stage of the subject is T0, T1 or T2, it is predicted that a prognosis after the anticancer therapy is good.

22. The method of claim 17, wherein when the expression level of the second molecular subtype of the subject is higher than a control, and the TNM stage of the subject is N0, it is predicted that a prognosis after anticancer therapy is good.

23. The method of claim 10, wherein the cancer is one or more types of cancer selected from the group consisting of breast cancer, uterine cancer, esophageal cancer, stomach cancer, brain cancer, rectal cancer, colon cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma, and leukemia.

24. A device for predicting a therapeutic response to anticancer therapy or a prognosis after anticancer therapy, or identifying a target patient for total neoadjuvant therapy prior to anticancer therapy, comprising:
a measurement unit for measuring the expression level of one or more genes of a first molecular subtype and a second molecular subtype or a protein encoded thereby in a biological sample isolated from a target subject; and
a calculation unit that provides information for predicting a therapeutic response to anticancer therapy or a prognosis after anticancer therapy, and identifying a target patient for total neoadjuvant therapy from the expression level in the subject,
wherein the first molecular subtype comprises one or more types of genes selected from PMP2, AGTR1, PLCXD3, TCEAL6, ANKRD1, ARHGAP26-AS1, and TCEAL6, and
the second molecular subtype comprises one or more types of genes selected from PGP, SLC26A3, HIST1H4C, RUVBL2, RAB19, HIST2H2AC, and SNORD69.

25. The device of claim 24, wherein the anticancer therapy is chemotherapy, radiation therapy, surgical treatment or a combination thereof.

26. The device of claim 24, wherein the anticancer therapy is standard neoadjuvant chemoradiotherapy or surgical treatment after standard neoadjuvant chemoradiotherapy.

27. The device of claim 24, further comprising:
an input unit for receiving the TNM stage, age or sex of the subject, a pathologic complete response (pCR) or combined information thereof.
